# EUROPÄISCHE PATENTSCHRIFT

(11) **EP 2 176 264 B1**
(45) Veröffentlichungstag und Bekanntmachung des Hinweises auf die Patenterteilung: **19.12.2012**
(21) Anmeldenummer: 08773748.2
(22) Anmeldetag: 28.06.2008
(51) Int. Cl.: C07D 487/04, A61K 31/53, A61P 7/00

(54) **IMIDAZO-, PYRAZOLOPYRAZINE UND IMIDAZOTRIAZINE ZUR BEHANDLUNG HÄMATOLOGISCHER ERKRANKUNGEN**
IMIDAZO-, PYRAZOLOPYRAZINES AND IMIDAZOTRIAZINES FOR TREATING HEMATOLOGICAL DISORDERS
IMIDAZOPYRAZINES, PYRAZOLOPYRAZINES ET IMIDAZOTRIAZINES POUR LE TRAITEMENT DE TROUBLES HEMATOLOGIQUES

(30) Priorität: 11.07.2007 DE 102007032349
(43) Veröffentlichungstag der Anmeldung: 21.04.2010
(73) Patentinhaber: Bayer Intellectual Property GmbH, 40789 Monheim (DE)
(72) Erfinder: SIEGEL, Stephan, 10779 Berlin (DE); WILMEN, Andreas, 50676 Köln (DE); RÖHRIG, Susanne, 40724 Hilden (DE); SVENSTRUP, Niels, 42553 Velbert (DE); GNOTH, Mark, Jean, 40822 Mettmann (DE); HEITMEIER, Stefan, 42489 Wülfrath (DE); RESTER, Ulrich, 42115 Wupertal (DE); TERSTEEGEN, Adrian, 42111 Wuppertal (DE); GERISCH, Michael, 42119 Wuppertal (DE)
(74) Vertreter: Bayer Intellectual Property GmbH
(86) Internationale Anmeldenummer: PCT/EP2008/005305
(87) Internationale Veröffentlichungsnummer: WO 2009/007029

(56) Entgegenhaltungen:
- WO-A-03/049739
- WO-A-2004/026877

## Beschreibung

Die Erfindung betrifft substituierte Imidazo-, Pyrazolopyrazine und Imidazotriazine und Verfahren zu ihrer Herstellung sowie ihre Verwendung zur Herstellung von Arzneimitteln zur Behandlung und/oder Prophylaxe von Krankheiten, insbesondere von hämatologischen Erkrankungen, vorzugsweise von Leukopenien und Neutropenien.

Glykogen Synthase Kinase 3 (GSK3) gehört zur Familie der Serine/Threonin-Kinasen. Spezifische Substrate sind unter anderem Zytoskelettproteine und Transkriptionsfaktoren. Zwei Isoformen, GSK3α und GSK3β, wurden bisher identifiziert (Woodgett JR., Trends Biochem. Sci. (1991), 16(5), 177-81). Beide Isoformen sind in vornehmlich ruhenden, nicht proliferierenden Zellen konstitutiv aktiv.

GSK3β kommt eine zentrale Bedeutung innerhalb des Wnt/Wingless-Signaltransduktionsweges zu. Dieser stellt einer der wichtigsten, evolutionärkonservierten Signalsysteme dar. Wnt-Signale kontrollieren sehr frühe musterbildende Prozesse während der Embryogenese, sie induzieren Mesodermbildung und viele Organe, und sie steuern die Proliferation und Differenzierung von Stammzellen (Wodarz A., Nusse R., Annu. Rev. Cell Dev. Biol. (1998), 14, 59-88; Kirstetter et al., Nat Immunol. (2006), 7(10), 1048-56). Der Wnt-Signalweg ist intrazellulär aufgegliedert, wodurch unterschiedlichste Prozesse gesteuert werden können. Innerhalb der Wnt-Kaskade ist die Glykogen Synthase Kinase 3 Bestandteil eines Multiproteinkomplexes, zu dem u.a. das Strukturmoleküle Axin, das Tumorsuppressor-Protein APC sowie der Transkriptionskofaktor β-Catenin gehören. β-Catenin ist dabei das wichtigste Substrat der GSK3β. Die Konsequenz dieser GSK3β-vermittelten Phophorylierung ist der proteasomale Abbau von β-Catenin. Inhibition der GSK3-Aktivität führt zu einer Akkumulation des β-Catenins in der Zelle mit einer sich anschließenden Translokation in den Zellkern. Dort fungiert β-Catenin als ein Kofaktor in Transkriptionskomplexe und damit für die Expression definierter Zielgene mit verantwortlich.

Strahlen- oder Chemotherapien gehören zu den Standardansätzen bei der Krebsbekämpfung. Beide Therapieformen sind im Bezug auf ihre Zielzellen unspezifisch, d.h. es werden nicht nur Tumorsondern auch nicht-transformierte, proliferierende Zellen getroffen. Zu diesen nichttransformierten, proliferienden Zellen gehören auch hämatopoetische Vorläuferzellen, die sich u.a. zu neutrophilen Granulozyten entwickeln. Eine signifikante Verringerung der Anzahl an Neutrophilen wird als Neutropenie bezeichnet. Eine durch Chemo- oder Strahlentherapie induzierte Neutropenie resultiert klinisch in einer erhöhten Infektanfälligkeit. Bei ausgeprägter Neutropenie erhöht sich die Morbidität und unter Umständen auch die Mortalität einer Therapie (O'Brien et al., British Journal of Cancer (2006), 95, 1632 - 1636).

Inhibition der GSK3-Aktivität führt zu einer gesteigerten Proliferations- und Differenzierungsrate hämatopoetischer Stammzellen und kann dementsprechend zur therapeutischen Intervention hinsichtlich einer therapieinduzierten Neutropenie genutzt werden.

WO99/064401 beschreibt unter anderem Imidazopyrazine als Somatostatin Rezeptor Liganden zur Behandlung von Diabetes. WO2004/026877, US2006/0183746, US2006/0106023 und WO2007/058873 beschreiben die Verwendung von Imidazopyrazinylaminen zur Behandlung von Krebs. Pyrazolo- und Imidazopyrazine zur Behandlung von Krebs werden in WO2006/044687 offenbart. WO03/000693 beansprucht Imidazotriazine als PDE10-Inhhibitoren zur Behandlung von neurodegenerativen Krankheiten. WO 2007/145921 beschreibt Imidazopyrazine als Protein Kinase Inhibitoren zur Behandlung von Krebs.

Eine Aufgabe der vorliegenden Erfindung ist daher die Bereitstellung neuer Verbindungen als GSK3β-Inhibitoren zur Behandlung von hämatologischen Erkrankungen, vorzugsweise von Neutropenie bei Menschen und Tieren.

Gegenstand der Erfindung sind Verbindungen der Formel 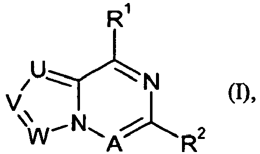 in welcher
entweder
- U: für N steht,
- V: für CR¹² steht,
- W: für CH steht,
- A: für CR¹⁵ steht,
oder
- U: für CH steht,
- V: für CR¹² steht,
- W: für N steht,
- A: für CR¹⁵ steht,
oder
- U: für CR¹⁶ steht,
- V: für N steht,
- W: für CR¹⁷ steht,
- A: für N steht,
wobei
R¹² für Wasserstoff, Hydroxy, Amino, Hydroxycarbonyl, Aminocarbonyl, Trifluormethyl, Trifluormethoxy, Cyano, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonylamino, 5-oder 6-gliedriges Heterocyclylcarbonyl, -CH₂R¹³ oder -CH₂CH₂R¹⁴ steht, wobei Heterocyclylcarbonyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkoxy, Alkylamino, Alkylcarbonyl, Alkoxycarbonyl, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylarninocarbonyl, C₁-C₄-Alkylcarbonylamino, 5- oder 6-gliedriges Heterocyclyl und Phenyl,
worin Phenyl substituiert sein kann mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylarnino,
und
worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Wkylcarbonyl, C₁-C₄-Alkoxycarbonyl und C₁-C₄-Alkylaminocarbonyl,
und
wobei
R¹³ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, C₃-C₆-Cycloalkylamino oder 5- oder 6-gliedriges Heterocyclyl steht,
worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei
R¹⁴ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino oder 5- oder 6-gliedriges Heterocyclyl steht,
worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
R¹⁵ für Wasserstoff, Halogen, Cyano, Trifluormethyl, C₁-C₃-Alkyl, Methoxy, Methylthio oder Cyclopropyl steht,
R¹⁶ für Wasserstoff oder Methyl steht,
R¹⁷ für Wasserstoff oder Methyl steht,
- R¹: für eine Gruppe der Formel 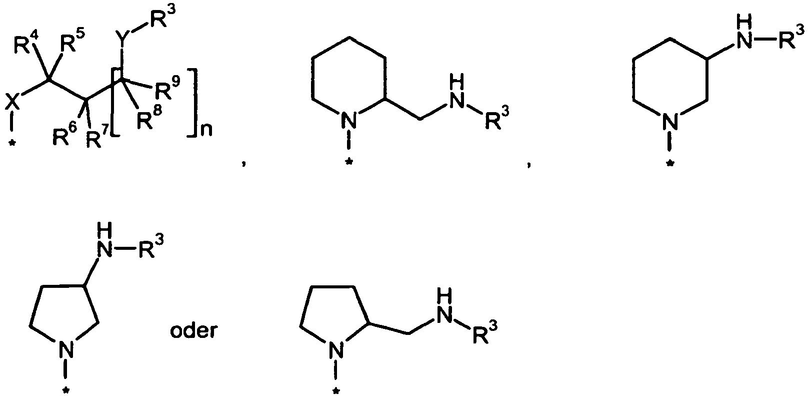 steht,
wobei
* die Anknüpfstelle an den Heterocyclus bedeutet,
n für die Zahl 0 oder 1 steht,
X für NR¹⁰, S oder O steht,
worin
R¹⁰ für Wasserstoff, C₁-C₃-Alkyl oder Cyclopropyl steht,
Y für NR¹¹ oder S steht,
worin
R¹¹ für Wasserstoff, C₁-C₃-Alkyl oder Cyclopropyl steht,
R³ für 2-Pyridyl, Pyrimid-2-yl, 2-Aminopyrimid-4-yl, 2-(Mono-C₁-C₄-Alkylamino)pyrimid-4-yl, 2-(Mono-C₃-C₄-Cycloalkylamino)pyrimid-4-yl, Pyridazin-3(2H)-on-6-yl, 1,3-Oxazol-2-yl, 1,3-Oxazol-4-yl, 1,2,4-Oxadiazol-3-yl, 1,2,3-Oxadiazol-4-yl, 1,3-Thiazol-2-yl, 1,3-Thiazol-4-yl, 1H-1,2,4-Triazol-5-yl, 2,4-Dihydro-3H-1,2,4- triazol-3-on-5-yl oder 1,2-Pyrazol-5-yl steht,
wobei 2-Pyridyl, Pyrimid-2-yl, 1,3-Oxazol-2-yl, 1,3-Oxazol-4-yl, 1,3-Thiazol-2-yl und 1,3-Thiazol-4-yl substituiert sind mit 1 oder 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₃-C₄-Cycloalkylamino, C₁-C₄-Alkylcarbonyl, C₁-C₄-Alkoxycarbonyl, C₁-C₄-Alkylanünocarbonyl und C₃-C₆-Cycloalkylcarbonyl,
worin Alkyl, Alkoxy, Alkylanüno, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl, und C₃-C₆-Cycloalkyl,
und
wobei 2-Aminopyrimid-4-yl, 2-(Mono-C₁-C₄-Alkylamino)pyrimid-4-yl, 2-(Mono-C₃-C₄-Cycloalkylamino)pyrimid-4-yl, Pyridazin-3(2H)-on-6-yl, 1,2,4-Oxadiazol-3-yl, 1,2,3-Oxadiazol-4-yl, 1H-1,2,4-Triazol-5-yl, 2,4-Dihydro-3H-1,2,4-triazol-3-on-5-yl und 1,2-Pyrazol-5-yl substituiert sein können mit einem Substituenten, wobei der Substituent ausgewählt wird aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₃-C₄-Cycloalkylamino, C₁-C₄-Alkoxycarbonyl, C₁-C₄-Alkylaminocarbonyl und C₃-C₆-Cycloalkylcarbonyl,
R⁴ für Wasserstoff, C₁-C₃-Alkyl oder Cyclopropyl steht,
R⁵ für Wasserstoff oder C₁-C₃-Alkyl steht,
R⁶ für Wasserstoff, C₁-C₃-Alkyl oder Cyclopropyl steht,
R⁷ für Wasserstoff oder C₁-C₃-Alkyl steht,
R⁸ für Wasserstoff, C₁-C₃-Alkyl oder Cyclopropyl steht,
R⁹ für Wasserstoff oder C₁-C₃-Alkyl steht,
R² für C₆-C₁₀-Aryl oder 5- bis 10-gliedriges Heteroaryl steht,
wobei Aryl und Heteroaryl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5- oder 6-gliedrigem Heterocyclylcarbonyl, 5- oder 6-gliedrigem Heterocyclylmethyl und 5- oder 6-gliedrigem Heteroaryl,
worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino, oder
zwei der Substituenten am Aryl zusammen mit den Kohlenstoffatomen, an die sie gebunden sind, ein 1,3-Dioxolan oder 1,4-Dioxan bilden,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausfühzungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin, N-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkylcarbonyl, Alkoxycarbonyl, Alkylaminocarbonyl, Alkylcarbonylamino, Alkylsulfonyl, Alkylsulfonylamino und Alkylaminosulfonyl stehen für einen linearen oder verzweigten Alkylrest mit 1 bis 4 Kohlenstoffatomen, beispielhaft und vorzugsweise für Methyl, Ethyl, n-Propyl, iso-Propyl, n-Butyl und tert-Butyl.

Alkoxy steht beispielhaft und vorzugsweise für Methoxy, Ethoxy, n-Propoxy, iso-Propoxy, n-Butoxy und tert-Butoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, tert-Butylamino, *N*,*N*-Dimethylamino, *N*,*N*-Diethylamino, *N-*Ethyl-*N-*methylamino, *N*-Methyl-*N*-n-propylamino, *N-*iso-Propyl-*N-*n-propylamino und *N*-tert-Butyl-*N-*methylamino. C₁-C₄-Alkylamino steht beispielsweise für einen Monoalkylaminorest mit 1 bis 4 Kohlenstoffatomen oder für einen Dialkylaminorest mit jeweils 1 bis 4 Kohlenstoffatomen pro Alkylsubstituent.

Mono-Alkylamino steht für einen Alkylaminorest mit einem linearen oder verzweigten Alkylsubstituenten, beispielhaft und vorzugsweise für Methylamino, Ethylamino, n-Propylamino, iso-Propylamino und tert-Butylamino.

Mono-Cycloalkylamino steht für einen Cycloalkylaminorest mit einem Cycloalkyl-Substituenten und der weitere Substituent am Aminorest ist Wasserstoff, beispielhaft und vorzugsweise für Cyclopropylamino und Cyclobutylamino.

Alkylcarbonyl steht beispielhaft und vorzugsweise für Methylcarbonyl, Ethylcarbonyl, n-Propylcarbonyl, iso-Propylcarbonyl, n-Butylcarbonyl und tert-Butylcarbonyl.

Alkoxycarbonyl steht beispielhaft und vorzugsweise für Methoxycarbonyl, Ethoxycarbonyl, n-Propoxycarbonyl, iso-Propoxycarbonyl, n-Butoxycarbonyl und tert-Butoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, N,N-Dimethylaminocarbonyl, N,N-Diethylaminocarbonyl, N-Ethyl-N-methylaminocarbonyl, *N-*Methyl-N-n-propylaminocarbonyl, N-iso-Propyl-N-n-propylaminocarbonyl und N-tert-Butyl-N-methylaminocarbonyl. C₁-C₄-Alkylaminocarbonyl steht beispielsweise für einen Monoalkylaminocarbonylrest mit 1 bis 4 Kohlenstoffatomen oder für einen Dialkylaminocarbonylrest mit jeweils 1 bis 4 Kohlenstoffatomen pro Alkylsubstituent.

Alkylcarbonylamino steht beispielhaft und vorzugsweise für Methylcarbonylamino, Ethylcarbonylamino, n-Propylcarbonylamino, iso-Propylcarbonylamino, n-Butylcarbonylamino und tert-Butylcarbonylamino.

Alkylsulfonyl steht beispielhaft und vorzugsweise für Methylsulfonyl, Ethylsulfonyl, n-Propylsulfonyl, iso-Propylsulfonyl, n-Butylsulfonyl und tert-Butylsulfonyl.

Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, iso-Propylaminosulfonyl, tert-Butylaminosulfonyl, *N,N-*Dimethylaminosulfonyl, *N,N-*Diethylaminosulfonyl, *N*-Ethyl-*N*-methylaminosulfonyl, *N*-Methyl-*N*-n-propylaminosulfonyl, *N*-iso-Propyl-*N*-n-propylaminosulfonyl und *N*-tert-Butyl-*N*-methylaminosulfonyl. C₁-C₄-Alkylaminosulfonyl steht beispielsweise für einen Monoalkylaminosulfonylrest mit 1 bis 4 Kohlenstoffatomen oder für einen Dialkylamino-sulfonylrest mit jeweils 1 bis 4 Kohlenstoffatomen pro Alkylsubstituent.

Alkylsulfonylamino steht beispielhaft und vorzugsweise für Methylsulfonylamino, Ethylsulfonylamino, n-Propylsulfonylamino, iso-Propylsulfonylamino, n-Butylsulfonylamino und tert-Butylsulfonylamino.

Cycloalkyl 1 steht für eine monocyclische Cycloalkylgruppe mit in der Regel 3 bis 6 Kohlenstoffatomen, beispielhaft und vorzugsweise für Cycloalkyl seien genannt Cyclopropyl, Cyclobutyl, Cyclopentyl und Cyclohexyl.

Cycloalkylamino steht für einen Cycloalkylaminorest mit einem Cycloalkyl-Substituenten und der weitere Substituent am Aminorest ist Wasserstoff oder ein Alkylrest, beispielhaft und vorzugsweise für Cyclopropylamino, Cyclobutylamino, N-Cyclopropyl-N-methylamino und N-Cyclobutyl-N-methylamino.

Heterocyclyl steht für einen monocyclischen, heterocyclischen Rest mit 5 oder 6 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Piperazin-1-yl, Piperazin-2-yl.

Heteroaryl steht für einen aromatischen, mono- oder bicyclischen Rest mit in der Regel 5 bis 10, vorzugsweise 5 oder 6 Ringatomen und bis zu 5, vorzugsweise bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Benzofuranyl, Benzothiophenyl, Chinolinyl, Isochinolinyl, Benzoxazolyl, Benzimidazolyl.

Halogen steht für Fluor, Chlor, Brom und Jod, vorzugsweise für Fluor und Chlor.

In den Formeln der Gruppe, die für R¹ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹ gebunden ist.

Bevorzugt sind Verbindungen der Formel (I), in welcher
entweder
- U: für N steht,
- V: für CR¹² steht,
- W: für CH steht,
- A: für CR¹⁵ steht,
oder
- U: für CH steht,
- V: für CR¹² steht,
- W: für N steht,
- A: für CR¹⁵ steht,
oder
- U: für CR¹⁶ steht,
- V: für N steht,
- W: für CR¹⁷ steht,
- A: für N steht,
wobei
R¹² für Wasserstoff, Hydroxycarbonyl, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl, C₁-C₄-Alkylcarbonylamino, 5- oder 6-gliedriges Heterocyclylcarbonyl, -CH₂R¹³ oder -CH₂CH₂R¹⁴ steht,
wobei Heterocyclylcarbonyl substituiert sein kann mit 1 bis 3 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkoxy, Alkylamino, Alkylcarbonyl, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
worin Heterocyclyl substituiert sein kann mit 1 bis 2 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei
R¹³ für Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, C₃-C₆-Cycloalkylamino oder 5- oder 6-gliedriges Heterocyclyl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 2 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei
R¹⁴ für Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino oder 5- oder 6-gliedriges Heterocyclyl steht,
worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 2 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
R¹⁵ für Wasserstoff, Halogen, Cyano oder Trifluormethyl steht,
R¹⁶ für Wasserstoff oder Methyl steht,
R¹⁷ für Wasserstoff oder Methyl steht,
- R¹: für eine Gruppe der Formel 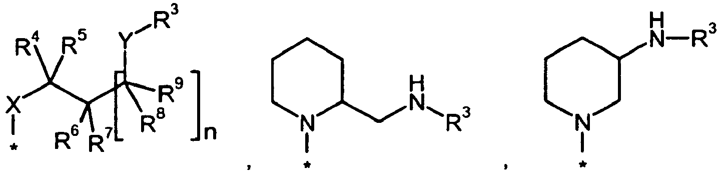 oder 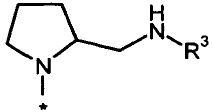 steht,
wobei
* die Anknüpfstelle an den Heterocyclus bedeutet,
n für die Zahl 0 oder 1 steht,
X für NR¹⁰, S oder O steht,
worin
R¹⁰ für Wasserstoff oder Methyl steht,
Y für NR¹¹ oder S steht, worin
R¹¹ für Wasserstoff oder Methyl steht,
R³ für 2-Pyridyl, Pyrimid-2-yl, 2-Aminopyrimid-4-yl, 1,3-Oxazol-2-yl, 1,3-Oxazol-4-yl, 1,2,4-Oxadiazol-3-yl, 1,2,3-Oxadiazol-4-yl, 1,3-Thiazol-2-yl oder 1,3-Thiazol-4-yl steht,
wobei 2-Pyridyl, Pyrimid-2-yl, 1,3-Oxazol-2-yl, 1,3-Oxazol-4-yl, 1,3-Thiazol-2-yl und 1,3-Thiazol-4-yl substituiert sind mit 1 oder 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl Methyl, Ethyl, Methoxy, Ethoxy, C₁-C₄-Alkylamino, Methylcarbonyl, Ethylcarbonyl, Cyclopropylcarbonyl, Methoxycarbonyl und Ethoxycarbonyl,
und
wobei 2-Aminopyrimid-4-yl, 1,2,4-Oxadiazol-3-yl und 1,2,3-Oxadiazol-4-yl substituiert sein können mit einem Substituenten, wobei der Substituent ausgewählt wird aus der Gruppe bestehend aus Halogen, Cyano, Nitro, Amino, Trifluormethyl, Trifluormethoxy, Aminocarbonyl, Trifluormethylcarbonyl, Methyl, Ethyl, Methoxy, Ethoxy, C₁-C₄-Alkylamino, Methylcarbonyl, Ethylcarbonyl, Cyclopropylcarbonyl, Methoxycarbonyl und Ethoxycarbonyl,
R⁴ für Wasserstoff oder Methyl steht,
R⁵ für Wasserstoff oder Methyl steht,
R⁶ für Wasserstoff oder Methyl steht,
R⁷ für Wasserstoff oder Methyl steht,
R⁸ für Wasserstoff oder Methyl steht,
R⁹ für Wasserstoff oder Methyl steht,
- R²: für C₆-C₁₀-Aryl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Chinolinyl, Benzfuranyl oder Benzoxazolyl steht,
wobei Aryl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Chinolinyl, Benzfuranyl und Benzoxazolyl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5- oder 6-gliedrigem Heterocyclylcarbonyl, 5- oder 6-gliedrigem Heterocyclylmethyl und 5- oder 6-gliedrigem Heteroaryl,
worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino,
und ihre Salze, ihre Solvate und die Solvate ihrer Salze.

Bevorzugt sind auch Verbindungen der Formel (I), in welcher
entweder
- U: für N steht,
- V: für CR¹² steht,
- W: für CH steht,
- A: für CR¹⁵ steht,
oder
- U: für CH steht,
- V: für CR¹² steht,
- W: für N steht,
- A: für CR¹⁵ steht,
oder
- U: für CR¹⁶ steht,
- V: für N steht,
- W: für CR¹⁷ steht,
- A: für N steht,
wobei
R¹² für Wasserstoff, Hydroxycarbonyl, Aminocarbonyl, Methyl, Ethyl, C₁-C₄-Alkylcarbonyl, C₁-C₄-Alkoxycarbonyl, C₁-C₄-Alkylaminocarbonyl, C₁-C₄-Alkylcarbonylamino, Pyrrolidinylcarbonyl, Piperidinylcarbonyl, Piperazinylcarbonyl, Mopholinylcarbonyl oder -CH₂R¹³ steht,
wobei Pyrrolidinylcarbonyl, Piperidinylcarbonyl, Piperazinylcarbonyl und Mopholinylcarbonyl substituiert sein können mit 1 bis 2 Substituenten, wobei die Substituenten unabhängig voneinander ausgewählt werden aus der Gruppe bestehend aus Oxo, Methyl und Ethyl,
und
wobei Alkylcarbonyl, C₂-C₄-Alkoxycarbonyl und C₂-C₄-Alkylaminocarbonyl substituiert sein können mit einem Substituenten, wobei der Substituent ausgewählt wird aus der Gruppe bestehend aus Hydroxy, Amino, C₁-C₄-Alkylamino, Pyrrolidinyl, Piperidinyl, Piperazinyl und Morphlinyl,
worin Pyrrolidinyl, Piperidinyl, Piperazinyl und Morphlinyl substituiert sein können mit 1 bis 2 Substituenten, wobei die Substituenten unabhängig voneinander ausgewählt werden aus der Gruppe bestehend aus Oxo, Methyl und Ethyl,
und
wobei
R¹³ für Hydroxy, Amino, Hydroxycarbonyl, Aminocarbonyl, C₁-C₄-Alkoxy, C₁-C₄-Alkylamino, Pyrrolidinyl, Piperidinyl, Piperazinyl oder Morphlinyl steht,
worin Pyrrolidinyl, Piperidinyl, Piperazinyl und Morphlinyl substituiert sein können mit 1 bis 2 Substituenten, wobei die Substituenten unabhängig voneinander ausgewählt werden aus der Gruppe bestehend aus Oxo, Methyl und Ethyl,
R¹⁵ für Wasserstoff steht,
R¹⁶ für Wasserstoff oder Methyl steht,
R¹⁷ für Wasserstoff oder Methyl steht,
- R¹: für eine Gruppe der Formel 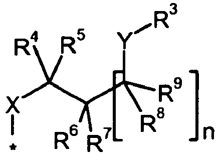 steht,
wobei
* die Anknüpfstelle an den Heterocyclus bedeutet,
n für die Zahl 0 steht,
X für NR¹⁰ steht,
worin
R¹⁰ für Wasserstoff steht,
Y für NR¹¹ steht,
worin
R¹¹ für Wasserstoff oder Methyl steht,
R³ für 2-Pyridyl, Pyrimid-2-yl, 2-Aminopyrimid-4-yl, 1,3-Thiazol-2-yl oder 1,3-Thiazol-4-yl steht,
wobei 2-Pyridyl, Pyrimid-2-yl, 1,3-Thiazol-2-yl und 1,3-Thiazol-4-yl substituiert sind mit 1 oder 2 Substituenten, wobei die Substituenten unabhängig voneinander ausgewählt werden aus der Gruppe bestehend aus Fluor, Chlor, Cyano, Nitro, Amino und Trifluormethyl,
und
wobei 2-Aminopyrimid-4-yl substituiert sein kann mit einem Substituenten, wobei der Substituent ausgewählt wird aus der Gruppe bestehend aus Fluor, Chlor, Cyano, Nitro, Amino und Trifluormethyl,
R⁴ für Wasserstoff steht,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff oder Methyl steht,
- R²: für Phenyl, Thienyl, Pyrazolyl oder Pyridyl steht,
wobei Phenyl, Thienyl, Pyrazolyl und Pyridyl substituiert sein können mit 1 bis 2 Substituenten, wobei die Substituenten unabhängig voneinander ausgewählt werden aus der Gruppe bestehend aus Halogen, Trifluormethyl, Trifluormethoxy, Aminocarbonyl, C₁-C₄-Alkyl, C₁-C₄-Alkoxy, C₁-C₄-Alkoxycarbonyl, C₁-C₄-Alkylaminocarbonyl, Pyrrolidinyl, Piperidinyl, Morpholinyl und Morpholinylcarbonyl,
und ihre Salze, ihre Solvate und die Solvate ihrer Salze.

Besonders bevorzugt sind Verbindungen der Formel (I), in welcher
entweder
- U: für N steht,
- V: für CR¹² steht,
- W: für CH steht,
- A: für CR¹⁵ steht,
oder
- U: für CH steht,
- V: für CR¹² steht,
- W: für N steht,
- A: für CR¹⁵ steht,
oder
- U: für CR¹⁶ steht,
- V: für N steht,
- W: für CR¹⁷ steht,
- A: für N steht,
wobei
R¹² für Wasserstoff, Hydroxycarbonyl, Methyl, Ethyl, Methoxycarbonyl, Ethoxycarbonyl, C₁-C₄-Alkylaminocarbonyl, Piperidinylcarbonyl oder Mopholinylcarbonyl steht,
wobei Piperidinylcarbonyl und Mopholinylcarbonyl substituiert sein können mit einem Substituenten, wobei der Substituent ausgewählt wird aus der Gruppe bestehend aus Methyl und Ethyl,
und
wobei C₂-C₄-Alkylaminocarbonyl substituiert sein kann mit einem Substituenten, wobei der Substituent ausgewählt wird aus der Gruppe bestehend aus C₁-C₄-Alkylamino, Piperazinyl und Morphlinyl,
worin Piperazinyl und Morphlinyl substituiert sein können mit einem Substituenten, wobei der Substituent ausgewählt wird aus der Gruppe bestehend aus Methyl und Ethyl,
R¹⁵ für Wasserstoff steht,
R¹⁶ für Methyl steht,
R¹⁷ für Methyl steht,
- R¹: für eine Gruppe der Formel 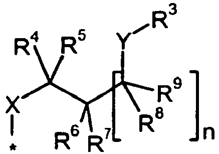 steht,
wobei
* die Anknüpfstelle an den Heterocyclus bedeutet,
n für die Zahl 0 steht,
X für NR¹⁰ steht,
worin
R¹⁰ für Wasserstoff steht,
Y für NR¹¹ steht,
worin
R¹¹ für Wasserstoff oder Methyl steht,
R³ für eine Gruppe der Formel 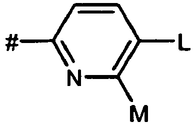 steht,
wobei
# die Anknüpfstelle an Y bedeutet,
L für Cyano, Nitro oder Trifluormethyl steht,
M für Wasserstoff oder Amino steht,
R⁴ für Wasserstoff steht,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
- R²: für Phenyl steht,
wobei Phenyl substituiert sein kann mit 1 bis 2 Substituenten, wobei die Substituenten unabhängig voneinander ausgewählt werden aus der Gruppe bestehend aus Fluor, Chlor, Trifluormethyl, Trifluormethoxy, C₁-C₃-Alkyl, Methoxy, Methoxycarbonyl und Ethoxycarbonyl,
und ihre Salze, ihre Solvate und die Solvate ihrer Salze.

Bevorzugt sind auch Verbindungen der Formel (I), in welcher entweder U für N, V für CR¹², W für CH und A für CH oder U für CH, V für CR¹², W für N und A für CH oder U für CR¹⁶, V für N, W für CR¹⁷ und A für N stehen,
wobei
- R¹²: für Wasserstoff, Hydroxycarbonyl, Methyl, Ethyl, Methoxycarbonyl, Ethoxycarbonyl, C₁-C₄-Alkylaminocarbonyl, Piperidinylcarbonyl oder Mopholinylcarbonyl steht,
wobei Piperidinylcarbonyl und Mopholinylcarbonyl substituiert sein können mit einem Substituenten, wobei der Substituent ausgewählt wird aus der Gruppe bestehend aus Methyl und Ethyl,
und
wobei C₂-C₄-Alkylaminocarbonyl substituiert sein kann mit einem Substituenten, wobei der Substituent ausgewählt wird aus der Gruppe bestehend aus C₁-C₄-Alkylamino, Piperazinyl und Morphlinyl,
worin Piperazinyl und Morphlinyl substituiert sein können mit einem Substituenten, wobei der Substituent ausgewählt wird aus der Gruppe bestehend aus Methyl und Ethyl,
- R¹⁶: für Methyl steht,
und
- R¹⁷: für Methyl steht.

Bevorzugt sind auch Verbindungen der Formel (I), in welcher U für N, V für CR¹², W für CH und A für CH stehen,
wobei
- R¹²: für Wasserstoff, Hydroxycarbonyl, Methyl, Ethyl, Methoxycarbonyl, Ethoxycarbonyl, C₁-C₄-Alkylaminocarbonyl, Piperidinylcarbonyl oder Mopholinylcarbonyl steht,
wobei Piperidinylcarbonyl und Mopholinylcarbonyl substituiert sein können mit einem Substituenten, wobei der Substituent ausgewählt wird aus der Gruppe bestehend aus Methyl und Ethyl,
und
wobei C₂-C₄-Alkylaminocarbonyl substituiert sein kann mit einem Substituenten, wobei der Substituent ausgewählt wird aus der Gruppe bestehend aus C₁-C₄-Alkylamino, Piperazinyl und Morphlinyl,
worin Piperazinyl und Morphlinyl substituiert sein können mit einem Substituenten, wobei der Substituent ausgewählt wird aus der Gruppe bestehend aus Methyl und Ethyl,

Bevorzugt sind auch Verbindungen der Formel (I), in welcher U für CH, V für CR¹², W für N und A für CH stehen,
wobei
- R¹²: für Wasserstoff, Hydroxycarbonyl, Methyl, Ethyl, Methoxycarbonyl, Ethoxycarbonyl, C₁-C₄-Alkylaminocarbonyl, Piperidinylcarbonyl oder Mopholinylcarbonyl steht,
wobei Piperidinylcarbonyl und Mopholinylcarbonyl substituiert sein können mit einem Substituenten, wobei der Substituent ausgewählt wird aus der Gruppe bestehend aus Methyl und Ethyl,
und
wobei C₂-C₄-Alkylaminocarbonyl substituiert sein kann mit einem Substituenten, wobei der Substituent ausgewählt wird aus der Gruppe bestehend aus C₁-C₄-Alkylamino, Piperazinyl und Morphlinyl,
worin Piperazinyl und Morphlinyl substituiert sein können mit einem Substituenten, wobei der Substituent ausgewählt wird aus der Gruppe bestehend aus Methyl und Ethyl,

Bevorzugt sind auch Verbindungen der Formel (I), in welcher U für CR¹⁶, V für N, W für CR¹⁷ und A für N stehen, wobei R¹⁶ und R¹⁷ für Methyl stehen.

Bevorzugt sind auch Verbindungen der Formel (I), in welcher R¹ für -NHCH₂CH₂NH-R³ steht, wobei R³ für 5-Cyanopyrid-2-yl steht.

Bevorzugt sind auch Verbindungen der Formel (I), in welcher n für die Zahl 0 steht.

Bevorzugt sind auch Verbindungen der Formel (I), in welcher X für NR¹⁰ steht, wobei R¹⁰ für Wasserstoff steht.

Bevorzugt sind auch Verbindungen der Formel (I), in welcher Y für NR¹¹ steht, wobei R¹¹ für Wasserstoff steht.

Bevorzugt sind auch Verbindungen der Formel (I), in welcher R³ für 5-Cyanopyrid-2-yl steht.

Bevorzugt sind auch Verbindungen der Formel (I), in welcher R⁴, R⁵, R⁶, R⁷, R⁸ und R⁹ für Wasserstoff stehen.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 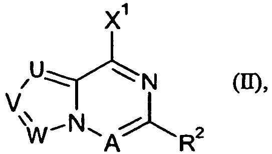 in welcher
A, U, V, W und R² die oben angegebene Bedeutung haben,
und
- X¹: für Halogen, bevorzugt Chlor oder Fluor, steht,
mit Verbindungen der Formel

R¹-H (III),

in welcher
R¹ die oben angegebene Bedeutung hat,
umgesetzt werden,
oder
[B] die Verbindungen der Formel 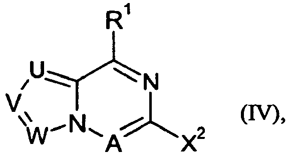 in welcher
R¹ die oben angegebene Bedeutung hat,
und
- A: für CR¹⁵ steht,
wobei R¹⁵ die oben angegebene Bedeutung hat,
- U: für N steht,
- V: für CR¹² steht,
- wobei R¹²: die oben angegebene Bedeutung hat,
- W: für CH steht,
- X²: für Iod, Brom, Chlor oder Trifluormethansulfonyl, bevorzugt Iod oder Brom, steht,
mit Verbindungen der Formel

Q-R² (V),

in welcher
R² die oben angegebene Bedeutung hat, und
- Q: für -B(OH)₂, einen Boronsäure-Ester, bevorzugt Boronsäurepinakolester, oder -BF₃⁻K⁺ steht,
unter Suzuki-Kupplungsbedingungen zu Verbindungen der Formel 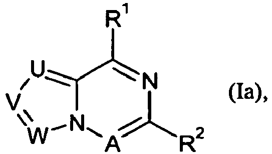 in welcher
R¹ und R² die oben angegebene Bedeutung haben,
und
- A: für CR¹⁵ steht,
wobei R¹⁵ die oben angegebene Bedeutung hat,
- U: für N steht,
- V: für CR¹² steht,
wobei R¹² die oben angegebene Bedeutung hat,
- W: für CH steht,
umgesetzt werden.

Die Verbindungen der Formel (Ia) sind eine Teilmenge der Verbindungen der Formel (I).

Die Umsetzung nach Verfahren [A] erfolgt im Allgemeinen in inerten Lösungsmitteln, gegebenenfalls in Gegenwart einer Base, gegebenenfalls in einer Mikrowelle, bevorzugt in einem Temperaturbereich von 50°C bis 200°C bei Normaldruck bis 3 bar.

Basen sind beispielsweise beispielsweise Alkalicarbonate, wie z.B. Natrium-, Kalium- oder Caesiumcarbonat, oder organische Basen wie Trialkylamine, z.B. Triethylamin, *N*-Methylmorpholin, *N*-Methylpiperidin, 4-Dimethylaminopyridin oder Diisopropylethylamin, oder andere Basen wie beispielsweise Natriumhydrid oder Kalium-tert.-butylat, bevorzugt ist Diisopropylethylamin oder Natriumhydrid.

Inerte Lösungsmittel sind beispielsweise Halogenkohlenwasserstoffe wie Methylenchlorid oder Trichlormethan, Alkohole wie Methanol, Ethanol, n-Propanol oder Isopropanol, oder Ether wie Dioxan oder Tetrahydrofuran, oder andere Lösungsmittel wie beispielsweise Dimethylsulfoxid, Dimethylformamid oder N-Methylpyrrolidon, oder Gemische dieser Lösungsmittel, bevorzugt ist Isopropanol oder Dimethylsulfoxid.

Die Umsetzung nach Verfahren [B] erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)(1,3-dimesityl-1,3-dihydro-2H-imidazol-2-yliden)palladium oder Palladium(II)acetat/Dicyclohexyl-(2',4',6'-triisopropyl-biphenyl-2-yl)-phosphin. Als Palladiumquelle kann auch Tris(dibenzylidenaceton)dipalladium eingesetzt werden.

Zusatzreagenzien sind beispielsweise Kaliumacetat, Cäsium-, Kalium- oder Natriumcarbonat, Kalium-tert.-butylat, Cäsiumfluorid oder Kaliumphosphat durchgeführt, bevorzugt sind Zusatzreagenzien wie z.B. Kaliumacetat und/oder wässrige Natriumcarbonatlösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Dioxan oder Acetonitril oder ein Gemisch aus einem dieser Lösungsmittel mit Wasser.

Die Verbindungen der Formeln (II) und (IV) sind bekannt, lassen sich nach bekannten Verfahren aus den entsprechenden Ausgangsverbindungen synthetisieren oder können analog den im Beispielteil (Beispiel 3A bis 5A, Beispiel 9A, Beispiel 10A bis 12A und Beispiel 13A bis 16A) beschriebenen Verfahren oder analog zu J. Org. Chem. (2005), 70 (18), 7331-7337 und WO 03/000693 hergestellt werden.

Die Verbindungen der Formel (III) sind bekannt, lassen sich nach bekannten Verfahren aus den entsprechenden Ausgangsverbindungen synthetisieren oder können analog den im Beispielteil (Beispiel 1A bis 2A und Beispiel 6A bis 8A) beschriebenen Verfahren hergestellt werden.

Die Verbindungen der Formel (V) sind bekannt oder lassen sich nach bekannten Verfahren aus den entsprechenden Ausgangsverbindungen synthetisieren.

Die Herstellung der Ausgangsverbindungen und der Verbindungen der Formel (I) kann durch die folgenden Syntheseschemata verdeutlicht werden.

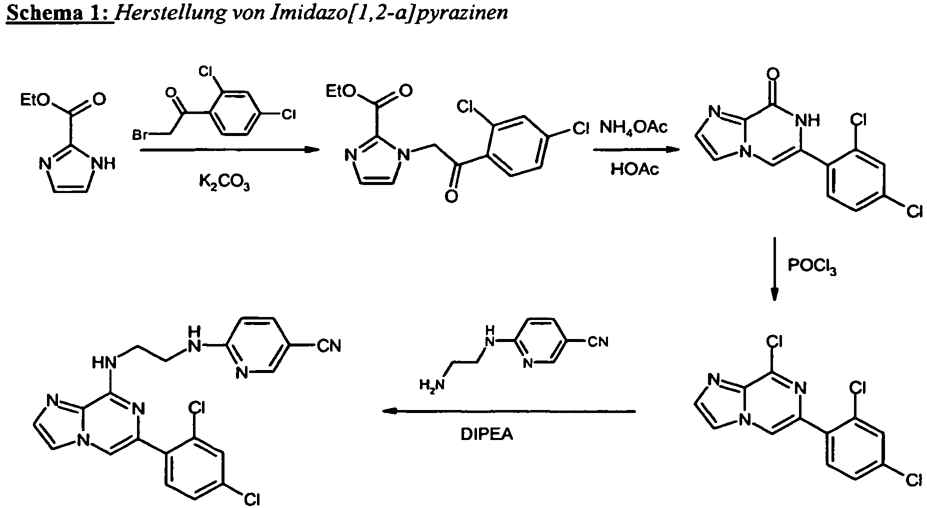

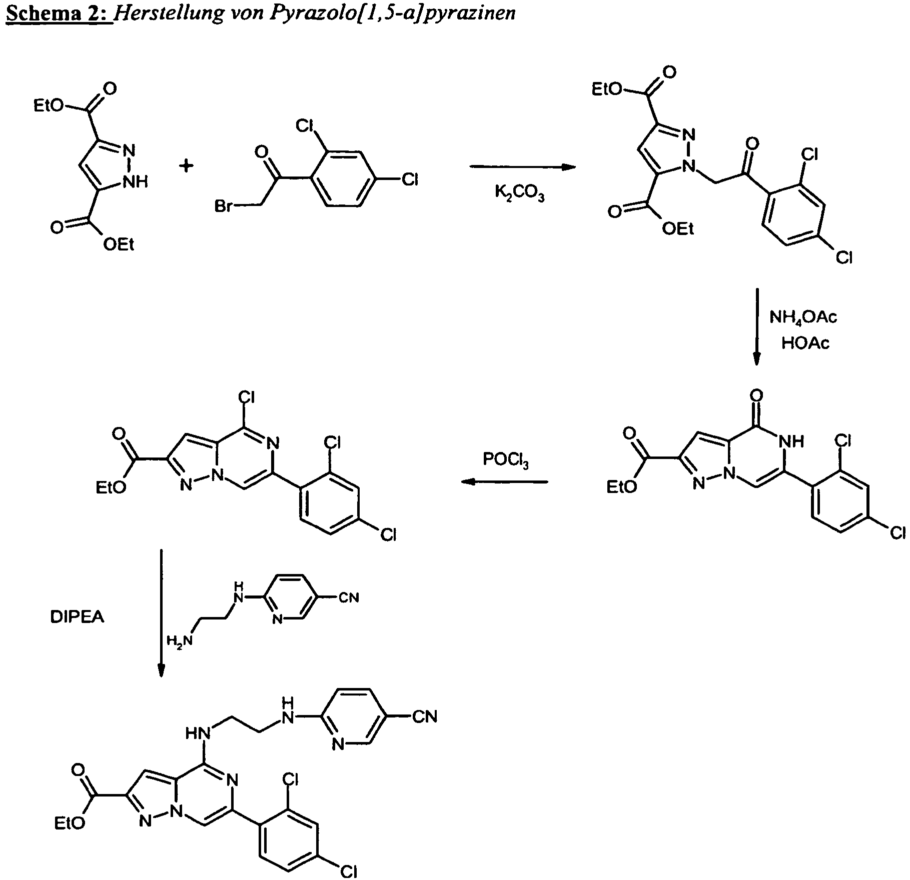

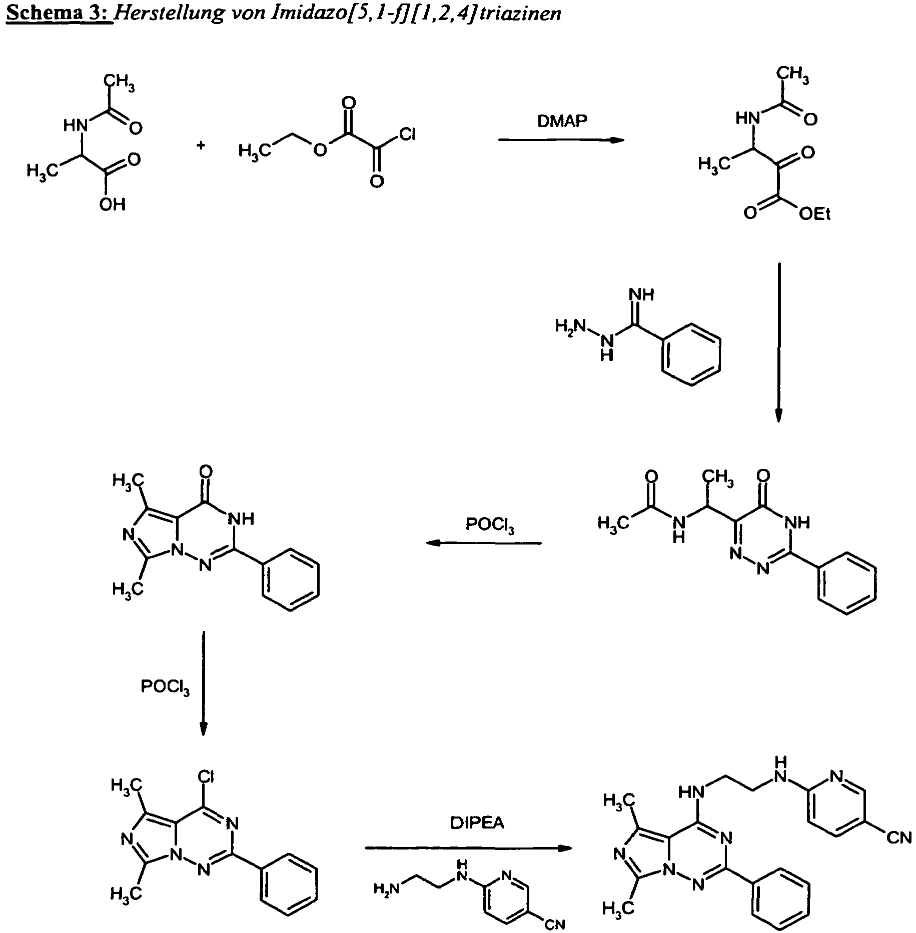

Die erfindungsgemäßen Verbindungen zeigen ein nicht vorhersehbares, wertvolles pharmakologisches und pharmakokinetisches Wirkspektrum.

Sie eigenen sich daher zur Verwendung als Arzneimittel zur Behandlung und/oder Prophylaxe von Krankheiten bei Menschen und Tieren.

Die erfindungsgemäßen Verbindungen eignen sich daher zur Prophylaxe und/oder Behandlung von neurodegenerativen Erkrankungen wie z.B. Alzheimer, Parkinson, Schizophrenie, Degeneration, Dementia, Depressionen; Aggression, zerebrovaskulär Ischämie, Schlafstörungen, Huntington-Chorea, neurotraumatische Erkrankungen wie z.B. Schlaganfall; Typ 2 Diabetes Mellitus und assoziierte Erkrankungen wie z.B. das metabolische Syndrom oder Fettleibigkeit, Typ 1 Diabetes Mellitus, Diabetische Nephrophatie, Diabetische Neurophatie, Diabetische Retinophatie, Glomerulonephritis, Hyperkalzämie, Hyperglykämie, Hyperlipidimie, Glukose-Galaktose-Malabsorption, allgemeine endokrine Dysfunktionen wie z.B. Pankreatitis; hämatologische Erkrankungen, wie zum Beispiel erworbene und angeborene Neutropenie, medikamentös induzierte Neutropenie, parasitär induzierte Neutropenie, Chemotherapie-induzierte Neutropenie, Granulozytopenie, erworbene und angeborene Leukopenie, erworbene und angeborene Anämie, hämolytische Anämie, Sichelzellenanämie, erworbene und angeborenen Thrombozytopenie, Leukozytenfunktionssörungen, Störungen der Blutgerinnung, ex vivo Vermehrung embryonaler und adulter Stammzellen, ex vivo Differenzierung embryonaler und adulter Stammzellen, Knochenmarks, Graft-versus-host-Reaktion; Krebs, wie zum Beispiel Glaukom, Mammakarzinom, Kolontumor, gastrointestinale Tumore, Hodgkin-Lymphom, Non- Hodgkin-Lymphom, Kaposisarkom, Lebertumor, Pankreastumor, Hauttumor, Knochenmarkstumor, Leukämien wie z.B. akute lymphatische Leukämie, akute myeloische Leukämie, chronische myeloische Leukämie, chronische lymphatische Leukämie, Prostatatumore, Lungenkrebs, Nierentumore; Asthma, progrediente, nicht vollständig reversible Obstruktion der Atemwege, Lungenentzündug, Lungenfehlfunktion; Entzündungserkrankungen wie z.B. Autoimmunerkrankungen wie Multiple Sklerose, rheumatoide Arthritis, Infektionen durch gram-negative und gram-positive Bakterien, virale Infektionen, Pilzinfektionen wie z. B. durch Candida albicans, HIV- und HIV-assoziierte Infektionen, Hepatitis der Typen A, B und C, parasitäre Infektionen; Haarausfall, verminderte Beweglichkeit von Spermien, Wundheilung; Osteoporose, Knochenmarkserkrankungen, Knochen- und Gelenkserkrankungen; Herzkreislauferkrankungen wie z.B. Herzfehler, Herzinsuffizienz, Herzfibrose, Herzrhythmusstörungen, Myokardinfarkt, Medikamenten- oder Substanzinduzierte Kardiotoxizität, Atherosklerose, Bluthochdruck.

Die erfindungsgemäßen Verbindungen eignen sich besonders zur Prophylaxe und/oder Behandlung von neurodegenerativen Erkrankungen wie z.B. Alzheimer und Schizophrenie, von Typ 2 Diabetes Mellitus und assoziierten Erkrankungen, von Krebs, von Leukopenien und/oder von Neutropenien.

Die erfindungsgemäßen Verbindungen können darüber hinaus auch zur effizienten *ex vivo* Vermehrung von adulten hämatopoetischen Stammzellen aus dem Knochenmark, aus peripherem Blut oder Nabelschnurblut eingesetzt werden.

Diese so expandierten Zellen können dann zur Verkürzung der durch myeloablative Therapien induzierten Zytopenien oder im Rahmen von therapeutischen Transplantationsverfahren oder bei hämatologischen Systemerkrankungen, wie z.B. Leukämien, oder mit nach der Expansion gentechnisch veränderter Zellen für Gentherapien verwendet werden.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind Arzneimittel, enthaltend eine erfindungsgemäße Verbindung und einen oder mehrere weitere Wirkstoffe.

Weiterer Gegenstand der vorliegenden Erfindung ist ein Verfahren zur ex vivo Vermehrung von adulten hämatopoetischen Stammzellen aus dem Knochenmark, aus peripherem Blut oder Nabelschnurblut, das dadurch gekennzeichnet ist, dass eine wirksame Menge der erfindungsgemäßen Verbindung zugegeben wird.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 500 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- abs.: absolut
- Boc: *tert*-Butoxycarbonyl
- CDCl₃: Deuterochloroform
- d: Tag
- DIEA: *N,N*-Diisopropylethylamin
- DMAP: 4-*N,N*-Dimethylaminopyridin
- DMF: Dimethylformamid
- DMSO: Dimethylsulfoxid
- d. Th.: der Theorie
- EDC: *N'*-(3-Dimethylaminopropyl)-*N*-ethylcarbodiimid x HCl
- eq.: Äquivalent
- ESI: Elektrospray-Ionisation (bei MS)
- ges.: gesättigt
- h: Stunde
- HOBt: 1-Hydroxy-1H-benzotriazol x H₂O
- HPLC: Hochdruck-, Hochleistungsflüssigchromatographie
- konz.: konzentriert
- LC-MS: Flüssigchromatographie-gekoppelte Massenspektrometrie
- min.: Minuten
- MS: Massenspektrometrie
- MW: Molekulargewicht [g/mol]
- NMR: Kernresonanzspektroskopie
- OAc: Acetat
- OEt: Ethoxy
- p.a.: zur Analyse
- PyBOP: 1-Benzotriazolyloxy-tripyrrolidinophosphoniumhexafluorophosphat
- R_{f}: Retentionsindex (bei DC)
- RP-HPLC: Reverse Phase HPLC
- RT: Raumtemperatur
- R,: Retentionszeit (bei HPLC)
- TBTU: (Benzotriazol-1-yloxy)bisdimethylaminomethyliumfluoroborat
- TFA: Trifluoressigsäure
- THF: Tetrahydrofuran

### LC-MS Methoden:

Methode 1: Instrument: Micromass Quattro LCZ mit HPLC Agilent Serie 1100; Säule: Phenomenex Synergi 2.5 µ MAX-RP 100A Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208- 400 nm.

Methode 2: Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%ige Ameisensäure / 1; Eluent B: Acetonitril + 500 µl 50%ige Ameisensäure / 1; Gradient: 0.0 min 10%B→ 7.0 min 95%B→ 9.0 min 95%B; Ofen: 35 °C; Fluss: 0.0 min 1.0 ml/min→ 7.0 min 2.0 ml/min→ 9.0 min 2.0 ml/min; UV-Detektion: 210 nm

Methode 3: Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 4: Instrument: Micromass Platform LCZ mit HPLC Agilent Serie 1100; Säule: Thermo Hypersil GOLD 3µ 20 mm x 4 mm; Eluent A: 1 l Wasser + 0.5 ml 50%ige Ameisensäure , Eluent B: 1l Acetonitril + 0.5 ml 50%ige Ameisensäure; Gradient: 0.0 min 100%A → 0.2 min 100%A → 2.9 min 30%A → 3.1 min 10%A →5.5 min 10%A; Ofen: 50°C; Fluss: 0.8 ml/min; UV-Detektion: 210 nm.

Methode 5: Gerätetyp MS: Waters ZQ; Gerätetyp HPLC: Waters Alliance 2795; Säule: Phenomenex Onyx Monolithic C18, 100 mm x 3 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 6: Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 l Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min;; Ofen: 50°C; UV-Detektion: 210 nm.

Methode 7: Instrument: Micromass Quattro LCZ mit HPLC Agilent Serie 1100; Säule: Phenomenex Onyx Monolithic C18, 100 mm x 3 mm. Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 8: Instrument: Micromass QuattroPremier mit Waters UPLC Acquity; Säule: Thermo Hypersil GOLD 1.9µ 50 mm x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 0.1 min 100%A → 1.5 min 10%A → 2.2 min 10%A; Ofen: 50°C; Fluss: 0.33 ml/min; UV-Detektion: 210 nm.

Methode 9: Instrument: Micromass Quattro Micro MS mit HPLC Agilent Serie 1100; Säule: Thermo Hypersil GOLD 3µ 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 3.0 min 10% A → 4.0 min 10%A → 4.01 min 100%A → 5.00 min 100%A; Fluss: 0.0 min/3.0 min/4.0 min/4.01 min 2.5 ml/min, 5.00 min 2 ml/min; Ofen: 50°C; UV-Detektion: 210 nm.

Methode 10: Instrument MS: Waters ZQ 2000; Instrument HPLC: Agilent 1100, 2-Säulen-Schaltung, Autosampler: HTC PAL; Säule: YMC-ODS-AQ, 50 mm x 4.6 mm, 3.0 µm; Eluent A: Wasser + 0.1% Ameisensaeure, Eluent B: Acetonitril + 0.1 % Ameisensaeure; Gradient: 0.0 min 100%A → 0.2 min 95%A → 1.8 min 25%A → 1.9 min 10%A → 2.0 min 5%A → 3.2 min 5%A → 3.21 min 100%A → 3.35 min 100%A; Ofen: 40°C; Fluss: 3.0 ml/min; UV-Detektion: 210 nm.

Methode 11: Instrument MS: Micromass TOF (LCT); Instrument HPLC: Waters 2690, Autosampler: Waters 2700; Säule: YMC-ODS-AQ, 50 mm x 4.6 mm, 3.0 µm; Eluent A: Wasser + 0.1% Ameisensaeure, Eluent B: Acetonitril + 0.1% Ameisensaeure; Gradient: 0.0 min 100%A - 0.2 min 95%A → 1.8 min 25%A → 1.9 min 10%A → 2.0 min 5%A → 3.2 min 5%A → 3.21 min 100%A → 3.35 min 100%A; Ofen: 40°C; Fluss: 3.0 ml/min; UV-Detektion: 210 nm.

### Ausgangsverbindungen

### Beispiel 1A

### tert-Butyl-{2-[(5-cyanopyridin-2-yl)amino]ethyl}carbamat

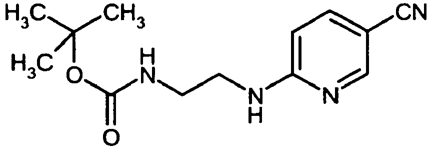

5.5 g (39.7 mmol) 6-Chlornicotinsäurenitril wurden in 70 ml DMSO gelöst, und mit 10.2 g (63.5 mmol) N-Boc-Ethylendiamin und 11 g (79.4 mmol) Kaliumcarbonat versetzt. Es wurde 12 h bei 90°C nachgerührt. Der Rückstand wurde in einem Gemisch von Wasser und Essigsäureethylester aufgenommen. Die organische Phase wurde mit gesättigter wässriger Natriumchloridlösung gewaschen, über Magnesiumsulfat getrocknet und am Rotationsverdampfer eingeengt. Der Rückstand wurde an Kieselgel 60 chromatographiert (Laufmittel: Cyclohexan /Essigsäureethylester 10:1 bis 2:1). Es wurden 7.9 g (77% d. Th.) des Produktes als Feststoff erhalten.
LCMS (Methode 6): Rₜ = 1.46 min. (m/z = 263 (M+H)⁺)
¹H-NMR (400MHz, DMSO-d₆): δ = 8.37 (d, 1H), 7.66 (d, 1H) 7.6 (s, 1H), 6.87 (t, 1H), 6.53 (d, 1H), 3.32 (q, 2H), 3.09 (q, 2H), 1.37 (s, 9H).

### Beispiel 2A

### 6-[(2-Aminoethyl)amino]nicotinnitril Dihydrochlorid

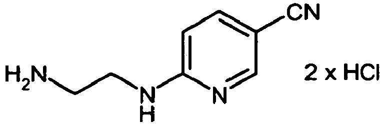

7.9 g (30 mmol) tert-Butyl-{2-(5-cyanopyridin-2-yl)amino]ethyl}carbamat (Beispiel 1A) wurden in 100 ml 4N Chlorwasserstoff in Dioxan gelöst und 30 min nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 7 g (94% d. Th.) des Produktes als Feststoff erhalten.
LCMS (Methode 4): Rₜ = 0.51 min. (m/z = 162 (M+H)⁺)
¹H-NMR (400MHz, DMSO-d₆): δ = 8.44 (s, 1H), 7.76 (d, 1H), 6.67 (d, 1H), 3.58 (t, 2H), 2.98 (q, 2H).

### Beispiel 3A

### 1-[2-(2,4-Dichlorphenyl)-2-oxoethyl]-1H-imidazol-2-carbonsäureethylester

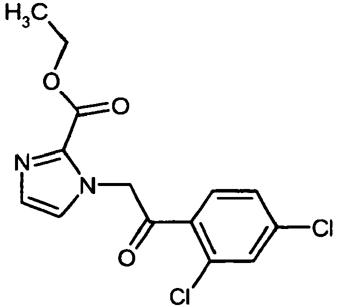

0.5 g (3.6 mmol) Imidazol-2-carbonsäureethylester wurden in 35 ml Aceton gelöst, und mit 0.96 g (3.6 mmol) 2-Brom-2,4-dichloracetophenon und 0.49 g (3.6 mmol) Kaliumcarbonat versetzt. Es wurde 12 h bei RT nachgerührt. Das Reaktionsgemisch wurde eingeengt, und in Wasser und Dichlormethan aufgenommen. Die organische Phase wurde mit gesättigter wässriger Natriumchloridlösung gewaschen, über Magnesiumsulfat getrocknet und am Rotationsverdampfer eingeengt. Der Rückstand wurde mit Diethylether verrührt und der Feststoff abgesaugt. Es wurden 0.9 g (77% d. Th.) des Produktes als Feststoff erhalten.
LCMS (Methode 1): Rₜ = 1.19 min. (m/z = 327 (M+H)⁺)
¹H-NMR (400MHz, DMSO-d₆): δ = 7.96 (d, 1H), 7.84 (d, 1H), 7.68 (dd, 1H), 7.5 (s, 1H), 7.16 (s, 1H), 5.87 (s, 2H), 4.22 (q, 2H), 3.32 (s, 2H), 1.23 (t, 3H).

### Beispiel 4A

### 6-(2,4-Dichlorphenyl)imidazo[1,2-a]pyrazin-8(7H)-on

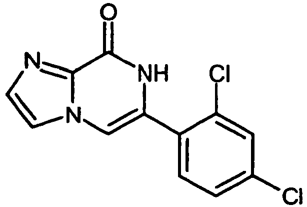

920 mg (2.8 mmol) 1-[2-(2,4-Dichlorphenyl)-2-oxoethyl]-1H-imidazol-2-carbonsäureethylester (Beispiel 3A) wurden in 45 ml Eisessig gelöst und mit 2.17 g (28 mmol) Ammoniumacetat versetzt. Es wurde 12 h bei Rückfluß nachgerührt. Das Reaktionsgemisch wurde auf Eiswasser gegeben und mit Natriumcarbonat neutralisiert. Der Niederschlag wurde abfiltriert und am Hochvakuum nachgetrocknet. Es wurden 650 mg (82% d. Th.) des Produktes als Feststoff erhalten.
LCMS (Methode 3): Rₜ = 1.79 min. (m/z = 281 (M+H)⁺)
¹H-NMR (400MHz, DMSO-d₆): δ = 11.5 (s, 1H), 7.83 (s, 1H) 7.81 (d, 1H), 7.63 (s, 1H), 7.61 (s, 1H), 7.58 (d, 1H), 7.52 (s, 1H).

### Beispiel 5A

### 8-Chlor-6-(2,4-dichlorphenyl)imidazo[1,2-a]pyrazin

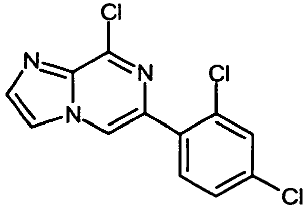

650 mg (2.3 mmol) 6-(2,4-Dichlorphenyl)imidazo[1,2-a]pyrazin-8(7H)-on (Beispiel 4A) wurden in 7 ml Phosphorylchlorid gelöst und 12 h bei Rückfluß nachgerührt. Das Reaktionsgemisch wurde auf 100 ml gesättigter Natriumhydrogencarbonat-Lösung gegossen und solange mit festem Natriumhydrogencarbonat versetzt bis der pH-Wert 7 erreicht wurde. Es wurde Essigsäureethylester zugegeben und extrahiert. Die organische Phase wurde mit gesättigter wässriger Natriumchloridlösung gewaschen, über Magnesiumsulfat getrocknet und am Rotationsverdampfer eingeengt. Zum Rückstand wurde Methanol gegeben, der ausfallende Feststoff wurde abfiltriert, und mit Methanol sowie Diethylether nachgewaschen und am Hochvakuum getrocknet. Es wurden 400 mg (58% d. Th.) des Produktes als Feststoff erhalten.
LCMS (Methode 3): Rₜ = 2.43 min. (m/z = 299 (M+H)⁺)
¹H-NMR (400MHz, DMSO-d₆): δ = 9.01 (s, 1H), 8.34 (s, 1H) 7.93 (s, 1H), 7.81 (d, 1H), 7.72 (d, 1H), 7.6 (dd,1H).

### Beispiel 6A

### 4-Amino-2-(methylsulfonyl)-1,3-thiazol-5-carbonitril

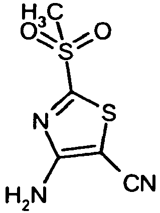

2.7 g (15.8 mmol) 4-Amino-2-(methylsulfanyl)-1,3-thiazol-5-carbonitril wurden in 500 ml Dichlormethan gelöst und mit 12 g (34.7 mmol) 50%-iger 3-Chlorperbenzoesäure versetzt. Es wurde 30 min bei RT nachgerührt. Das Reaktionsgemisch wurde mit 6 ml DMSO versetzt und mit gesättigter Natriumhydrogencarbonat-Lösung, sowie Wasser gewaschen. Die organische Phase wurde über Magnesiumsulfat getrocknet und am Rotationsverdampfer eingeengt. Es wurden 2.2 g (46% d. Th.) des Produktes als Feststoff erhalten.
LCMS (Methode 4): Rₜ = 1.19 min. (m/z = 204 (M+H)⁺).

### Beispiel 7A

### tert-Butyl-{2-[(4-amino-5-cyano-1,3-thiazol-2-yl)amino]ethyl}carbamat

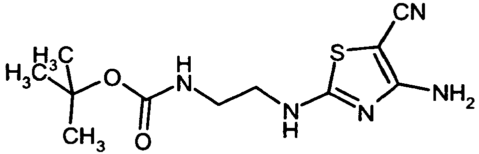

3.25 g (16 mmol) 4-Amino-2-(methylsulfonyl)-1,3-thiazol-5-carbonitril (Beispiel 6A) wurden in 50 ml DMSO gelöst und mit 3.8 g (24 mmol) N-Boc-Ethylendiamin, sowie 2.8 ml (16 mmol) N,N-Diisopropytethylamin versetzt. Es wurde 12 h bei 120°C nachgerührt. Das Reaktionsgemisch wurde in einem Gemisch von Essigsäureethylester und Wasser aufgenommen. Die organische Phase wurde mit gesättigter wässriger Natriumchloridlösung gewaschen, über Magnesiumsulfat getrocknet und am Rotationsverdampfer eingeengt. Das Rohprodukt wurde in Acetonitril verrührt und die ausfallende Kristalle wurden abgesaugt. Es wurden 2.5 g (55 % d. Th.) des Produktes als Feststoff erhalten.
LCMS (Methode 4): Rₜ = 1.81 min. (m/z = 284 (M+H)⁺).
¹H-NMR (400MHz, DMSO-d₆): δ = 8.35 (s, 1H), 6.9 (t, 1H), 6.68 (s, 2H), 3.22 (q, 2H), 3.07 (q, 2H), 1.36 (s, 9H).

### Beispiel 8A

### 4-Amino-2-[(2-aminoethyl)amino]-1,3-thiazol-5-carbonitril Dihydrochlorid

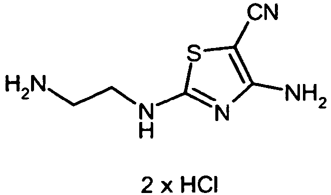

2.18 g (7.7 mmol) tert-Butyl-{2-[(4-Amino-5-cyano-1,3-thiazol-2-yl)amino]ethyl}carbamat (Beispiel 7A) wurden in 100 ml 4N Chlorwasserstoff in Dioxan 30 min bei RT gerührt. Das Reaktionsgemisch wurde eingeengt. Es wurden 2 g (100 % d. Th.) des Produktes als Feststoff erhalten.
LCMS (Methode 6): Rₜ = 0.19 min. (m/z = 183 (M+H)⁺).
¹H-NMR (400MHz, DMSO-d₆): δ = 3.46 (d, 2H), 3.0 (d, 2H).

### Beispiel 9A

### 4-Amino-2-({2-[(6-bromimidazo[1,2-a]pyrazin-8-yl)amino]ethyl}amino)-1,3-thiazol-5-carbonitril

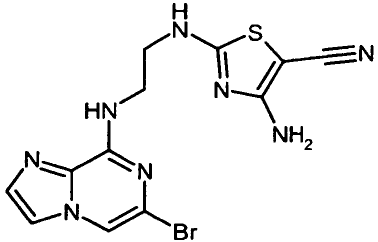

300 mg (1.08 mmol) 6,8-Dibromoimidazo[1,2-a]Pyrazin wurden in 10 ml DMSO gelöst und mit 0.6 ml (4.3 mmol) Triethylamin, sowie 360 mg (1.3 mmol) 4-Amino-2-[(2-aminoethyl)amino]-1,3-thiazol-5-carbonitril Dihydrochlorid (Beispiel 8A) versetzt. Es wurde bei 140°C für 1 h in der Mikrowelle erhitzt. Man erhielt nach Reinigung mittels präparativer HPLC 376 mg (83% d. Th.) des Produktes als Feststoff.
LCMS (Methode 8): Rₜ = 0.88 min. (m/z = 381 (M+H)⁺).
¹H-NMR (400MHz, DMSO-d₆): δ = 8.46 (t, 1H), 8.12 (t, 1H), 8.05 (s, 1H), 7.85 (s, 1H), 7.53 (s, 1H), 3.6 (d, 2H), 3.48 (d, 2H).

### Beispiel 10A

### 1-[2-(2,4-Dichlorphenyl)-2-oxoethyl]-1H-pyrazol-3,5-dicarbonsäurediethylester

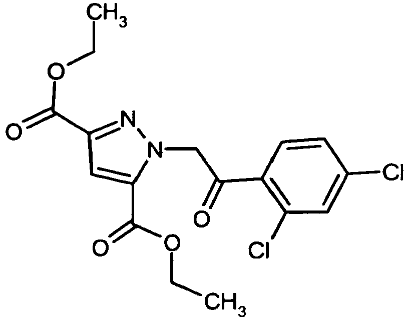

5 g (23.6 mmol) 1H-Pyrazol-3,5-dicarbonsäurediethylester wurden in 100 ml Aceton gelöst, mit 6.3 g (23.6 mmol) 2-Brom-2,4-dichloracetophenon und 3.6 g (26 mmol) Kaliumcarbonat versetzt. Es wurde 12 h bei RT nachgerührt. Das Reaktionsgemisch wurde eingeengt, und in Wasser und Dichlormethan wieder aufgenommen. Die organische Phase wurde mit gesättigter wässriger Natriumchloridlösung gewaschen, über Magnesiumsulfat getrocknet und am Rotationsverdampfer eingeengt. Der Rückstand wurde mit Diethylether verrührt und der Feststoff wurde abgesaugt. Es wurden 9.48 g (94% d. Th.) des Produktes als Feststoff erhalten.
LCMS (Methode 6): Rₜ = 2.34 min. (m/z = 399 (M+H)⁺)
¹H-NMR (400MHz, DMSO-d₆): δ = 8.02 (d, 1H), 7.84 (s, 1H), 7.68 (dd, 1H), 7.35 (s, 1H), 6.13 (s, 2H), 4.32 (q, 2H), 4.26 (q, 2H), 1.31 (t, 3H), 1.25 (t, 3H).

### Beispiel 11A

### 6-(2,4-Dichlorphenyl)-4-oxo-4,5-dihydropyrazolo[1,5-a]pyrazin-2-carbonsäureethylester

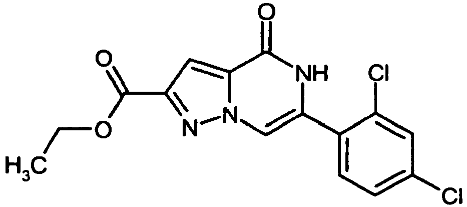

9.5 g (23.7 mmol) 1-[2-(2,4-Dichlorphenyl)-2-oxoethyl]-1H-pyrazol-3,5-dicarbonsäurediethylester (Beispiel 10A) wurden in 300 ml Eisessig gelöst und mit 18.3 g (237 mmol) Ammoniumacetat versetzt. Es wurde 12 h bei Rückfluß nachgerührt. Das Reaktionsgemisch wurde auf Eiswasser gegeben und mit Natriumcarbonat neutralisiert. Der Niederschlag wurde abfiltriert und am Hochvakuum nachgetrocknet. Es wurden 6.86 g (82% d. Th.) des Produktes als Feststoff erhalten.
LCMS (Methode 3): Rₜ = 2.31 min (m/z = 354 (M+H)⁺)
¹H-NMR (400MHz, DMSO-d₆): δ = 11.85 (s, 1H), 7.98 (s, 1H) 7.83 (d, 1H), 7.64 (d, 1H), 7.59 (dd, 1H), 7.44 (s, 1H), 4.34 (q, 2H), 1.33 (t, 3H).

### Beispiel 12A

### 4-Chlor-6-(2,4-dichlorphenyl)pyrazolo[1,5-a]pyrazin-2-carbonsäureethylester

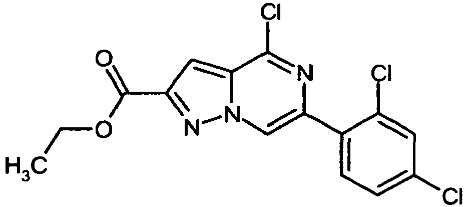

6.86 g (19.5 mmol) 6-(2,4-Dichlorphenyl)-4-oxo-4,5-dihydropyrazolo[1,5-a]pyrazin-2-carbon-säureethylester (Beispiel 11A) wurden in 50 ml Phosphorylchlorid gelöst und 12 h bei Rückfluß nachgerührt. Das Reaktionsgemisch wurde auf 1.25 l gesättigte Natriumhydrogencarbonat-Lösung gegossen und solange mit festem Natriumhydrogencarbonat versetzt bis der pH-Wert 7 erreicht wurde. Der Feststoff wurde abfiltriert und in Dichlormethan gelöst und an Kieselgel chromatographiert (Laufinittel Cyclohexan/Essigsäureethylester 10:1). Es wurden 6.34 g (85% d. Th.) des Produktes als Feststoff erhalten.
LCMS (Methode 3): Rₜ = 3.15 min. (m/z = 371 (M+H)⁺).
¹H-NMR (400MHz, DMSO-d₆): δ = 9.3 (s, 1H), 7.84 (d, 1H) 7.73 (d, 1H), 7.62 (dd, 1H), 7.56 (s, 1H), 4.4 (q, 2H), 1.36 (t, 3H).

### Beispiel 13A

### 3-Acetamido-2-oxobutansäureethylester

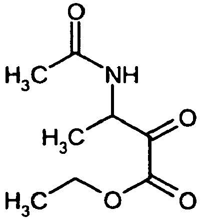

52 g (396 mmol) Acetylalanin wurden in 800 ml THF gelöst, mit 45 mg (0.4 mmol) 4-Dimethylaminopyridin und 96 ml (1188 mmol) Pyridin versetzt. Es wurde auf Rückfluß erhitzt und über 45 min 88 ml (792 mmol) Ethyloxalylchlorid zugetropft. Es wurde 3 h bei Rückfluß nachgerührt. Das Reaktionsgemisch wurde auf Eiswasser gegeben, es wurde Essigsäureethylester zugegeben und extrahiert. Die organische Phase wurde mit gesättigter wässriger Natriumchloridlösung gewaschen, getrocknet über Natriumsulfat und am Rotationsverdampfer eingeengt. Das Rohgemisch wurde ohne weitere Aufreinigung weiter umgesetzt.
¹H-NMR (400MHz, CDCl₃): δ = 10.86 (s, 1H), 4.42 (q, 1H) 4.23 (q, 2H), 2.16 (s, 3H), 1.42 (t, 3H), 1.29 (t, 3H).

### Beispiel 14A

### N-[1-(5-oxo-3-phenyl-4,5-dihydro-1,2,4-triazin-6-yl)ethyl]acetamid

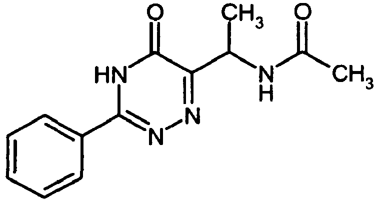

48 g (306 mmol) Benzolcarboximidamid-Hydrochlorid wurden in 1.2 1 Ethanol gelöst und mit 16.4 ml (337 mmol) Hydrazinhydrat versetzt. Es wurde 3 h bei 45°C nachgerührt. 86 g (459 mmol) 3-Acetamido-2-oxobutansäureethylester (Beispiel 13A), in 600 ml Ethanol gelöst, wurden zugetropft. Es wurde 6 h bei 80°C und 12 h bei RT nachgerührt. Man chromatographierte das Reaktionsgemisch an Kieselgel (Laufmittel Dichlormethan/Methanol 20:1). Es wurden 23.2 g (26% d. Th.) des Produktes als Feststoff erhalten.
¹H-NMR (400MHz, CDCl₃): δ = 8.21 (d, 2H), 7.56 (m, 3H) 5.36 (q, 1H), 2.05 (s, 3H), 1.55 (d, 3H).

### Beispiel 15A

### 5,7-Dimethyl-2-phenylimidazo[5,1-f][1,2,4]triazin-4(3H)-on

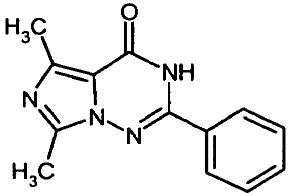

8.34 g (32.3 mmol) N-[1-(5-Oxo-3-phenyl-4,5-dihydro-1,2.4-triazin-6-yl)ethyl]acetamid] (Beispiel 14A) wurden in 330 ml 1,2-Dichlorethan gelöst und mit 4.5 ml (48.5 mmol) Phosphorylchlorid versetzt. Es wurde 24 h bei Rückfluß nachgerührt. Nach Abkühlung filtrierte man den Niederschlag ab und wusch mit Wasser und Diethylether nach. Das Produkt wurde am Hochvakuum nachgetrocknet. Es wurden 4.6 g (59% d. Th.) des Produktes als Feststoff erhalten.
¹H-NMR (400MHz, DMSO-d₆): δ = 12.45 (s, 1H), 8.08 (d, 2H), 7.6 (m, 3H), 2.69 (s, 3H), 2.59 (s, 3H).

### Beispiel 16A

### 4-Chlor-5,7-dimethyl-2-phenylimidazo[5,1-f][1,2,4]triazin

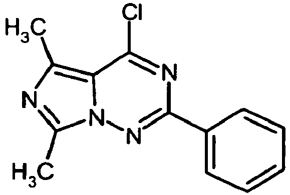

700 mg (2.9 mmol) 5,7-Dimethyl-2-phenylimidazo[5,1-f][1,2,4]triazin-4(3H)-on (Beispiel 15A) wurden in 8 ml Phosphorylchlorid gelöst und 12 h bei Rückfluß nachgerührt. Das Reaktionsgemisch wurde auf 170 ml gesättigte Natriumhydrogencarbonat-Lösung gegossen und solange mit festem Natriumhydrogencarbonat versetzt bis der pH-Wert 7 erreicht wurde. Man gab Essigsäureethylester zu und extrahiert. Die organische Phase wurde mit gesättigter wässriger Natriumchloridlösung gewaschen, über Magnesiumsulfat getrocknet und am Rotationsverdampfer eingeengt. Es wurden 657 mg (80% d. Th.) des Produktes als Feststoff erhalten.
LCMS (Methode 6): Rₜ = 2.17 min. (m/z = 259 (M+H)⁺).
¹H-NMR (400MHz, DMSO-d₆): δ = 8.26 (dd, 2H), 7.58 (m, 3H), 2.68 (s, 3H), 2.66 (s, 3H).

### Beispiel 17A

### 6-(2,4-Dichlorphenyl)-2-(hydroxymethyl)pyrazolo[1,5-a]pyrazin-4(5H)-on

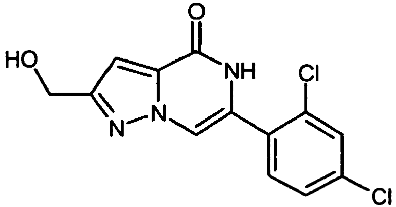

In 700 ml THF wurden 5 g (12.55 mmol) 6-(2,4-Dichlorphenyl)-4-oxo-4,5-dihydropyrazolo[1,5-a]pyrazin-2-carbonsäureethylester (Beispiel 11A) gelöst und bei RT 952 mg (25.1 mmol) Lithiumaluminiumhydrid zugegeben. Man rührte für 2 h bei RT und gab nach vollständigem Umsatz zuerst Methanol zu und stellte dann mit verdünnter Salzsäure pH=6 ein. Es wurde mehrmals mit Essigsäureethylester extrahiert. Nach Entfernen des Lösungsmittels wurden 3.12 g (80% d. Th.) des Produktes als Feststoff erhalten.
LCMS (Methode 3): Rₜ = 1.66 min. (m/z = 310 (M+H)⁺).
¹H-NMR (400MHz, DMSO-d₆): δ = 11.55 (s, 1H), 7.81 (d, 1H), 7.77 (s, 1H), 7.62 (d, 1H), 7.66 (dd, 1H), 6.94 (s, 1H), 5.32 (t, 1H), 4.58 (d, 2H).

### Beispiel 18A

### 6-(2,4-Dichlorphenyl)-4-oxo-4,5-dihydropyrazolo[1,5-a]pyrazin-2-carbaldehyd

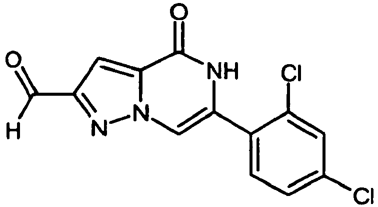

1.15 g (3.37 mmol) 6-(2,4-Dichlorphenyl)-2-(hydroxymethyl)pyrazolo[1,5-a]pyrazin-4(5H)-on (Beispiel 17A) wurden in 100 ml Dichlormethan suspendiert und 1 Tropfen Wasser wurde zugegeben. Daraufhin wurden 100 ml 1,2-Dimethoxyethan und 9.6 ml DMF zugegeben und auf 0°C abgekühlt. Es wurden 7.16 g (16.87 mmol) Dess-Martin-Periodinan zugegeben, die Eisbadkühlung entfernt und 15 h bei RT gerührt. Der Ansatz wurde mit Essigsäureethylester versetzt, 200 ml einer 10%igen Natriumthiosulfatlösung wurden zugegeben und die organische Phase wurde mit gesättigter wässriger Natriumhydrogencarbonat-Lösung gewaschen. Nach Trocknung der organischen Phase mit Natriumsulfat und Entfernen des Lösungsmittels im Vakuum erhielt man 660 mg (57% d. Th.) des Produktes als Feststoff, der ohne weitere Aufreinigung umgesetzt wurde.
LCMS (Methode 8): Rₜ = 1.04 min. (m/z = 308 (M+H)⁺).
¹H-NMR (400MHz, DMSO-d₆): δ = 11.89 (s, 1H), 10.07 (s, 1H), 8.02 (s, 1H), 7.84 (d, 1H), 7.66 (d, 1H), 7.60 (dd, 1H), 7.52 (s, 1H).

### Beispiel 19A

### 6-(2,4-Dichlorphenyl)-2-[(4-methylpiperazin-1-yl)methyl]pyrazolo[1,5-a]pyrazin-4(5H)-on

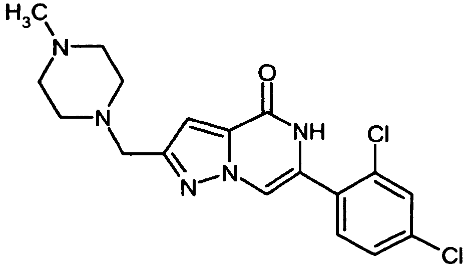

145 mg (0.32 mmol) 6-(2,4-Dichlorphenyl)-4-oxo-4,5-dihydropyrazolo[1,5-a]pyrazin-2-carbaldehyd (Beispiel 18A) wurden in 6 ml Methanol gelöst und 64 mg (0.64 mmol) 1-Methylpiperazin, 4Å Molsieb und 58 mg (0.96 mmol) Essigsäure wurden zugegeben. Zuletzt gab man 40.2 mg (0.64 mmol) Natriumcyanoborhydrid zu und rührte für 15 h bei RT. Der Rohansatz wurde mit 2N Salzsäure angesäuert und der ausfallende Niederschlag abgesaugt. Es wurden 120 mg (96% d. Th.) des Produktes als Feststoff erhalten, der ohne weitere Aufreinigung weiter umgesetzt wurde.
LCMS (Methode 6): Rₜ = 0.76 min. (m/z = 392 (M+H)⁺).
¹H-NMR (400MHz, DMSO-d₆): δ = 11.63 (s, 1H), 7.82 (d, 1H), 7.81 (s, 1H), 7.61 (d, 1H), 7.57 (dd, 1H), 7.0 (s, 1H), 3.7-4.0 (m, 4H), 3.38 (m, 2H), 3.04 (m, 4H), 2.78 (s, 3H).

### Beispiel 20A

### 4-Chlor-6-(2,4-dichlorphenyl)-2-[(4-methylpiperazin-1-yl)methyl]pyrazolo[1,5-a]pyrazin

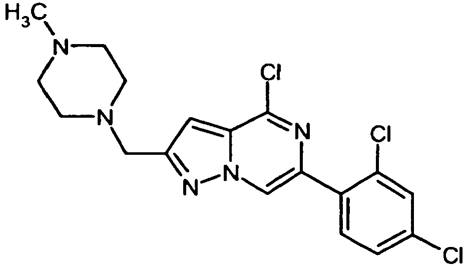

Analog der Herstellung von Beispiel 16A wurden aus 160 mg (0.34 mmol) 6-(2,4-Dichlorphenyl)-2-[(4-methylpiperazin-1-yl)methyl]pyrazolo[1,5-a]pyrazin-4(5H)-on durch Umsetzung mit Phosphorylchlorid 108 mg (70% d. Th.) des Produktes als Feststoff erhalten.
LCMS (Methode 6): Rₜ = 0.76 min. (m/z = 392 (M+H)⁺).
¹H-NMR (400MHz, DMSO-d₆): δ = 9.12 (s, 1H), 7.81 (d, 1H), 7.71 (d, 1H), 7.60 (dd, 1H), 6.99 (s, 1H), 3.74 (s, 2H), 2.4-2.55 (m, 4H), 2.25-2.4 (m, 4H), 2.15 (s, 3H).

### Beispiel 21A

### 6-(2,4-Dichlorphenyl)-2-(morpholin-4-ylmethyl)pyrazolo[1,5-a]pyrazin-4(5H)-on Trifluoracetat

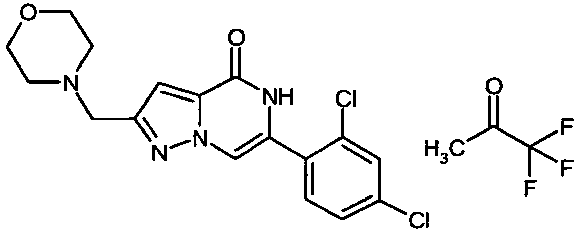

760 mg (2.17 mmol) 6-(2,4-Dichlorphenyl)-4-oxo-4,5-dihydropyrazolo[1,5-a]pyrazin-2-carbaldehyd (Beispiel 18A) wurden in 46 ml Methanol gelöst und 378 mg (4.34 mmol) Mopholin, 4Å Molsieb und 0.373 ml (6.51 mmol) Essigsäure wurden zugegeben. Zuletzt gab man 272.8 mg (4.34 mmol) Natriumcyanoborhydrid zu und rührte für 15 h bei RT. Der Rohansatz wurde mit 2N Salzsäure angesäuert und der ausfallende Niederschlag abgesaugt. Nach Aufreinigung des Niederschlags mittels präparativer HPLC (Laufmittel: Gradient Acetonitril/Wasser mit 0.1% Trifluoressigsäure) wurden 693 mg (65% d. Th.) des Produktes als Feststoff erhalten.
LCMS (Methode 8): Rₜ = 0.75 min. (m/z = 379 (M+H)⁺).
¹H-NMR (400MHz, DMSO-d₆): δ = 11.79 (s, 1H), 10.31 (s, br, 1H), 7.91 (s, 1H), 7.84 (d, 1H), 7.64 (d, 1H), 7.59 (dd, 1H), 7.22 (s, 1H), 4.54 (s, 2H), 3.96 (m, 2H), 3.64 (m, 2H), 3.39 (m, 2H), 3.18 (m, 2H).

### Beispiel 22A

### 4-Chlor-6-(2,4-dichlorphenyl)-2-(morpholin-4-ylmethyl)pyrazolo[1,5-a]pyrazin Hydrochlorid

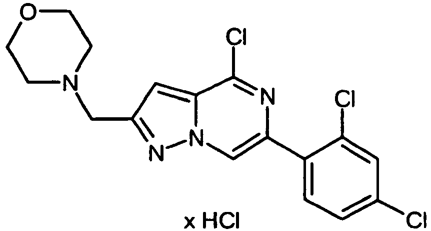

Analog der Herstellung von Beispiel 16A wurden aus 690 mg (1.18 mmol) 6-(2,4-Dichlorphenyl)-2-(morpholin-4-ylmethyl)pyrazolo[1,5-a]pyrazin-4(5H)-on Trifluoracetat durch Umsetzung mit Phosphorylchlorid 395 mg (85% d. Th.) des Produktes als Feststoff erhalten.
LCMS (Methode 6): Rₜ = 1.32 min. (m/z = 397 (M+H)⁺).
¹H-NMR (400MHz, DMSO-d₆): δ = 11.50 (s, 1H), 9.23 (s, 1H), 7.84 (d, 1H), 7.72 (d, 1H), 7.62 (dd, 1H), 7.44 (s, br, 1H), 4.65 (s, br, 2H), 3.9-4.0 (m, 2H), 3.7-3.85 (m, 2H), 3.3-3.45 (m, 2H), 3.1-3.25 (m, 2H).

### Beispiel 23A

### 6-({2-[(6-Bromimidazo[1,2-a]pyrazin-8-yl)amino]ethyl}amino)pyridin-3-carbonitril

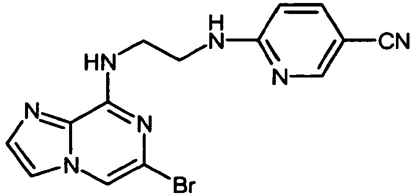

8 g (28.9 mmol) 6,8-Dibromimidazo[1,2-a]pyrazin wurden in 80 ml DMSO gelöst und mit 16.1 ml (115.6 mmol) Triethylamin, sowie 5.154 g (31.78 mmol) 6-[(2-Aminoethyl)amino]pyridin-3-carbonitril (Beispiel 2A) versetzt. Es wurde bei 140°C für 1.5 h in der Mikrowelle erhitzt. Die Lösung wurde auf Wasser gegossen und der Niederschlag abfiltriert. Nach Waschen mit Wasser und Trocknen im Hochvakuum wurden 10.3 g (94% d. Th.) des Produktes als Feststoff erhalten.
LCMS (Methode 8): Rₜ = 0.97 min. (m/z = 358 (M+H)⁺).
¹H-NMR (400MHz, DMSO-d₆): δ = 8.39 (s, 1H), 8.08 (s, br, 1H), 8.03 (s, 1H), 7.83 (s, 1H), 7.73 (m, 2H), 7.51 (s, 1H), 6.56 (s, br, 1H), 3.5-3.65 (m, 4H).

### Beispiel 24A

### tert-Butyl-(6-chlorpyridin-2-yl)carbamat

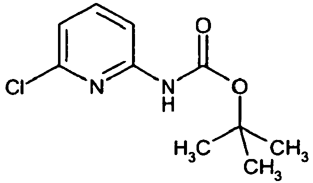

23.4 g (181.8 mmol) 2-Chlor-5-aminopyridin wurden unter Argon mit 150 ml THF versetzt und auf 0°C gekühlt. Es wurden 73.3 g (400 mmol) Bis-(trimethylsilyl)-natriumamid und 43.65 g (200 mmol) Di-tert-butyldicarbonat, gelöst in 150 ml THF, zugetropft. Nach 15 min wurde das Kühlbad entfernt und weitere 15 min bei RT nachgerührt. Das THF wurde abrotiert und der Rückstand mit Essigsäureethylester und 0.5 N Salzsäure versetzt und extrahiert. Die organische Phase wurde abgetrennt, über Magnesiumsulfat getrocknet und am Rotationsverdampfer eingeengt. Man chromatographierte das Reaktionsgemisch an Kieselgel (Laufmittel Dichlormethan/Methanol 100% → 100:3). Es wurden 36.54 g (88% d. Th.) des Produktes als Feststoff erhalten.
LCMS (Methode 3): Rₜ = 2.41 min. (m/z = 175 (M+H)⁺).
¹H-NMR. (400MHz, DMSO-d₆): δ = 10.11 (s, 1H), 7.78 (d, 2H), 7.1 (t, 1H), 1.47 (s, 9H).

### Beispiel 25A

### tert-Butyl-(6-chlor-3-formylpyridin-2-yl)carbamat

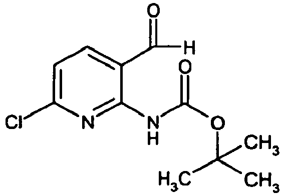

Die Reaktionsapparatur wurde ausgeheizt, und die Reaktion erfolgte unter Argon und wurde gerührt. 15 g (65.6 mmol) tert-Butyl-(6-chlorpyridin-2-yl)carbamat (Beispiel 24A) und 19 g (164 mmol) 1,2-Bis(dimethylamino)ethan wurden in 270 ml THF vorgelegt und auf -78°C abgekühlt. Es wurden 102.5 ml (164 mmol) Butyllithium (1.6N) zugetropft. Nach Beendigung des Zutropfens wurde die Reaktion langsam auf -10°C erwärmt und bei -10°C für 2 h gehalten. Dann wieder auf -78°C abgekühlt, und es wurden 10 ml (131 mmol) DMF dazugegeben. Die Reaktion wurde langsam auf RT erwärmt und das Reaktionsgemisch wurde auf 1 l Essigsäureethylester und 350 ml 1N Salzsäure gegeben, 15 min nachgerührt und die organische Phase wurde abgetrennt. Es wurde mit Wasser und gesättigter Natriumhydrogencarbonat-Lösung gewaschen, über Magnesiumsulfat getrocknet und am Rotationsverdampfer eingeengt. Der Rückstand wurde mit Diethylether versetzt und der Feststoff wurde abgesaugt und nachgetrocknet. Es wurden 12.3 g (73% d. Th.) des Produktes als Feststoff erhalten.
LCMS (Methode 3): Rₜ = 2.19 min. (m/z = 255 (M+H)⁻).
¹H-NMR (400MHz, DMSO-d₆): δ = 10.37 (s, 1H), 9.83 (s, 1H), 8.2 (d, 1H), 7.42 (d, 1H), 1.46 (s, 9H).

### Beispiel 26A

### tert-Butyl- {6-chlor-3-[(hydroxyimino)methyl]pyridin-2-yl}carbamat

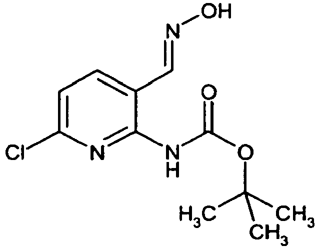

15.45 g (60.2 mmol) tert-Butyl-(6-chlor-3-formylpyridin-2-yl)carbamat (Beispiel 25A) wurde in 750 ml Ethanol vorgelegt und mit einer Lösung aus 225 ml Wasser und 9.38 g (120.4 mmol) Natriumacetat versetzt und 5 min gerührt. Eine Lösung aus 225 ml Wasser und 8.36 g ( 114.4 mmol) Hydroxylamin Hydrochlorid wurde zugegeben und 4 h bei RT gerührt. Bei 20°C wurde das Reaktionsgemisch am Rotationsverdampfer eingeengt. Der Rückstand wurde in Essigsäureethylester aufgenommen, zweimal mit gesättigter Natriumhydrogencarbonat-Lösung und einmal mit gesättiger Natriumchlorid-Lösung gewaschen. Die organische Phase wurde abgetrennt, über Magnesiumsulfat getrocknet und bei 20°C am Rotationsverdampfer eingeengt. Es wurden 15.5 g (80% d. Th.) des Produktes als Feststoff erhalten.
LCMS (Methode 3): Rₜ = 2.08 min. (m/z = 270 (M+H)⁻).
¹H-NMR (400MHz, DMSO-d₆): δ = 11.71 (s, 1H), 9.91 (s, 1H), 8.14 (s, 1H), 8.02 (d, 1H), 7.3 (d, 1H), 1.49 (s, 9H).

### Beispiel 27A

### 2-Amino-6-chlorpyridin-3-carbaldehydoxim Hydrochlorid

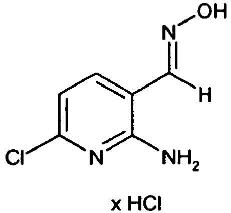

15.5 g (57 mmol) tert-Butyl-{6-chlor-3-[(hydroxyimino)methyl]pyridin-2-yl}carbamat (Beispiel 26A) wurden in 285 ml 4N Chlorwasserstoff in Dioxan gelöst und 30 min nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 11 g (94% d. Th.) des Produktes als Feststoff erhalten.
LCMS (Methode 6): Rₜ = 1.09 min. (m/z = 172 (M+H)⁺)
¹H-NMR (400MHz, DMSO-d₆): δ = 8.27 (s, 1H), 7.61 (d, 1H), 6.65 (d, 1H).

### Beispiel 28A

### 2-Amino-6-chlorpyridin-3-carbonitril

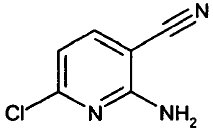

11.15 g (53.6 mmol) 2-Amino-6-chlorpyridin-3-carbaldehydoxim Hydrochlorid (Beispiel 27A) wurden in Dioxan vorgelegt, mit 13 ml (161 mmol) Pyridin versetzt und auf 0°C abgekühlt. Es wurden 8.3 ml (58.95 mmol) Trifluoressigsäureanhydrid zugegeben, man erwärmte die Reaktion auf RT und rührte anschließend 2 h bei 60°C. Das Reaktionsgemisch wurde in einem Gemisch von Essigsäureethylester und Natriumhydrogencarbonat-Lösung aufgenommen. Die organische Phase wurde mit gesättigter wässriger Natriumchlorid-Lösung gewaschen, über Magnesiumsulfat getrocknet und am Rotationsverdampfer eingeengt. Der Rückstand wurde in Dichlormethan:Diethylether 3:1 suspendiert, und der Feststoff wurde abgesaugt und getrocknet. Es wurden 5.56 g (66% d. Th.) des Produktes als Feststoff erhalten.
LCMS (Methode 6): Rₜ = 1.0 min. (m/z = 154 (M+H)⁺).
¹H-NMR (400MHz, DMSO-d₆): δ = 7.91 (d, 1H), 7.38 (s, 2H), 6.69 (d, 1H).

### Beispiel 29A

### tert-Butyl-{2-[(6-amino-5-cyanopyridin-2-yl)amino]ethyl}carbamat

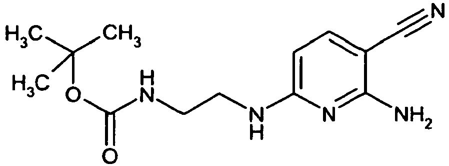

2 g (13 mmol) 2-Amino-6-chlorpyridin-3-carbonitril (Beispiel 28A) wurden in 15 ml DMSO vorgelegt und mit 2.71 g (16.93 mmol) N-Boc-Ethylenamin und 3.4 ml (19.54 mmol) N,N-Diisopropylethylamin versetzt. Das Reaktionsgemisch wurde 1.5 h bei 115°C in dem Mikrowellenreaktor bestrahlt. Das Reaktionsgemisch wurde in einem Gemisch von Essigsäureethylester und Wasser aufgenommen. Die organische Phase wurde mit gesättigter wässriger Natriumchlorid-Lösung gewaschen, über Magnesiumsulfat getrocknet und am Rotationsverdampfer eingeengt. Es wurden 23.38 g (88% d. Th.) des Produktes als Feststoff erhalten.
LCMS (Methode 3): Rₜ = 1.7 min. (m/z = 278 (M+H)⁺).
¹H-NMR (400MHz, DMSO-d₆): δ = 7.3 (s, 1H), 7.0 (br, s, 1H), 6.83 (s, 1H), 6.25 (s, 2H), 5.78 (d, 1H), 3.25 (q, 2H), 3.06 (q, 2H), 1.36 (s, 9H).

### Beispiel 30A

### 2-Amino-6-[(2-aminoethyl)amino]pyridin-3-carbonitril Dihydrochlorid

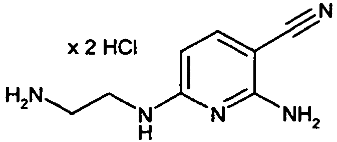

6.76 g (24.38 mmol) tert-Butyl-{2-(6-amino-5-cyanopyridin-2-yl)amino]ethyl}carbamat (Beispiel 29A) wurden in 122 ml 4N Chlorwasserstofflösung in Dioxan gelöst und 30 min nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 5.43 g (89% d. Th.) des Produktes als Feststoff erhalten.
LCMS (Methode 6): Rₜ = 0.92 min. (m/z = 177 (M+H)⁺)
¹H-NMR (400MHz, DMSO-d₆): δ = 8.1 (s, 2H), 7.5 (d, 1H), 5.96 (d, 1H), 3.53 (q, 2H), 3.0 (q, 2H).

### Beispiel 31A

### 4-(Trifluoracetyl)morpholin

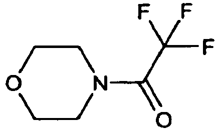

15 g (172 mmol) Morpholin wurden in 750 ml Dichlormethan vorgelegt, und es wurden bei 0°C 29 ml (206 mmol) Trifluoressigsäureanhydrid und 119 ml (688 mmol) N,N-Diisopropylethylamin zugegeben. Das Reaktionsgemisch wurde auf RT erwärmt und 3 h bei RT nachgerührt. Das Reaktionsgemisch wurde eingeengt und der Rückstand in Essigsäureethylester aufgenommen und nacheinander mit wässriger Natriumhydrogencarbonat-Lösung, 1N Salzsäure und wieder mit wässriger Natriumhydrogencarbonat-Lösung gewaschen. Die organische Phase wurde über Magnesiumsulfat getrocknet und am Rotationsverdampfer eingeengt. Es wurden 28 g (88% d. Th.) des Produktes als Öl erhalten.
LCMS (Methode 9): Rₜ = 1.22 min. (m/z = 184 (M+H)⁺)
¹H-NMR (400MHz, DMSO-d₆): δ = 3.65 (m, 2H), 3.56 (m, 2H).

### Beispiel 32A

### tert-Butyl-[6-chlor-3-(trifluoracetyl)pyridin-2-yl]carbamat

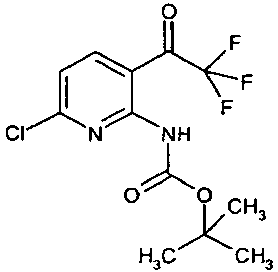

8 g (35 mmol) tert-Butyl-(6-chlorpyridin-2-yl)carbamat (Beispiel 24A) wurden in 100 ml THF vorgelegt und auf -50°C gekühlt. Es wurden 55 ml (87 mmol) Butyllithium (1.6N) zugetropft. Nach Beendigung des Zutropfens wurde die Reaktion langsam auf -10°C erwärmt und bei 0°C für 2 h gerührt. Anschließend wurde wieder auf -40°C abgekühlt, und es wurden 12.8 g (70 mmol) 4-(Trifluoracetyl)morpholin (Beispiel 21A), gelöst in 4 ml THF, zugegeben. Die Reaktionslösung wurde 1 h bei -40°C nachgerührt, danach bei -40°C auf 1 1 Essigsäureethylester und 350 ml Ammoniumchloridlösung gegossen und extrahiert. Die organische Phase wurde abgetrennt, über Magnesiumsulfat getrocknet und am Rotationsverdampfer eingeengt. Man chromatographierte das Reaktionsgemisch an Kieselgel (Laufmittel Cyclohexan/ Essigsäureethylester 10:1). Es wurden 9 g (79% d. Th.) des Produktes als Öl erhalten.
¹H-NMR (400MHz, DMSO-d₆): δ = 10.96 (s, 1H), 7.99 (d, 1H), 7.4 (d, 1H), 1.43 (s, 9H).

### Beispiel 33A

### tert-Butyl-[6-({2-[(tert-butoxycarbonyl)amino]ethyl}amino)-3-(trifluoracetyl)pyridin-2-yl]carbamat

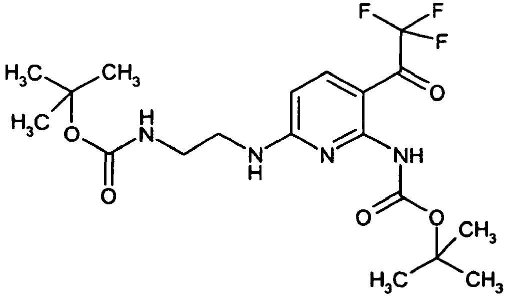

5 g (15.4 mmol) tert-Butyl-[6-chlor-3-(trifluoracetyl)pyridin-2-yl]carbamat (Beispiel 32A) wurden in 37.5 ml DMSO vorgelegt und mit 3.2 g (20 mmol) N-Boc-Ethylendiamin und 4 ml (23 mmol) N,N-Diisopropylethylamin versetzt. Das Reaktionsgemisch wurde 0.5 h bei 90°C in dem Mirkrowellenreaktor bestrahlt. Das Reaktionsgemisch wurde in einem Gemisch von Essigsäureethylester und Wasser aufgenommen. Die organische Phase wurde mit gesättigter wässriger Natriumchlorid-Lösung gewaschen, über Magnesiumsulfat getrocknet und am Rotationsverdampfer eingeengt. Man chromatographierte das Reaktionsgemisch an Kieselgel (Laufmittel Cyclohexan/Essigsäureethylester 5:1 → 1:1). Es wurden 2.5 g (34% d. Th.) des Produktes als Feststoff erhalten.
LCMS (Methode 6): Rₜ = 2.44 min. (m/z = 449 (M+H)⁺).
¹H-NMR (400MHz, DMSO-d₆): δ = 10.75 (s, 1H), 8.44 (s, 1H), 7.70 (d, 1H), 6.77 (s, 1H), 6.28 (d, 1H), 3.48 (br, s, 2H), 3.17 (br, s, 2H), 1.46 (s, 9H), 1.30 (s, 9H).

### Beispiel 34A

### 1-{2-Amino-6-[(2-aminoethyl)amino]pyridin-3-yl}-2,2,2-trifluorethanon Hydrochlorid

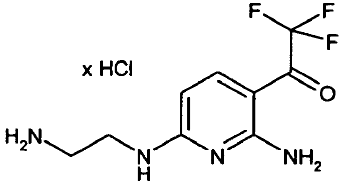

2.5 g (5.57 mmol) tert-Butyl-[6-({2-[(tert-butoxycarbonyl)amino]ethyl}amino)-3-(trifluoracetyl)-pyridin-2-yl]carbamat (Beispiel 33A) wurden in 15 ml 4N Chlorwasserstofflösung in Dioxan gelöst und 20 h nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 1.4 g (89% d. Th.) des Produktes als Feststoff erhalten.
LCMS (Methode 6): Rₜ = 0.73 min. (m/z = 249 (M+H)⁺).

### Beispiel 35A

### tert-Butyl 3-[(5-cyanopyridin-2-yl)amino]piperidin-1-carboxylat

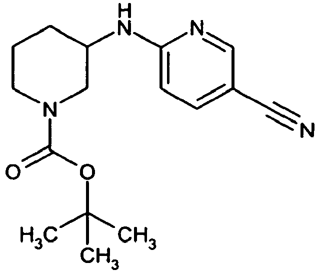

1.0 g (4.99 mmol) tert-Butyl-3-aminopiperidin-1-carboxylat und 1.383 g (9.99 mmol) 6-Chlorpyridin-3-carbonitril und 1.29 g (9.99 mmol) Diisopropylethylamin wurden in 40 ml DMSO suspendiert und für 45 min in einer Mikrowelle auf 140°C erhitzt. Der Ansatz wurde durch Kugelrohrdestillation weitgehend vom DMSO befreit, mit Wasser versetzt und der ausfallende Niederschlag abfiltriert. Nach Trocknen im Hochvakuum erhielt man 2.24 g (46% d. Th.) des Produktes.
LCMS (Methode 3): Rₜ = 2.23 min. (m/z = 303 (M+H)⁺).

### Beispiel 36A

### 6-(Piperidin-3-ylamino)pyridin-3-carbonitril Hydrochlorid

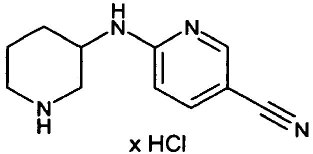

In 4.3 ml einer Salzsäurelösung in Dioxan (4 M) wurden 2.24 g (3.4 mmol) tert-Butyl-3-[(5-cyanopyridin-2-yl)amino]piperidin-1-carboxylat (Beispiel 35A) gelöst und 3 h bei RT gerührt. Nach vollständiger Reaktion wurde das Lösungsmittel komplett entfernt. Es wurden 1.74 g (90% d. Th.) des Produktes als Feststoff erhalten.
LCMS (Methode 8): Rₜ = 0.27 min. (m/z = 203 (M+H)⁺)
¹H-NMR (400MHz, DMSO-d₆): δ = 9.13 (m, 1H), 9.0 (m, 1H), 8.44 (d, 1H), 7.89 (m, 1H), 7.74 (dd, 1H), 6.63 (d, 1H), 5.58 (s, br), 4.19 (s, br, 1H), 3.57 (s, 1H), 3.34 (d, 1H), 3.14 (d, 1H), 2.88 (m, 1H), 2.7-2.81 (m, 1H), 1.82-2.0 (m, 2H), 1.63-1.79 (m, 1H), 1.48-1.59 (m, 1H).

### Beispiel 37A

### tert-Butyl-3-[(6-amino-5-cyanopyridin-2-yl)amino]piperidin-1-carboxylat

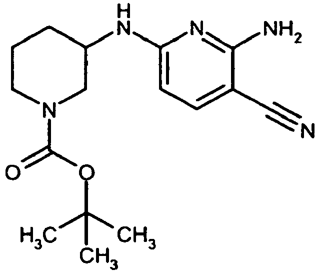

2.15 g (10.7 mmol) tert-Butyl-3-aminopiperidin-1-carboxylat, 1.50 g (9.77 mmol) 2-Amino-6-chlorpyridin-3-carbonitril (Beispiel 28A) und 1.89 g (14.7 mmol) Diisopropylethylamin wurden in 6 ml DMSO suspendiert und für 8 h in einem Mikrowellenreaktor auf 130°C erhitzt. Das Reaktionsgemisch wurde mit Ethylacetat (100 ml) und Wasser (40 ml) verdünnt, die organische Phase wurde getrennt und mit gesättigter wässriger Natriumchlorid-Lösung (50 ml) gewaschen, über Magnesiumsulfat getrocknet und eingeengt. Der Rückstand wurde chromatographiert auf Kieselgel (Laufmittel: Cyclohexan-Ethylacetat 4:1 bis 1:1). Es wurden 2.04 g (60% d. Th.) des Produktes als Feststoff isoliert.
LCMS (Methode 6): Rₜ = 1.69 min. (m/z = 318 (M+H)⁺)

### Beispiel 38A

### 2-Amino-6-(piperidin-3-ylamino)pyridin-3-carbonitril Hydrochlorid

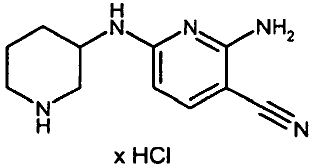

In 40 ml einer Salzsäurelösung in Dioxan (4 M) wurden 2.00 g (6.3 mmol) tert-Butyl-3-[(6-amino-5-cyanopyridin-2-yl)amino]piperidin-1-carboxylat (Beispiel 37A) gelöst und 2 h bei RT gerührt. Nach vollständiger Reaktion wurde das Lösungsmittel zur Hälfte eingeengt, und 20 ml Diethylether wurde zugegeben. Der Niederschlag wurde abfiltriert und getrocknet. Es wurden 1.80 g (100% d. Th.) des Produktes als Feststoff erhalten.
LCMS (Methode 8): Rₜ = 0.25 min. (m/z = 218 (M+H)⁺)
¹H-NMR (400MHz, DMSO-d₆): δ = 9.38 (br m, 1H), 8.97 (br m, 1H), 8.25 (br m, 1H), 7.53 (m, 1H), 7.40 (br s, 2H), 6.01 (d, 1H), 4.16 (br m, 1H), 3.34 (br m, 1H), 3.10 (m, 1H), 2.89 (m, 2H), 2.00-1.84 (m, 2H), 1.73 (m, 1H), 1.55 (m, 1H).

### Beispiel 39A

### tert-Butyl-3-({6-[(tert-butoxycarbonyl)amino]-5-(trifluoracetyl)pyridin-2-yl}amino)piperidin-1-carboxylat

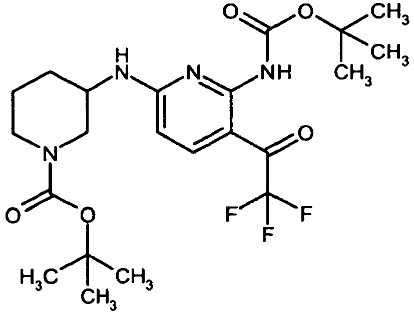

561 mg (2.8 mmol) tert-Butyl-3-aminopiperidin-1-carboxylat, 700 mg (2.16 mmol) tert-Butyl-[6-chlor-3-(trifluoracetyl)pyridin-2-yl]carbamat (Beispiel 32A) und 0.56 ml (3.23 mmol) Diisopropylethylamin wurden in 14 ml DMSO suspendiert und für 45 min in einem Mikrowellenreaktor auf 90°C erhitzt. Das Reaktionsgemisch wurde mit Ethylacetat (100 ml) verdünnt und mit gesättigter wässriger Ammoniumchloridlösung (dreimal 40 ml) und dann gesättigter wässriger Natriumhydrogencarbonatlösung (40 ml) gewaschen. Die organische Phase wurde getrocknet über Magnesiumsulfat und eingeengt. Der Rückstand wurde chromatographiert auf Kieselgel (Laufmittel: Cyclohexan-Ethylacetat 5:1 bis 1:1). Es wurden 670 mg (63% d. Th.) des Produktes isoliert.
LCMS (Methode 6): Rₜ = 2.70 min. (m/z = 489 (M+H)⁺)

### Beispiel 40A

### 1-[2-Amino-6-(piperidin-3-ylamino)pyridin-3-yl]-2,2,2-trifluorethanon Hydrochlorid

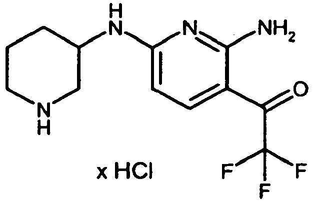

In 25 ml einer Salzsäurelösung in Dioxan (4 M) wurden 670 mg (1.37 mmol) tert-Butyl-3-({6-[(tert-butoxycarbonyl)amino]-5-(trifluoracetyl)pyridin-2-yl}amino)piperidin-1-carboxylat (Beispiel 39A) gelöst und 20 h bei RT gerührt. Nach vollständiger Reaktion wurde das Reaktionsmischung mit Diethylether (100 ml) verdünnt, und der Niederschlag wurde abfiltriert und mit Diethylether (100 ml) gewaschen und getrocknet. Es wurden 286 mg (64% d. Th.) des Produktes als Feststoff erhalten.
LCMS (Methode 6): Rₜ = 0.81 min. (m/z = 289 (M+H)⁺)
¹H-NMR (400MHz, DMSO-d₆): δ = 9.26 (br s, 1H), 9.07 (br s, 1H), 8.8.34 (br s, 1H), 7.59 (d, 1H), 6.22 (br, 2H), 6.03 (d, 1H), 4.25 (br m, 1H), 3.36 (m, 1H), 3.13 (m, 1H), 2.93 (m, 2H), 2.00-1.85 (m, 2H), 1.73 (m, 1H), 1.56 (m, 1 H).

### Beispiel 41A

### tert-Butyl-3-[(6-amino-5-nitropyridin-2-yl)amino]piperidin-1-carboxylat

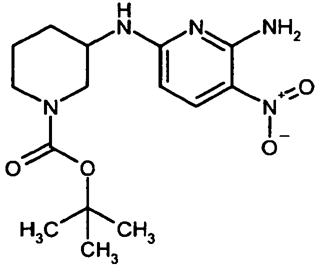

500 mg (2.11 mmol) tert-Butyl-3-aminopiperidin-1-carboxylat, 772 mg (4.22 mmol) 2-Amino-6-chlor-3-nitropyridin und 1.05 ml (6.34 mmol) Diisopropylethylamin wurden in 18 ml DMSO suspendiert und für 45 min in einem Mikrowellenreaktor auf 120°C erhitzt. Das Reaktionsgemisch wurde mittels präparativer reverse-phase HPLC aufgereinigt. Es wurden 600 mg (81% d. Th.) des Produktes als Feststoff isoliert.
LCMS (Methode 6): Rₜ = 1.77 min. (m/z = 338 (M+H)⁺)

### Beispiel 42A

### 3-Nitro-N⁶-(piperidin-3-yl)pyridin-2,6-diamin Hydrochlorid

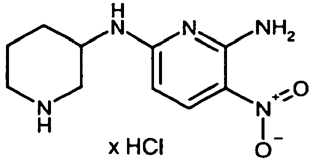

In 40 ml einer Salzsäurelösung in Dioxan (4 M) wurden 610 mg (1.62 mmol) tert-Butyl-3-[(6-amino-5-nitropyridin-2-yl)amino]piperidin-1-carboxylat (Beispiel 41A) gelöst und 30 min bei RT gerührt. Nach vollständiger Reaktion wurde das Lösungsmittel komplett entfernt. Es wurden 662 mg des Rohproduktes erhalten.
LCMS (Methode 4): Rₜ = 0.86 min. (m/z = 238 (M+H)⁺)

### Beispiel 43A

### Methyl-4-amino-2-(methylsulfonyl)-1,3-thiazol-5-carboxylat

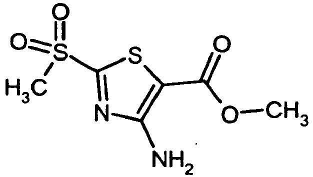

In 170 ml Wasser wurden 5.12 g (8.32 mmol) Oxone^{®} gelöst und auf 5°C abgekült. Dann tropfte man eine Lösung von 1 g (4.90 mmol) Methyl-4-amino-2-(methylsulfanyl)-1,3-thiazol-5-carboxylat in 18 ml Methanol zu und rührte die Lösung für 3 h bei RT. Die Hauptmenge Methanol wurde entfernt und dreimal mit Dichlormethan extrahiert. Die vereinigten organischen Phasen wurden mit Natriumsulfat getrocknet. Nach Entfernen des Lösungsmittels und Trocknen des Rückstandes im Hochvakuum wurde der erhaltene Feststoff (824 mg (43% d. Th.)) ohne weitere Reinigung eingesetzt.
LCMS (Methode 3): Rₜ = 1.52 min. (m/z = 237 (M+H)⁺).
¹H-NMR (400MHz, DMSO-d₆): d = 7.36 (s, br, 2H), 3.79 (s, 3H), 3.45 (s, 3H).

### Beispiel 44A

### tert-Butyl-3-{[4-amino-5-(methoxycarbonyl)-1,3-thiazol-2-yl]amino}piperidin-1-carboxylat

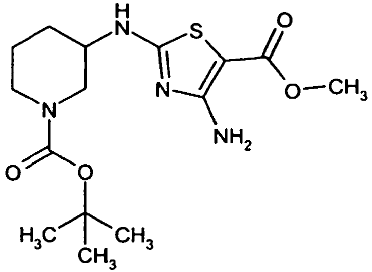

Analog der Herstellung von Beispiel 49A wurden aus 335 mg (1.42 mmol) tert-Butyl-3-aminopiperidin-1-carboxylat Hydrochlorid und 3190 mg (2.84 mmol) Methyl-4-amino-2-(methylsulfonyl)-1,3-thiazol-5-carboxylat 158 mg (29% d. Th.) des Produktes als Feststoff erhalten.
LCMS (Methode 8): Rₜ = 1.06 min. (m/z = 357 (M+H)⁺).
¹H-NMR (400MHz, DMSO-d₆): δ = 8.32 (d, 1H), 6.79 (s, br, 2H), 3.60 (s, 3H), 3.55 (m, 2H), 1.89 (m, 1H), 1.71 (m, 1H), 1.5 (m, 1H), 1.35 (s, 11H).

### Beispiel 45A

### Methyl-4-amino-2-(piperidin-3-ylamino)-1,3-thiazol-5-carboxylat Dihydrochlorid

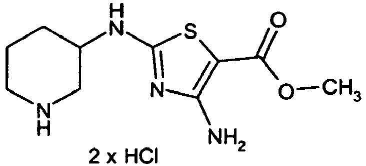

Analog der Herstellung von Beispiel 38A wurden aus 150 mg (0.39 mmol) tert-Butyl-3-{[4-amino-5-(methoxycarbonyl)-1,3-thiazol-2-yl]amino}piperidin-1-carboxylat und 20 ml Salzsäure in Dioxan (4M) 130 mg (99% d. Th.) des Produktes als Feststoff erhalten.
LCMS (Methode 8): Rₜ = 0.26 min. (m/z = 257 (M+H)⁺).
¹H-NMR (400MHz, DMSO-d₆): δ = 9.04 (s, br, 2H), 8.51 (d, 1H), 3.9 (m, 2H), 3.6 (s, 3H), 3.34 (d, 1H), 3.11 (d, 1H), 2.75-2.94 (m, 2H), 1.93-2.04 (m, 1H), 1.8-1.91 (m, 1H), 1.60-1.76 (m, 1H), 1.43-1.57 (m, 1H).

### Beispiel 46A

### tert-Butyl-3-[(4-amino-5-cyano-1,3-thiazol-2-yl)amino]piperidin-1-carboxylat

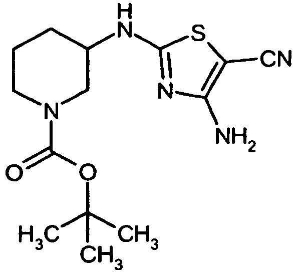

643 mg (3.17 mmol) 4-Amino-2-(methylsulfonyl)-1,3-thiazol-5-carbonitril (Beispiel 6A) wurden in 16 ml DMSO gelöst und mit 500 mg (2.11 mmol) tert-Butyl-3-aminopiperidin-1-carboxylat, sowie 3.49 ml (21.12 mmol) N,N-Diisopropylethylamin versetzt. Es wurde 45 min bei 120°C in der Mikrowelle erhitzt. Das Reaktionsgemisch wurde in einem Gemisch von Essigsäureethylester und Wasser aufgenommen. Die organische Phase wurde mit gesättigter wässriger Natriumchloridlösung gewaschen, über Magnesiumsulfat getrocknet und am Rotationsverdampfer eingeengt. Das Rohprodukt wurde ohne weitere Aufreinigung eingesetzt.
LCMS (Methode 8): Rₜ = 1.0 min. (m/z = 357 (M+H)⁺).

### Beispiel 47A

### 4-Amino-2-(piperidin-3-ylamino)-1,3-thiazol-5-carbonitril Dihydrochlorid

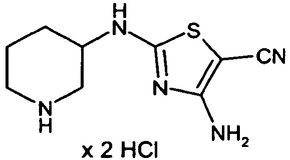

Analog der Herstellung von Beispiel 38A wurden aus 240 mg (0.7 mmol) tert-Butyl-3-[(4-amino-5-cyano-1,3-thiazol-2-yl)amino]piperidin-1-carboxylat (Beispiel 46A) und 25 ml Salzsäure in Dioxan (4M) 265 mg (57% d. Th.) des Produktes als Feststoff erhalten.
LCMS (Methode 9): Rₜ = 0.74 min. (m/z = 224 (M+H)⁺).

### Beispiel 48A

### 1-[4-Amino-2-(methylsulfonyl)-1,3-thiazol-5-yl]ethanon

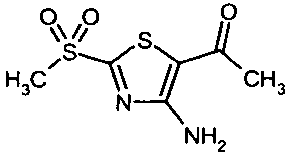

In 9 ml Wasser wurden 2.775 g (4.52 mmol) Oxone^{®} gelöst und 500 mg (2.66 mmol) 1-[4-Amino-2-(methylsulfanyl)-1,3-thiazol-5-yl]ethanon, gelöst in 9 ml Methanol, bei 5°C zugetropft. Man ließ für 3 h bei RT rühren, reduzierte das Methanol am Rotationsverdampfer und extrahierte den Rückstand zweimal mit Dichlormethan. Nach Entfernen des Lösungsmittels erhielt man 395 mg (52% d. Th.) des Produktes als Feststoff.
LCMS (Methode 3): Rₜ = 1.17 min. (m/z = 221 (M+H)⁺).

### Beispiel 49A

### tert-Butyl-3-[(5-acetyl-4-amino-1,3-thiazol-2-yl)amino]piperidin-1-carboxylat

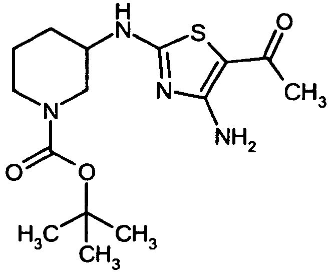

390 mg (1.77 mmol) 1-[4-Amino-2-(methylsulfonyl)-1,3-thiazol-5-yl]ethanon (Beispiel 48A) wurden in 5 ml DMSO gelöst und mit 209.6 mg (0.89 mmol) tert-Butyl-3-aminopiperidin-1-carboxylat, sowie 0.585 ml (3.54 mmol) N,N-Diisopropylethylamin versetzt. Es wurde 45 min bei 120°C in der Mikrowelle erhitzt. Man erhielt nach Reinigung mittels präparativer HPLC 162 mg (52% d. Th.) des Produktes als Feststoff.
LCMS (Methode 8): Rₜ = 1.87 min. (m/z = 341 (M+H)⁺).
¹H-NMR (400MHz, DMSO-d₆): δ = 8.56 (d, 1H), 7.70 (s, br, 2H), 3.81 (m, 4H), 3.56 (m, 2H), 2.02 (s, 3H), 1.91 (m, 1H), 1.71 (m, 1H), 1.51 (m, 1H), 1.36 (s, 9H).

### Beispiel 50A

### 1-[4-Amino-2-(piperidin-3-ylamino)-1,3-thiazol-5-yl]ethanon Dihydrochlorid

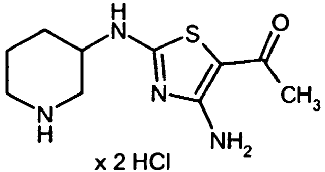

Analog der Herstellung von Beispiel 38A wurden aus 160 mg (0.15 mmol) tert-Butyl-3-[(5-acetyl-4-amino-1,3-thiazol-2-yl)amino]piperidin-1-carboxylat (Beispiel 49A) und 20 ml Salzsäure in Dioxan (4M) 40 mg (87% d. Th.) des Produktes als Feststoff erhalten.
LCMS (Methode 9): Rₜ = 0.81 min. (m/z = 241 (M+H)⁺.
¹H-NMR (400MHz, DMSO-d₆): δ = 9.22 (s, br, 2H), 8.93 (d, 1H), 7.7 (s, br, 1H), 3.96 (m, 1H), 3.35 (d, 1H), 3.12 (d, 1H), 2.86 (m, 2H), 2.05 (s, 3H), 1.93-2.05 (m, 1H), 1.8-1.93 (m, 1H), 1.61-1.78 (m, 1H), 1.45-1.6 (m, 1H).

### Beispiel 51A

### Methyl-2-[(tert-butoxycarbonyl)amino]-6-chlorpyridin-3-carboxylat

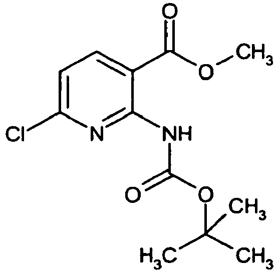

2.0 g (8.7 mmol) tert-Butyl-(6-chlorpyridin-2-yl)carbamat (Beispiel 24A) wurden in 50 ml THF vorgelegt und auf -78°C gekühlt. Es wurden 13.7 ml (22 mmol) Butyllithium (1.6 M) zugetropft. Nach Beendigung des Zutropfens wurde die Reaktion langsam auf -10°C erwärmt und bei -10°C für 2 h gehalten. Anschließend wurde wieder auf -78°C gekühlt und 870 mg (9.2 mmol) Chlorameisensäuremethylester wurden zugegeben. Die Reaktionslösung wurde über 12 h auf RT erwärmt, danach wurde das Reaktionsgemisch auf 150 ml Essigsäureethylester und 80 ml Salzsäure-Lösung (IN) gegossen und 15 min nachgerührt. Die organische Phase wurde abgetrennt, mit Wasser und gesättigter Natriumhydrogencarbonat-Lösung gewaschen, über Magnesiumsulfat getrocknet und am Roationsverdampfer eingeengt. Man chromatographierte das Reaktionsgemisch an Kieselgel (Laufmittel Cyclohexan/Essigsäureethylester 10:1). Es wurden 1018 mg (33% d. Th.) des Produktes als Öl erhalten.
LCMS (Methode 8): Rₜ = 1.25 min. (m/z = 187 (M+H-Boc)⁺)

### Beispiel 52A

### Methyl-2-[(tert-butoxycarbonyl)amino]-6-({2-[(tert-butoxycarbonyl)amino]ethyl}amino)pyridin-3-carboxylat

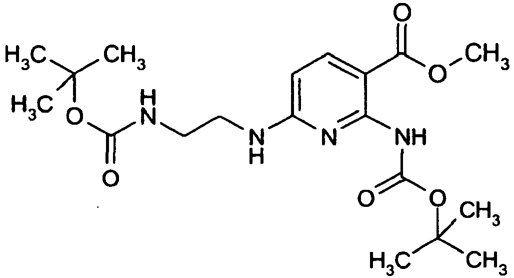

Analog der Herstellung von Beispiel 33A wurden aus 650 mg (2.3 mmol) Methyl-2-[(tert-butoxycarbonyl)amino]-6-chlorpyridin-3-carboxylat (Beispiel 51A) und 363 mg (2.3 mmol) N-Boc-ethylendiamin 500 mg (50% d. Th.) des Produktes als Feststoff erhalten.
LCMS (Methode 8): Rₜ = 1.32 min. (m/z = 411 (M+H)⁺).

### Beispiel 53A

### Methyl-2-amino-6-[(2-aminoethyl)amino]pyridin-3-carboxylat Dihydrochlorid

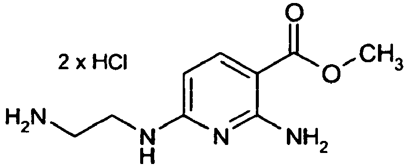

Analog der Herstellung von Beispiel 38A wurden aus 496 mg (1.2 mmol) Methyl-2-[(tert-butoxycarbonyl)amino]-6-({2-[(tert-butoxycarbonyl)amino]ethyl}amino)pyridin-3-carboxylat (Beispiel 52A) 363 mg (82% d. Th.) des Produktes als Feststoff erhalten.
LCMS (Methode 9): Rₜ = 0.75 min. (m/z = 212 (M+H-2HCl)⁺).

### Beispiel 54A

### 6-[(2-{[6-(2,4-Dichlorphenyl)-2-formylpyrazolo[1,5-a]pyrazin-4-yl]amino}ethyl)amino]pyridin-3-carbonitril

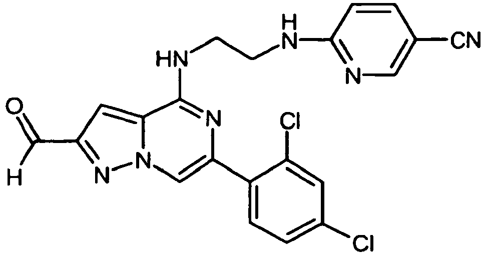

1.2 g (2.64 mmol) 6-[(2-{[6-(2,4-Dichlorphenyl)-2-(hydroxymethyl)pyrazolo[1,5-a]pyrazin-4-yl]-amino}ethyl)amino]pyridin-3-carbonitril (Beispiel 42) wurden in 80 ml Dichlormethan (vorher mit Wasser geschüttelt) suspendiert. Daraufhin wurden 80 ml 1,2-Dimethoxyethan und 40 ml DMF zugegeben und auf 0°C abgekühlt. Es wurden 2.46 g (5.81 mmol) Dess-Martin-Periodinan zugegeben, die Eisbadkühlung entfernt und 2 h bei RT gerührt. Der Ansatz wurde mit Essigsäureethylester versetzt und zuerst mit einer 10% Natriumthiosulfatlösung und dann mit gesättigter wäßriger Natriumhydrogencarbonat-Lösung gewaschen. Nach Trocknung der organischen Phase mit Natriumsulfat und Entfernen des Lösungsmittels im Vakuum erhielt man 1.2 g (94% d. Th.) des Produktes als Feststoff, der ohne weitere Aufreinigung umgesetzt wurde.
LCMS (Methode 9): Rₜ = 2.50 min. (m/z = 452 (M+H)⁺).
¹H-NMR (400MHz, DMSO-d₆): δ = 10.1 (s, 1H), 8.38 (d, 1H), 8.33 (s, 1H), 8.30 (t, 1H), 7.77 (m, 1H), 7.75 (d, 1H), 7.67 (d, 1H), 7.59 (d, 1H), 7.55 (s, 1H), 7.53 (dd, 1H), 6.52 (d, 1H), 3.55-3.7 (m, 4H).

### Beispiel 55A

### 6-(2,4-Dichlorphenyl)-4-oxo-4,5-dihydropyrazolo[1,5-a]pyrazin-2-carbonsäure

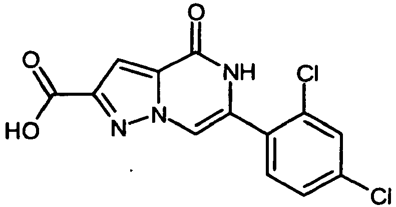

500 mg (1.42 mmol) 6-(2,4-Dichlorphenyl)-4-oxo-4,5-dihydropyrazolo[1,5-a]pyrazin-2-carbonsäureethylester (Beispiel 11A) wurden in 60 ml 1,2-Dimethoxyethan suspendiert, 1M wässrige Natriumhydroxid (5.7 ml, 5.7 mmol) wurde zugegeben, und der Ansatz wurde 3 h bei RT gerührt. Das Reaktionsgemisch wurde auf Wasser gegossen, und mit 2M wässriger Salzsäure auf pH 2 sauer gestellt. Der Niederschlag wurde abfiltriert und mit Wasser nachgewaschen. Nach Trocknung erhielt man 430 mg (93% d. Th.) des Produktes als Feststoff.
LCMS (Methode 6): Rₜ = 1.31 min. (m/z = 324 (M+H)⁺).
¹H-NMR (400MHz, DMSO-d₆): δ = 13.29 (br, 1H), 11.80 (s, 1H), 7.94 (s, 1H), 7.84 (d, 1H), 7.66 (d, 1H), 7.59 (dd, 1H), 7.48 (s, 1H).

### Beispiel 56A

### 6-(2,4-Dichlorphenyl)-4-oxo-4,5-dihydropyrazolo[1,5-a]pyrazin-2-carboxamid

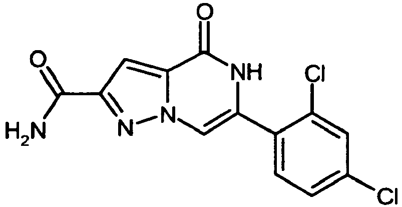

360 mg (1.11 mmol) 6-(2,4-Dichlorphenyl)-4-oxo-4,5-dihydropyrazolo[1,5-a]pyrazin-2-carbonsäure (Beispiel 55A) wurden in Dichlormethan (30 ml) und DMF (10 ml) vorgelegt, und 320 mg (1.67 mmol) EDC, 225 mg (1.67 mg) HOBt und 407 mg (3.33 mmol) DMAP wurden zugegeben, gefolgt von 0.5M Ammoniak in Dioxan (2.44 ml, 1.22 mmol). Das Reaktionsgemisch wurde 20 h bei RT gerührt, das Lösemittel wurde eingeengt, der Rückstand wurde auf Wasser (25 ml) gegossen, und der Niederschlag wurde abfiltriert und mit Wasser und Acetonitril gewaschen und getrocknet. Nach Trocknung erhielt man 260 mg (72% d. Th.) des Produktes als Feststoff.
LCMS (Methode 3): Rₜ = 1.69 min. (m/z = 323 (M+H)⁺).
¹H-NMR (400MHz, DMSO-d₆): δ = 11.77 (br, 1H), 7.86 (s, 1H), 7.84 (m, 2H), 7.65 (d, 1H), 7.59 (m, 2H), 7.47 (s, 1H).

### Beispiel 57A

### 4-Chlor-6-(2,4-dichlorphenyl)pyrazolo[1,5-a]pyrazin-2-carbonitril

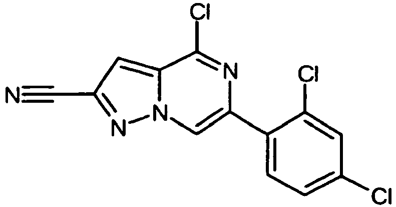

Analog der Herstellung von Beispiel 16A wurden aus 260 mg (0.81 mmol) 6-(2,4-Dichlorphenyl)-4-oxo-4,5-dihydropyrazolo[1,5-a]pyrazin-2-carboxamid (Beispiel 56A) durch Umsetzung mit Phosphorylchlorid 230 mg (77% d. Th.) des Produktes als Feststoff erhalten.
LCMS (Methode 3): Rₜ = 2.86 min. (m/z = 323 (M+H)⁺).

### Ausführungsbeispiele

### Beispiel 1

### 6-[(2-{[6-(2,4-Dichlorphenyl)imidazo[1,2-a]pyrazin-8-yl]amino}ethyl)amino]nicotinnitril

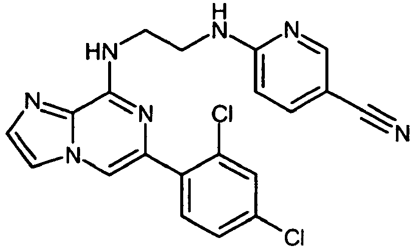

In 3 ml DMSO wurden 108 mg (0.3625 mmol) des 8-Chlor-6-(2,4-dichlorphenyl)-imidazo[1,2-a]pyrazin (Beispiel 5A) vorgelegt, und mit 150 mg (0.543 mmol) 6-[(2-Aminoethyl)amino]nicotinnitril (Beispiel 2A) und 0.63 ml (3.62 mmol) NN-Diisopropylethylamin versetzt. Es wurde 12 h bei 120°C erhitzt. Man erhielt nach Reinigung mittels Chromatographie an Kieselgel 60 (Laufmittel: Dichlormethan/Methanol 100:1) 12 mg (7% d. Th.) des Produktes.
LCMS (Methode 6): Rₜ = 1.97 min. (m/z = 424 (M+H)⁺)
¹H-NMR (400MHz, DMSO-d₆): δ = 8.35 (d, 1H), 8.09 (s, 1H), 7.94 (s, 1H), 7.74 (s, br, 1H), 7.70 (d, 2H), 7.63 (d, 1H), 7.56 (d, 2H), 7.49 (dd, 1H), 6.53 (s, br, 1H), 3.66 (t, 2H), 3.58 (t, br, 2H).

### Beispiel 2

### 4-Amino-2-[(2-{[6-(2,4-dichlorphenyl)imidazo[1,2-a]pyrazin-8-yl]amino}ethyl)amino]-1,3-thiazol-5-carbonitril Trifluoracetat

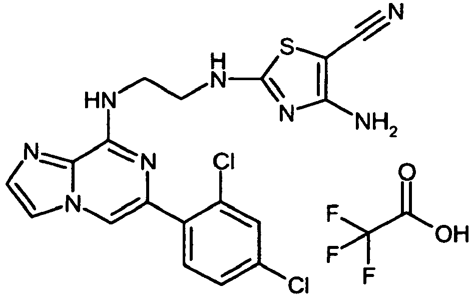

80 mg (0.2 mmol) 4-Amino-2-({2-[(6-bromimidazo[1,2-a]pyrazin-8-yl)amino]ethyl}amino)-1,3-thiazol-5-carbonitril (Beispiel 9A) wurden unter Argon in 4.5 ml Dioxan und 1.3 ml gesättigte Natriumcarbonatlösung vorgelegt und mit 48 mg (0.25 mmol) 2,4-Dichlorbenzolboronsäure, sowie 22 mg (0.02 mmol) Tetrakis(triphenylphosphin)palladium(0) versetzt. Es wurde 1 h bei 160°C in der Mirkrowelle erhitzt. Das Reaktionsgemisch wurde am Rotationsverdampfer eingeengt. Man erhielt nach Reinigung mittels präparativer HPLC 35 mg (33% d. Th.) des Produktes als Feststoff.
LCMS (Methode 8): Rₜ = 1.16 min. (m/z = 444 (M+H)⁺)
¹H-NMR (400MHz, DMSO-d₆): δ = 8.48 (t, 1H), 8.15 (s, 1H), 7.99 (s, 1H), 7.9 (s, breit, 1H), 7.72 (d, 1H), 7.65 (d, 1H), 7.62 (s, 1H), 7.51 (dd, 1H), 3.67 (q, 2H), 3.49 (q, 2H).

### Beispiel 3

### 4-({2-[(5-Cyanopyridin-2-yl)amino]ethyl}amino)-6-(2,4-dichlorphenyl)pyrazolo[1,5-a]pyrazin-2-carbonsäureethylester

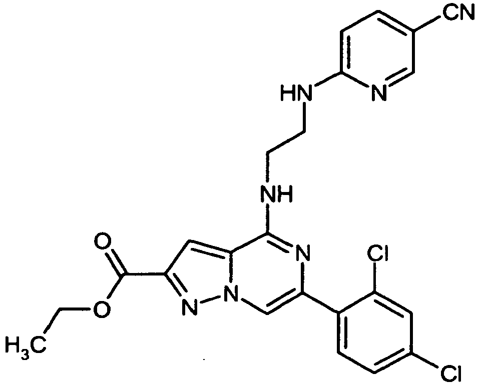

In 15 ml trockenem DMSO wurden 1 g (5 mmol) 4-Chlor-6-(2,4-dichlorphenyl)pyrazolo[1,5-a]pyrazin-2-carbonsäureethylester (Beispiel 12A) vorgelegt, und mit 1.55 g (4.22 mmol) 6-[(2-Aminoethyl)amino]nicotinnitril Dihydrochlorid (Beispiel 2A) und 5.8 ml (33.5 mmol) N,N-Diisopropylethylamin versetzt und bei 150°C für 30 min in der Mikrowelle erhitzt. Man gab zu dem Reaktionsgemisch Essigsäureethylester und 10%ige Zitronensäure und extrahierte. Die organische Phase wurde mit Natriumchloridlösung gewaschen und über Magnesiumsulfat getrocknet. Man erhielt nach dem Einengen 1.4 g (70% d. Th.) des Produktes als Feststoff.
LCMS (Methode 6): Rₜ = 2.31 min. (m/z = 496 (M+H)⁺)
¹H-NMR (400MHz, DMSO-d₆): δ = 8.38 (d, 1H), 8.3 (s, 1H), 8.15 (t, 1H), 7.73 (d, 1H), 7.66 (d, 2H), 7.59 (dd, 1H), 7.57 (s, 1H), 7.52 (dd, 1H), 6.52 (d, 1H), 4.35 (t, 2H), 3.7-3.55 (m, 4H), 1.34 (s, 3H).

### Beispiel 4

### 4-({2-[(5-Cyanopyridin-2-yl)amino]ethyl}amino)-6-(2,4-dichlorphenyl)pyrazolo[1,5-a]pyrazin-2-carbonsäure Hydrochlorid

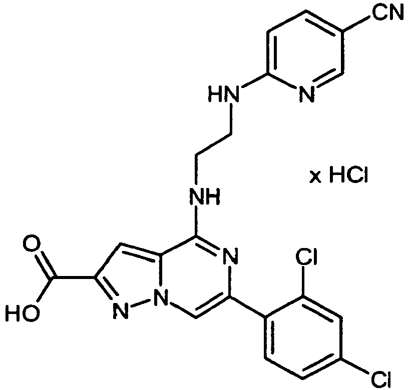

80 mg (0.16 mmol) Ester (Beispiel 3) wurden in 5 ml 1,2-Dimethoxyethan gelöst und mit 2.5 ml Wasser, sowie 0.43 ml (0.4 mmol) 1N Natriumhydroxid-Lösung versetzt. Die Lösung wurde 2 h bei RT gerührt. Es wurden 20 ml verdünnte Salzsäure (pH 3) vorgelegt und die Reaktionslösung langsam eingetropft. Es wurde 30 min nachgerührt, das Produkt abfiltriert und mit Diethylether nachgewaschen. Man erhielt nach Trocknung im Hochvakuum 70 mg (84% d. Th.) des Produktes als Feststoff.
LCMS (Methode 3): Rₜ = 2.59 min. (m/z = 468 (M+H)⁺)
¹H-NMR (400MHz, DMSO-d₆): δ = 8.38 (d, 1H), 8.26 (s, 1H), 8.17 (t, 1H), 7.77 (s, breit, 1H), 7.73 (d, 1H), 7.66 (d, 1H), 7.59 (d, 1H), 7.52 (dd, 1H), 7.51 (s, 1H), 6.53 (d, 1H), 3.7-3.56 (m, 4H).

### Allgemeine Versuchsbeschreibung für Amidkupplungen:

0.16 mmol Säure (Beispiel 4) werden in 2 ml DMF vorgelegt und nacheinander versetzt mit 0.17 mmol HATU, 0.48 mmol N,N-Diisopropylethylamin und 0.21 mmol Amin. Es wird 12 h bei RT nachrührt. Nach Aufreinigung mittels präparativer HPLC erhält man 60%-95% d. Th. des Produktes als Feststoff.

Nach der allgemeinen Versuchsbeschreibung für Amidkupplungen wurden die folgenden Verbindungen hergestellt.

| **Bsp.** | **Struktur** | **Charakterisierung** |
|---|---|---|
| **5** | 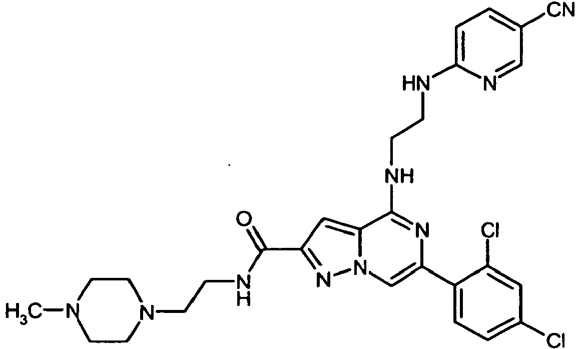 | LC/MS (Methode 8): Rₜ = 0.89 min, (m/z = 593 (M+H)⁺) |
| | | ¹H-NMR (400MHz, DMSO-d₆): δ = 10.81 (s, 1H), 8.38 (d, 1H), 8.28 (s, 1H), 8.22 (s, 1H), 7.91 (s, 1H), 7.74 (d, 1H), 7.66 (d, 1H), 7.61 (d, 1H), 7.53 (dd, 1H), 7.45 (s, 1H), 6.56 (d, 1H), 4.89 (d, 1H), 4.61 (d, 1H), 3.65 (m, 6H), 3.48 (q, 3H), 3.27 (t, 1H), 3.12 (m, 4H), 2.79 (d, 3H). |
| **6** | 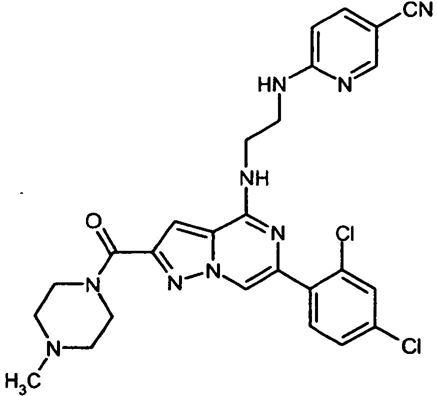 | LC/MS (Methode 6): Rₜ = 1.60 min, (m/z = 551 (M+H)⁺) |
| | | ¹H-NMR (400MHz, DMSO-d₆): δ = 8.38 (d, 1H), 8.28 (s, 1H), 8.22 (s, 1H), 7.91 (s, 1H), 7.74 (d, 1H), 7.66 (d, 1H), 7.61 (d, 1H), 7.53 (dd, 1H), 7.45 (s, 1H), 6.56 (d, 1H), 3.67 (m, 8H), 3.38 (s, breit, 4H), 2.38 (s, 3H). |
| **7** | 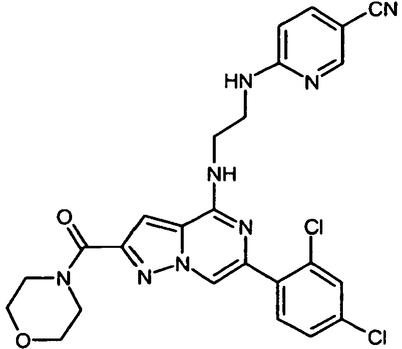 | LC/MS (Methode 8): Rₜ = 1.28 min, (m/z = 537 (M+H)⁺) |
| | | ¹H-NMR (400MHz, DMSO-d₆): δ = 8.39 (d, 1H), 8.25 (s, breit, 1H), 8.23 (s, 1H), 7.73 (d, 1H), 7.66 (d, 2H), 7.63 (dd, 1H), 7.52 (d, 1H), 7.41 (s, 1H), 6.6 (d, 1H), 3.73 (m, breit, 8H), 3.63 (s, breit, 4H). |
| **8** | 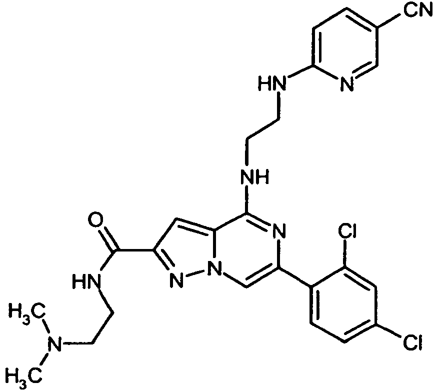 | LC/MS (Methode 8): Rₜ = 0.98 min, (m/z = 538 (M+H)⁺) |
| | | ¹H-NMR (400MHz, DMSO-d₆): δ = 10.09 (s, 1H), 8.8 (t, 1H), 8.39 (d, 2H), 8.19 (s, 1H), 7.75 (d, 1H), 7.7 (d, 1H), 7.63 (dd, 1H), 7.54 (m, 2H), 6.62 (d, 1H), 3.73-3.58 (m, 6H), 3.27 (q, 2H), 2.8 (d, 6H). |

### Beispiel 9

### 6-({2-[(5,7-Dimethyl-2-phenylimidazo[5,1-f][1,2,4]triazin-4-yl)amino]ethyl}amino)nicotinnitril

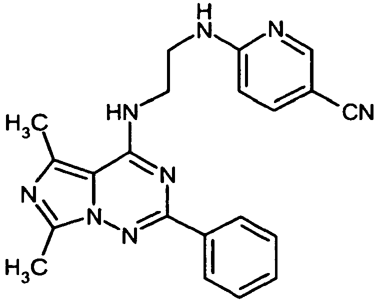

In 4 ml DMSO wurden 183 mg (0.71 mmol) des 4-Chlor-5,7-dimethyl-2-phenylimidazo[5,1-f][1,2,4]triazin (Beispiel 16A) vorgelegt, und mit 200 mg (0.85 mmol) 6-[(2-Aminoethyl)amino]nicotinnitril (Beispiel 2A) und 1.23 ml (7 mmol) N,N-Diisopropylethylamin versetzt und bei 150°C für 12 h erhitzt. Man erhielt nach Reinigung mittels Chromatographie an Kieselgel (Laufmittel: Dichlormethan/Methanol 100:1) 10 mg (4% d. Th.) des Produktes.
LCMS (Methode 6): Rₜ = 1.40 min. (m/z = 385 (M+H)⁺)
¹H-NMR (400MHz, DMSO-d₆): δ = 8.43 (s, 1H), 8.12 (d, 1H), 7.99 (d, 1H), 7.82 (t, 1H), 7.59 (t, 2H), 7.55 (q, 1H), 7.46 (m, 2H), 6.50 (d, 1H), 3.86 (q, 2H), 3.67 (s, breit, 2H), 2.5 (s, 6H).

### Beispiel 10

### 4-Amino-2-{[2-({6-[4-(trifluormethyl)phenyl]imidazo[1,2-a]pyrazin-8-yl}amino)ethyl]amino}-1,3-thiazol-5-carbonitril Trifluoracetat

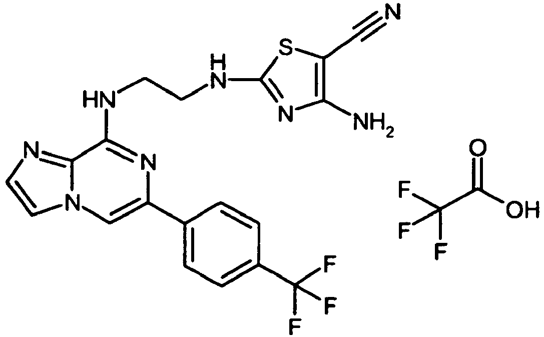

80 mg (0.192 mmol) 4-Amino-2-({2-[(6-bromimidazo[1,2-a]pyrazin-8-yl)amino] ethyl}amino)-1,3-thiazol-5-carbonitril (Beispiel 9A) wurden unter Argon in 4.5 ml Dioxan und 1.3 ml gesättigter Natriumcarbonatlösung vorgelegt und mit 47 mg (0.25 mmol) 4-(Trifluormethyl)-phenyl]boronsäure, sowie 22 mg (0.019 mmol) Tetrakis(triphenylphosphin)palladium(0) versetzt.

Es wurde 15 h bei 120°C erhitzt. Das Reaktionsgemisch wurde am Rotationsverdampfer eingeengt. Man erhielt nach Reinigung mittels präparativer HPLC 5 mg (5% d. Th.) des Produktes als Feststoff.
LCMS (Methode 8): Rₜ = 1.16 min. (m/z = 445 (M+H)⁺)
¹H-NMR (400MHz, DMSO-d₆): δ = 8.59 (s, 1H), 8.53 (t, 1H), 8.13 (d, 2H), 7.93 (s, 1H), 7.86 (t, 1H), 7.79 (d, 2H), 7.58 (s, 1H), 6.74 (s, breit, 2H), 3.80 (q, 2H), 3.54 (q, 2H).

### Beispiel 11

### Ethyl-4-({2-[(4-amino-5-cyano-1,3-thiazol-2-yl)amino]ethyl}amino)-6-(2,4-dichlorphenyl)-pyrazolo[1,5-a]pyrazin-2-carboxylat

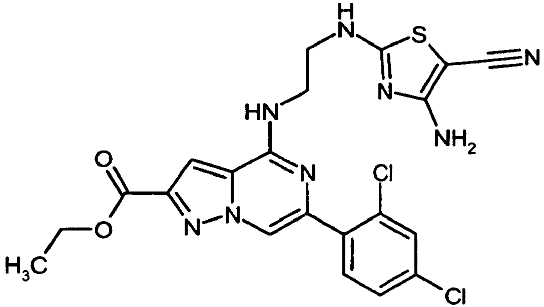

Analog der für Beispiel 3 beschriebenen Vorschrift wurden aus 300 mg (0.769 mmol) 4-Chlor-6-(2,4-dichlorphenyl)pyrazolo[1,5-a]pyrazin-2-carbonsäureethylester (Beispiel 12A) durch Umsetzung mit 239 mg (0.92 mmol) 4-Amino-2-[(2-aminoethyl)amino]-1,3-thiazol-5-carbonitril Dihydrochlorid (Beispiel 8A) und Aufreinigung durch präparative HPLC 110 mg (26% d. Th.) des Produktes als Feststoff erhalten.
LCMS (Methode 3): Rₜ = 2.58 min. (m/z = 517 (M+H)⁺)
¹H-NMR (400MHz, DMSO-d₆): δ = 8.49 (t, 1H), 8.32 (s, 1H), 8.17 (t, 1H), 7.74 (d, 1H), 7.66 (d, 1H), 7.58 (s, 1H), 7.53 (dd, 1H), 6.70 (s, br, 2H), 4.35 (q, 2H), 3.66 (dd, 2H), 3.52 (dd, 2H), 1.34 (t, 3H).

### Beispiel 12

### Ethyl-4-{3-[(4-amino-5-cyano-1,3-thiazol-2-yl)amino]piperidin-1-yl}-6-(2,4-dichlorphenyl)-pyrazolo[1,5-a]pyrazin-2-carboxylat

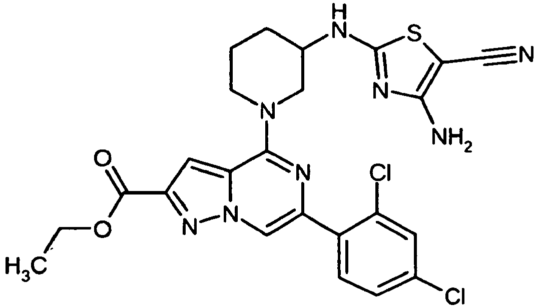

Analog der für Beispiel 3 beschriebenen Vorschrift wurden aus 52.1 mg (0.133 mmol) 4-Chlor-6-(2,4-dichlorphenyl)pyrazolo[1,5-a]pyrazin-2-carbonsäureethylester (Beispiel 12A) durch Umsetzung mit 132 mg (0.2 mmol) 4-Amino-2-(piperidin-3-ylamino)-1,3-thiazol-5-carbonitril Dihydrochlorid (Beispiel 47A) und Aufreinigung durch präparative HPLC 52 mg (70% d. Th.) des Produktes als Feststoff erhalten.
LCMS (Methode 8): Rₜ = 1.47 min. (m/z = 557 (M+H)⁺)
¹H-NMR (400MHz, DMSO-d₆): δ = 8.8 (s, 2H), 7.74 (d, 1H), 7.72 (d, 1H), 7.6 (s, 1H), 7.54 (dd, 1H), 6.72 (s, 2H), 4.3-4.44 (m, 3H), 4.08 (d, 1H), 3.86 (s, br, 1H), 3.4-3.5 (m, 2H), 2.04 (m, 1H), 1.90 (m, 1H), 1.65 (t, 2H), 1.35 (t, 3H).

### Beispiel 13

### Ethyl-4- {3-[(6-amino-5-nitropyridin-2-yl)amino]piperidin-1-yl}-6-(2,4-dichlorphenyl)pyrazolo-[1,5-a]pyrazin-2-carboxylat Trifluoracetat

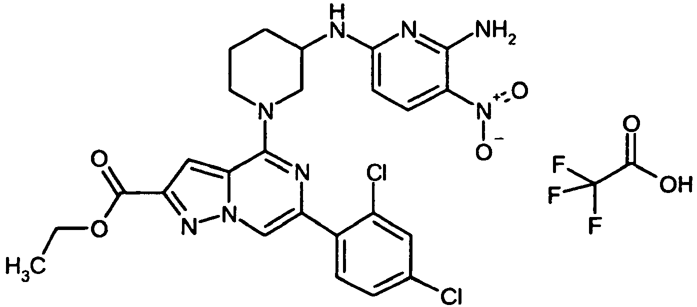

Analog der für Beispiel 3 beschriebenen Vorschrift wurden aus 70 mg (0.179 mmol) 4-Chlor-6-(2,4-dichlorphenyl)pyrazolo[1,5-a]pyrazin-2-carbonsäureethylester (Beispiel 12A) durch Umsetzung mit 83 mg (0.27 mmol) 3-Nitro-N⁶-(piperidin-3-yl)pyridin-2,6-diamin Dihydrochlorid (Beispiel 42A) und Aufreinigung durch präparative HPLC 83 mg (67% d. Th.) des Produktes als Feststoff erhalten.
LCMS (Methode 6): Rₜ = 2.56 min. (m/z = 571 (M+H)⁺)
¹H-NMR (400MHz, DMSO-d₆): δ = 8.48 (s, 1H), 8.14 (s, br, 1H), 7.95 (d, 1H), 7.90 (d, 1H), 7.72 (m, 3H), 7.51 (dd, 1H), 7.39 (s, 1H), 5.92 (d, 1H), 4.32 (q, 2H), 4.19 (d, 1H), 3.98 (m, 1H), 3.64 (m, 2H), 2.0 (m, 2H), 1.69 (m, 2H), 1.30 (t, 3H).

### Beispiel 14

### Ethyl-4-{3-[(6-amino-5-nitropyridin-2-yl)amino]piperidin-1-yl}-6-(2,4-dichlorphenyl)pyrazolo-[1,5-a]pyrazin-2-carboxylat Trifluoracetat

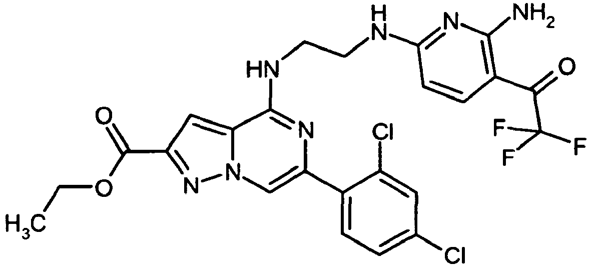

Analog der für Beispiel 3 beschriebenen Vorschrift wurden aus 70 mg (0.179 mmol) 4-Chlor-6-(2,4-dichlorphenyl)pyrazolo[1,5-a]pyrazin-2-carbonsäureethylester (Beispiel 12A) durch Umsetzung mit 83 mg (0.27 mmol) 1-{2-Amino-6-[(2-aminoethyl)amino]pyridin-3-yl}-2,2,2-trifluorethanon Hydrochlorid (Beispiel 34A) und Aufreinigung durch präparative HPLC (Laufmittel: Gradient Acetonitril/Wasser mit 0.1% Trifluoressigsäure) 83 mg (67% d. Th.) des Produktes als Feststoff erhalten.
LCMS (Methode 6): Rₜ = 2.56 min. (m/z = 571 (M+H)⁺)
¹H-NMR (400MHz, DMSO-d₆): δ = 8.48 (s, 1H), 8.14 (s, br, 1H), 7.95 (d, 1H), 7.90 (d, 1H), 7.72 (m, 3H), 7.51 (dd, 1H), 7.39 (s, 1H), 5.92 (d, 1H), 4.32 (q, 2H), 4.19 (d, 1H), 3.98 (m, 1H), 3.64 (m, 2H), 2.0 (m, 2H), 1.69 (m, 2H), 1.30 (t, 3H).

### Beispiel 15

### Ethyl-4-(3-{[4-amino-5-(methoxycarbonyl)-1,3-thiazol-2-yl]amino}piperidin-1-yl)-6-(2,4-dichlorphenyl)pyrazolo[1,5-a]pyrazin-2-carboxylat

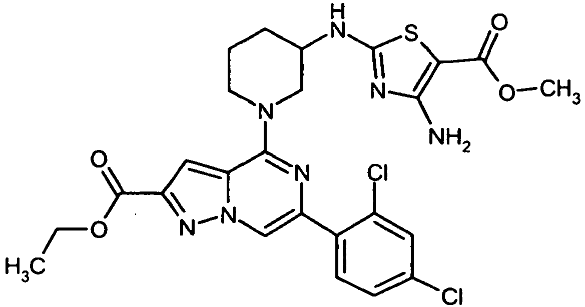

Analog der für Beispiel 3 beschriebenen Vorschrift wurden aus 63 mg (0.162 mmol) 4-Chlor-6-(2,4-dichlorphenyl)pyrazolo[1,5-a]pyrazin-2-carbonsäureethylester (Beispiel 12A) durch Umsetzung mit 89.9 mg (0.243 mmol) Methyl-4-amino-2-(piperidin-3-ylamino)-1,3-thiazol-5-carboxylat Dihydrochlorid (Beispiel 45A) und Aufreinigung durch präparative HPLC 68 mg (69% d. Th.) des Produktes als Feststoff erhalten.
LCMS (Methode 6): Rₜ = 2.58 min. (m/z = 590 (M+H)⁺)
¹H-NMR (400MHz, DMSO-d₆): δ = 8.50 (s, 1H), 8.47 (d, 1H), 7.72 (d, 2H), 7.65 (s, 1H), 7.52 (dd, 1H), 6.81 (s, br, 2H), 4.37 (m, 3H), 4.13 (dt, 1H), 3.87 (m, 1H), 3.61 (s, 3H), 2.06 (m, 1H), 1.92 (m, 1H), 1.65 (m, 2H), 1.34 (t, 3H).

### Beispiel 16

### Ethyl-4- {3-[(6-amino-5-nitropyridin-2-yl)amino]piperidin-1-yl}-6-(2,4-dichlorphenyl)pyrazolo-[1,5-a]pyrazin-2-carboxylat Trifluoracetat

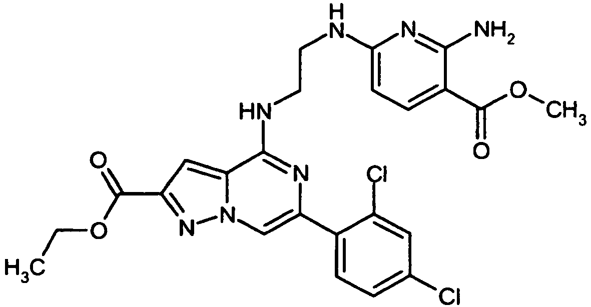

Analog der für Beispiel 3 beschriebenen Vorschrift wurden aus 70 mg (0.179 mmol) 4-Chlor-6-(2,4-dichlorphenyl)pyrazolo[1,5-a]pyrazin-2-carbonsäureethylester (Beispiel 12A) durch Umsetzung mit 83 mg (0.27 mmol) Methyl-2-amino-6-[(2-aminoethyl)amino]pyridin-3-carboxylat Dihydrochlorid (Beispiel 53A) und Aufreinigung durch präparative HPLC (Laufmittel: Gradient Acetonitril/Wasser mit 0.1% Trifluoressigsäure) 83 mg (67% d. Th.) des Produktes als Feststoff erhalten.
LCMS (Methode 6): Rₜ = 2.56 min. (m/z = 571 (M+H)⁺)
¹H-NMR (400MHz, DMSO-d₆): δ = 8.48 (s, 1H), 8.14 (s, br, 1H), 7.95 (d, 1H), 7.90 (d, 1H), 7.72 (m, 3H), 7.51 (dd, 1H), 7.39 (s, 1H), 5.92 (d, 1H), 4.32 (q, 2H), 4.19 (d, 1H), 3.98 (m, 1H), 3.64 (m, 2H), 2.0 (m, 2H), 1.69 (m, 2H), 1.30 (t, 3H).

### Beispiel 17

### Ethyl-4- {3-[(5-acetyl-4-amino-1,3-thiazol-2-yl)amino]piperidin-1-yl}-6-(2,4-dichlorphenyl)-pyrazolo[1,5-a]pyrazin-2-carboxylat Trifluoracetat

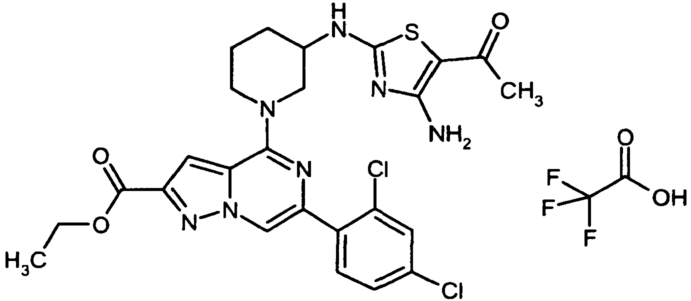

Analog der für Beispiel 3 beschriebenen Vorschrift wurden aus 61 mg (0.155 mmol) 4-Chlor-6-(2,4-dichlorphenyl)pyrazolo[1,5-a]pyrazin-2-carbonsäureethylester (Beispiel 12A) durch Umsetzung mit 73 mg (0.233 mmol) 1-[4-Amino-2-(piperidin-3-ylamino)-1,3-thiazol-5-yl]ethanon Dihydrochlorid (Beispiel 50A) und Aufreinigung durch präparative HPLC (Laufmittel: Gradient Acetonitril/Wasser mit 0.1 % Trifluoressigsäure) 63 mg (59% d. Th.) des Produktes als Feststoff erhalten.
LCMS (Methode 6): Rₜ = 2.40 min. (m/z = 574 (M+H)⁺)
¹H-NMR (400MHz, DMSO-d₆): δ = 8.65 (d, 1H), 8.51 (s, 1H), 7.74 (d, 1H), 7.71 (d, 1H), 7.64 (s, 1H), 7.52 (dd, 1H), 4.31-4.43 (m, 3H), 4.13 (d, 2H), 3.85 (s, br, 1H), 3.44 (m, 2H), 2.06 (m, 1H), 1.98 (s, 3H), 1.92 (m, 1H), 1.6-1.72 (m, 2H), 1.34 (t, 3H).

Analog der für Beispiel 4 beschriebenen Vorschrift wurden die jeweiligen Ester durch Hydrolyse mit Lithium- oder Natriumhydroxid-Lösung in die entsprechenden Säuren überführt.

| **Bsp.** | **Struktur** | **Charakterisierung** |
|---|---|---|
| **18** | 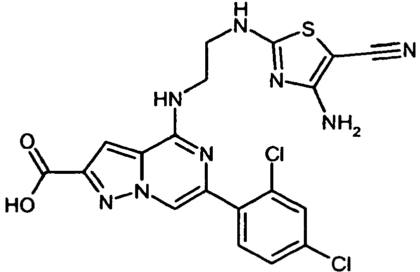 | LC/MS (Methode 8): Rₜ = 1.11 min, (m/z = 489 (M+H)⁺) |
| **19** | 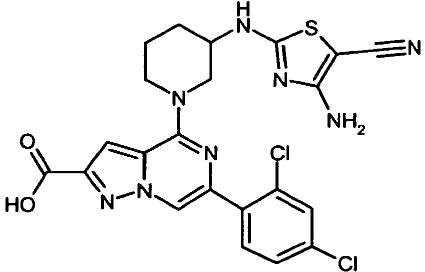 | LC/MS (Methode 6): Rₜ = 2.10 min, (m/z = 529 (M+H)⁺) |
| **20** | 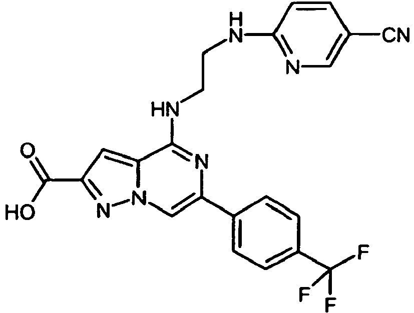 | LC/MS (Methode 6): Rₜ = 1.98 min, (m/z = 468 (M+H)⁺) |
| | | ¹H-NMR (400MHz, DMSO-d₆): δ = 8.79 (s, 1H), 8.45 (s, 1H), 8.24 (d, 2H), 8.18 (t, 1H), 7.84 (br, 1H), 7.79 (d, 2H), 7.63 (d, 1H), 7.51 (s, 1H), 6.53 (m, 1H), 3.79 (m, 2H), 3.66 (m, 2H). |

Folgende Amide wurden analog der allgemeinen Versuchsbeschreibung für Amidkupplungen (vgl. Beispiel 5) aus den entsprechenden Carbonsäuren hergestellt.

| **Bsp.** | **Struktur** | **Charakterisierung** |
|---|---|---|
| **21** | 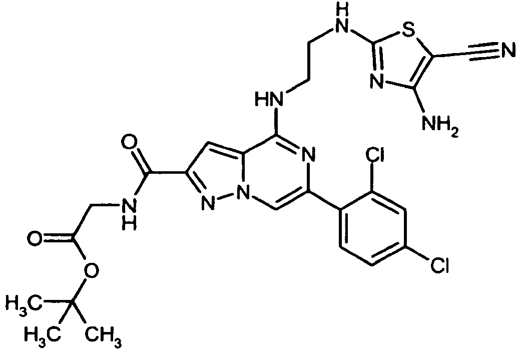 | LC/MS (Methode 3): Rₜ = 2.69 min, (m/z = 602 (M+H)⁺) |
| **22** | 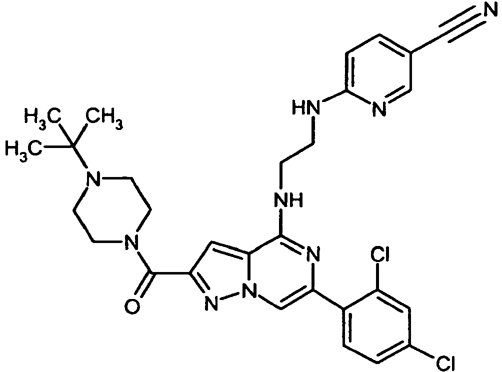 | LC/MS (Methode 6): Rₜ = 1.38 min, (m/z = 592 (M+H)⁺) |
| **23** | 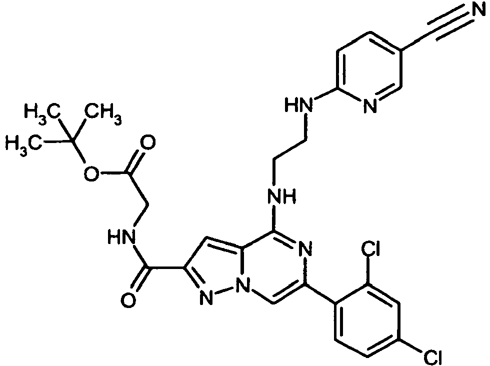 | LC/MS (Methode 3): Rₜ = 2.95 min, (m/z = 581 (M+H)⁺) |
| **24** | 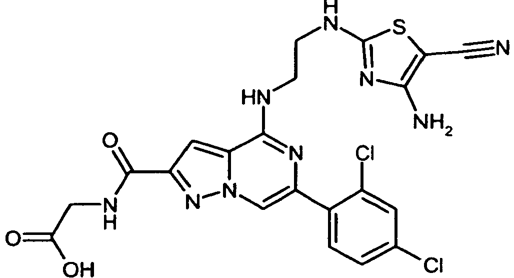 | LC/MS (Methode 6): Rₜ = 1.74 min, (m/z = 546 (M+H)⁺) |
| **25** | 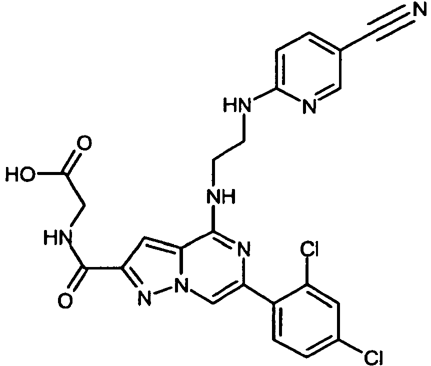 | LC/MS (Methode 3): Rₜ = 2.43 min, (m/z = 525 (M+H)⁺) |
| **26** | 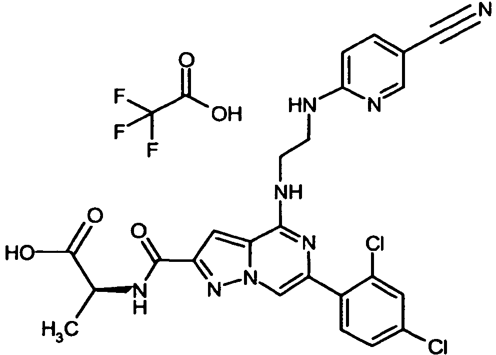 | LC/MS (Methode 8): Rₜ = 1.22 min, (m/z = 539 (M+H)⁺) |
| **27** | 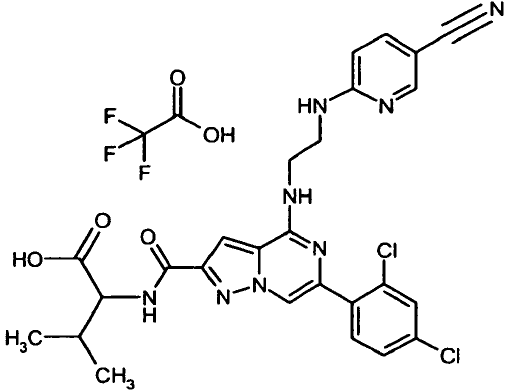 | LC/MS (Methode 6): Rₜ = 2.17 min, (m/z = 567 (M+H)⁺) |
| **28** | 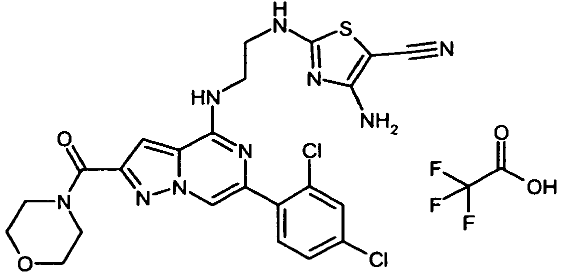 | LC/MS (Methode 9): Rₜ = 2.20 min, (m/z = 558 (M+H)⁺) |
| **29** | 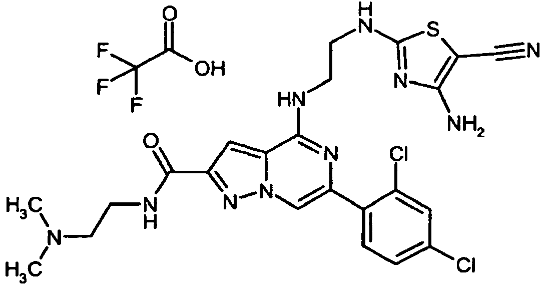 | LC/MS (Methode 3): Rₜ = 1.57 min, (m/z = 559 (M+H)⁺) |
| **30** | 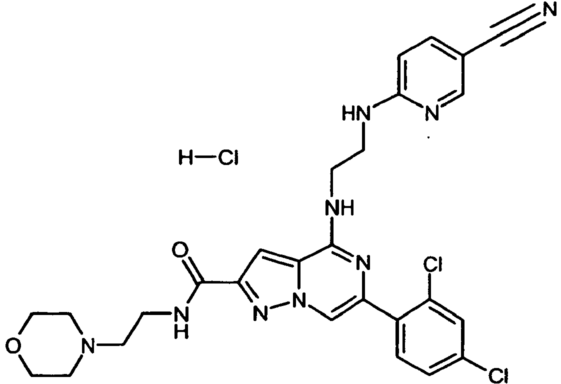 | LC/MS (Methode 3): Rₜ = 1.63 min, (m/z = 580 (M+H)⁺) |
| **31** | 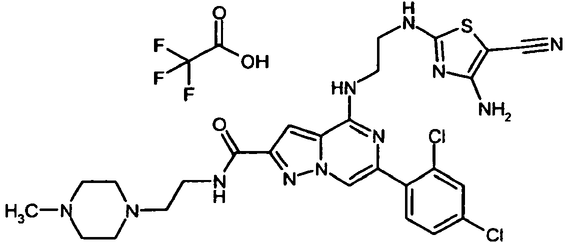 | LC/MS (Methode 6): Rₜ = 1.24 min, (m/z = 614 (M+H)⁺) |
| **32** | 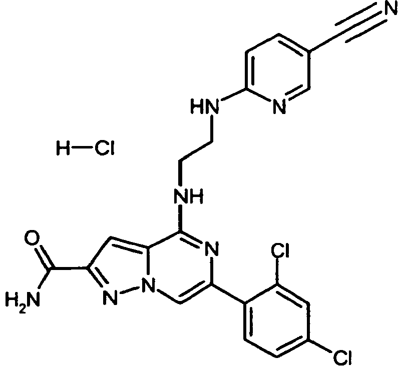 | LC/MS (Methode 8): Rₜ = 1.22 min, (m/z = 467 (M+H)⁺) |
| **33** | 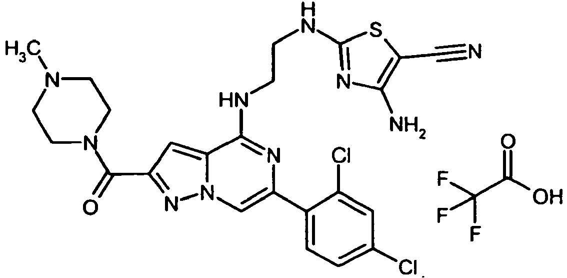 | LC/MS (Methode 3): Rₜ = 1.57 min, (m/z = 571 (M+H)⁺) |

### Beispiel 34

### 1- {2-Amino-6-[(2-{[6-(2,4-dichlorphenyl)-2-(morpholin-4-ylmethyl)pyrazolo[1,5-a]pyrazin-4-yl]amino}ethyl)amino]pyridin-3-yl} -2,2,2-trifluorethanon Trifluoracetat

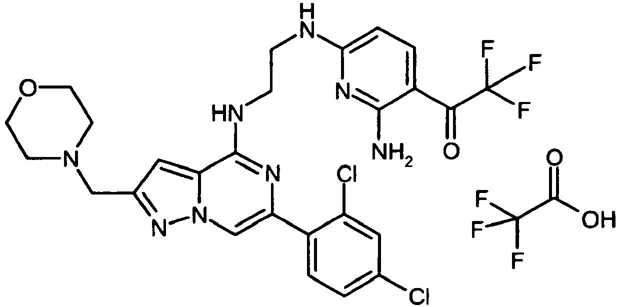

Analog der für Beispiel 1 beschriebenen Vorschrift wurden aus 60 mg (0.151 mmol) 4-Chlor-6-(2,4-dichlorphenyl)-2-(morpholin-4-ylmethyl)pyrazolo[1,5-a]pyrazin (Beispiel 22A) durch Umsetzung mit 53 mg (0.181 mmol) 1-{2-Amino-6-[(2-aminoethyl)amino]pyridin-3-yl}-2,2,2-trifluorethanon Hydrochlorid (Beispiel 34A) und Aufreinigung durch präparative HPLC 62 mg (57% d. Th.) des Produktes als Feststoff erhalten.
LCMS (Methode 3): Rₜ = 1.84 min. (m/z = 609 (M+H)⁺)
¹H-NMR (400MHz, DMSO-d₆): δ = 10.41 (s, br, 1H), 8.54 (s, 1H), 8.26 (s, 1H), 8.10 (s, 2H), 7.73 (d, 1H), 7.68 (d, 1H), 7.54 (s, br, 1H), 7.48 (dd, 1H), 7.14 (s, 1H), 5.91 (d, 1H), 4.59 (s, 2H), 3.96 (m, 2H), 3.51-3.75 (m, 6H), 3.39 (m, 2H), 3.19 (m, 2H).

Analog der für die Herstellung von Beispiel 1 beschriebenen Vorschrift wurden folgende Produkte aus den entsprechenden Chloriden durch Umsetzung mit den entsprechenden Aminen synthetisiert.

| **Bsp.** | **Struktur** | **Charakterisierung** |
|---|---|---|
| **35** | 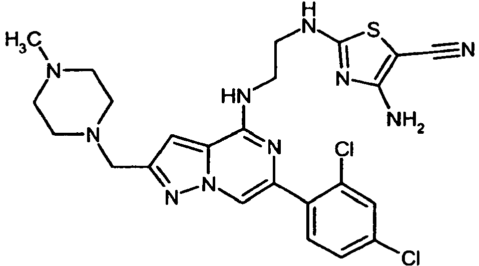 | LC/MS (Methode 3): Rₜ = 1.56 min, (m/z = 557 (M+H)⁺) |
| **36** | 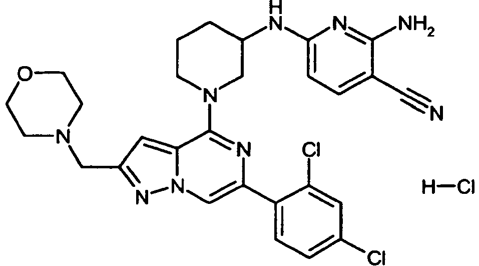 | LC/MS (Methode 3): Rₜ = 1.74 min, (m/z = 578 (M+H)⁺) |
| **37** | 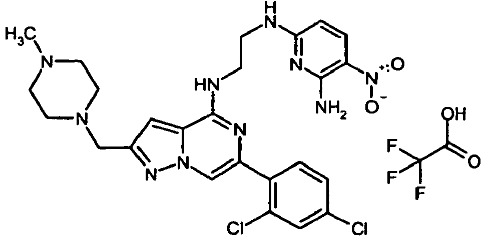 | LC/MS (Methode 8): Rₜ = 0.98 min, (m/z = 571 (M+H)⁺) |
| **38** | 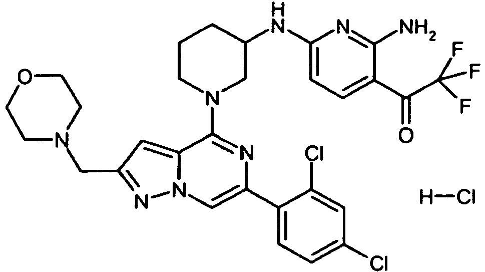 | LC/MS (Methode 6): Rₜ = 1.73 min, (m/z = 649 (M+H)⁺) |
| **39** | 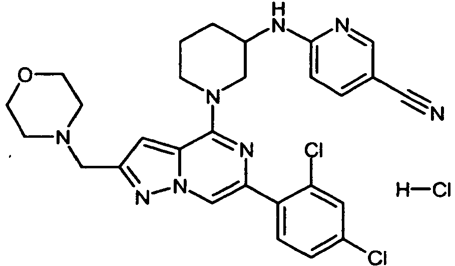 | LC/MS (Methode 3): Rₜ = 1.84 min, (m/z = 563 (M+H)⁺) |
| **40** | 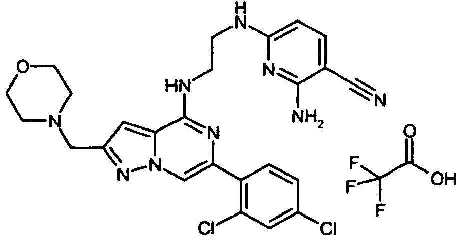 | LC/MS (Methode 3): Rₜ = 1.59 min, (m/z = 538 (M+H)⁺) |

### Beispiel 41

### 4-Amino-2-[(2-{[6-(2,4-dichlorphenyl)-2-(hydroxymethyl)pyrazolo[1,5-a]pyrazin-4-yl]amino}-ethyl)amino]-1,3-thiazol-5-carbonitril

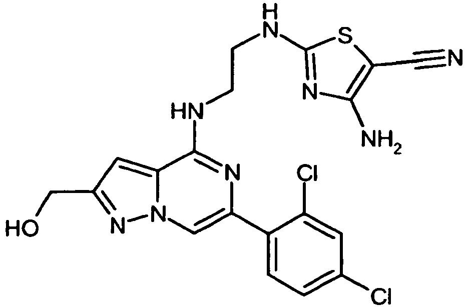

In 10 ml THF wurden 100 mg (0.184 mmol) Ethyl-4-({2-[(4-amino-5-cyano-1,3-thiazol-2-yl)amino]ethyl}amino)-6-(2,4-dichlorphenyl)pyrazolo[1,5-a]pyrazin-2-carboxylat (Beispiel 11) gelöst und bei RT wurden 0.276 ml (0.276 mmol) einer Lösung von Lithiumaluminiumhydrid (1 mol/l) in THF zugetropft. Man rührte für 2 h, gab nach vollständigem Umsatz zuerst Methanol zu und stellte dann mit verdünnter Salzsäure pH=5 ein. Es wurde mehrmals mit Essigsäureethylester extrahiert. Nach Entfernen des Lösungsmittels und Aufreinigung durch präparative HPLC wurden 61 mg (64% d. Th.) des Produktes als Feststoff erhalten.
LCMS (Methode 8): Rₜ = 1.10 min. (m/z = 475 (M+H)⁺)
¹H-NMR (400MHz, DMSO-d₆): δ = 8.50 (t, 1H), 8.15 (s, 1H), 7.91 (t, 1H), 7.70 (d, 1H), 7.68 (d, 1H), 7.51 (dd, 1H), 6.95 (s, 2H), 6.69 (s, br, 2H), 4.62 (s, 2H), 3.64 (dd, 2H), 3.51 (m, 2H).

### Beispiel 42

### 4-Amino-2-[(2-{[6-(2,4-dichlorphenyl)-2-(hydroxymethyl)pyrazolo[1,5-a]pyrazin-4-yl]amino}-ethyl)amino]-1,3-thiazol-5-carbonitril

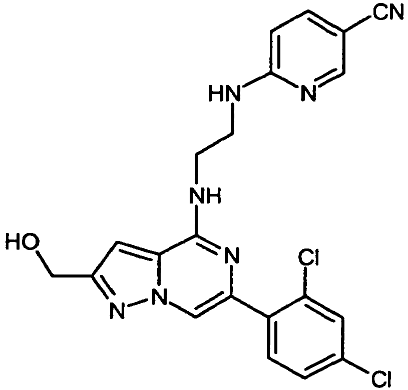

In 10 ml THF wurden 100 mg (0.184 mmol) Ethyl-4-({2-[(4-amino-5-cyano-1,3-thiazol-2-yl)amino]ethyl}amino)-6-(2,4-dichlorphenyl)pyrazolo[1,5-a]pyrazin-2-carboxylat (Beispiel 3) gelöst und bei RT wurden 0.276 ml (0.276 mmol) einer Lösung von Lithiumaluminiumhydrid (1 mol/l) in THF zugetropft. Man rührte für 2 h, gab nach vollständigem Umsatz zuerst Methanol zu und stellte dann mit verdünnter Salzsäure pH=5 ein. Es wurde mehrmals mit Essigsäureethylester extrahiert. Nach Entfernen des Lösungsmittels und Aufreinigung durch präparative HPLC wurden 61 mg (64% d. Th.) des Produktes als Feststoff erhalten.
LCMS (Methode 8): Rₜ = 1.10 min. (m/z = 475 (M+H)⁺)
¹H-NMR (400MHz, DMSO-d₆): δ = 8.50 (t, 1H), 8.15 (s, 1H), 7.91 (t, 1H), 7.70 (d, 1H), 7.68 (d, 1H), 7.51 (dd, 1H), 6.95 (s, 2H), 6.69 (s, br, 2H), 4.62 (s, 2H), 3.64 (dd, 2H), 3.51 (m, 2H).

Analog der für Beispiel 2 beschriebenen Vorschrift wurden folgende Produkte aus 6-({2-[(6-Bromimidazo[1,2-a]pyrazin-8-yl)amino]ethyl}amino)pyridin-3-carbonitril (Beispiel 23A) durch Palladium-kartalysierte Reaktion mit den entsprechenden Boronsäuren erhalten.

| **Bsp.** | **Struktur** | **Charakterisierung** |
|---|---|---|
| **43** | 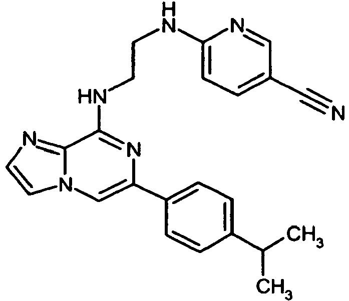 | LC/MS (Methode 8): Rₜ = 1.32 min, (m/z = 398 (M+H)⁺) |
| **44** | 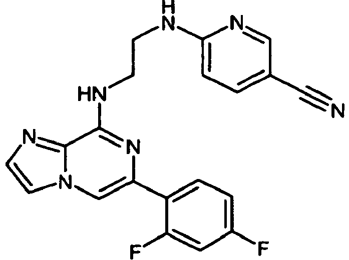 | LC/MS (Methode 10): Rₜ = 2.02 min, (m/z = 392 (M+H)⁺) |
| **45** | 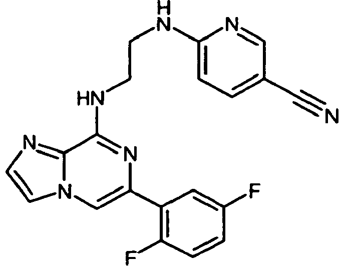 | LC/MS (Methode 11): Rₜ = 2.04 min, (m/z = 392 (M+H)⁺) |
| **46** | 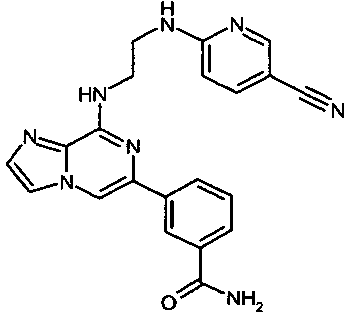 | LC/MS (Methode 10): Rₜ = 1.43 min, (m/z = 399 (M+H)⁺) |
| **47** | 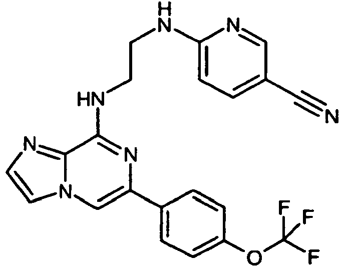 | LC/MS (Methode 11): Rₜ, = 2.16 min, (m/z = 440 (M+H)⁺) |
| **48** | 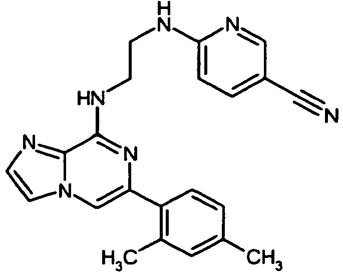 | LC/MS (Methode 11): Rₜ = 1.66 min, (m/z = 384 (M+H)⁺) |
| **49** | 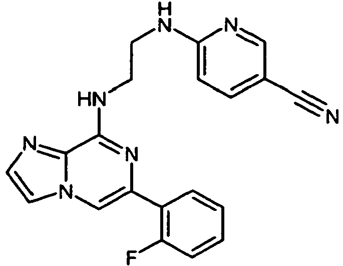 | LC/MS (Methode 11): Rₜ = 1.92 min, (m/z = 374 (M+H)⁺) |
| **50** | 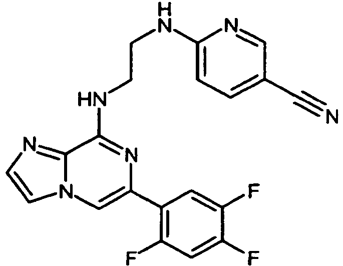 | LC/MS (Methode 10): Rₜ = 2.11 min, (m/z = 410 (M+H)⁺) |
| **51** | 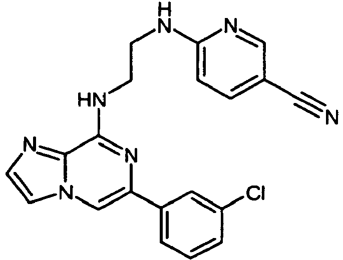 | LC/MS (Methode 11): Rₜ, = 2.08 min, (m/z = 390 (M+H)⁺) |
| **52** | 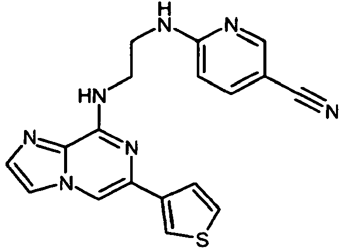 | LC/MS (Methode 10): Rₜ = 1.82 min, (m/z = 362 (M+H)⁺) |

### Beispiel 53

### 6-[(2-{[6-(2,4-Dichlorphenyl)-2-(morpholin-4-ylmethyl)pyrazolo[1,5-a]pyrazin-4-yl]amino}ethyl)-amino]nicotinnitril Hydrochlorid

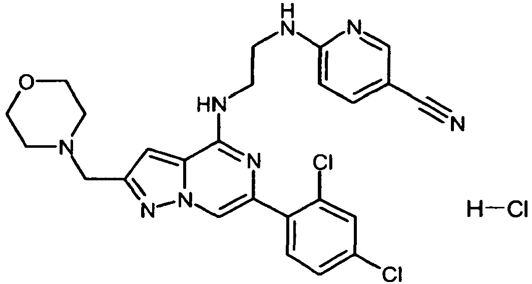

30 mg (0.066 mmol) 6-[(2-{[6-(2,4-Dichlorphenyl)-2-formylpyrazolo[1,5-a]pyrazin-4-yl]amino}ethyl)amino]pyridin-3-carbonitril (Beispiel 54A) wurden in 1 ml Methanol gelöst und 11.6 mg (0.133 mmol) Morpholin, 4Å Molsieb und 11.9 mg (0.199 mmol) Essigsäure wurden zugegeben. Zuletzt gab man 8.3 mg (0.133 mmol) Natriumcyanoborhydrid zu und rührte für 1 h bei RT. Der Rohansatz wurde mit 2N Salzsäure angesäuert und mittels präparative HPLC gereinigt. Nach Lyophilisieren wurden 25 mg (58% d. Th.) des Produktes als Feststoff erhalten.
LCMS (Methode 3): Rₜ = 1.74 min. (m/z = 523 (M+H)⁺).
¹H-NMR (400MHz, DMSO-d₆): δ = 11.0 (br, 1H), 8.39 (d, 1H), 8.27 (m, 1H), 8.23 (s, 1H), 7.87 (br, 1H), 7.74 (d, 1H), 7.67 (d, 1H), 7.61 (m, 1H), 7.54 (dd, 1H), 7.23 (s, 1H), 6.57 (m, 1H), 4.57 (s, 2H), 3.95 (m, 4H), 3.62 (m, 4H), 3.49 (m, 2H), 3.18 (m, 2H).

Analog der für die Herstellung von Beispiel 53 beschriebenen Vorschrift wurden folgende Produkte durch reduktive Aminierung, ausgehend von 6-[(2-{[6-(2,4-Dichlorphenyl)-2-formylpyrazolo[1,5-a]pyrazin-4-yl]amino}ethyl)amino]pyridin-3-carbonitril (Beispiel 54A) bzw. mit den entsprechenden Aminen, erhalten.

| **Bsp.** | **Struktur** | **Charakterisierung** |
|---|---|---|
| **54** | 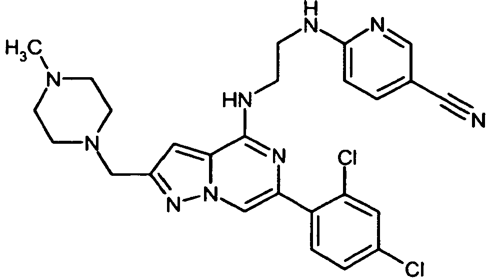 | LCMS (Methode 9): Rₜ = 1.72 min. (m/z = 536 (M+H)⁺). |
| | | ¹H-NMR (400MHz, DMSO-d₆): δ = 8.40 (d, 1H), 8.23 (s, 1H), 8.18 (m, 1H), 7.86 (br, 1H), 7.74 (d, 1H), 7.66 (d, 1H), 7.61 (m, 1H), 7.52 (dd, 1H), 7.13 (m, 1H), 6.50 (m, 1H), 3.50 (m, 2H), 3.20 (m, 2H), 2.81 (s, 3H). |
| | | Weitere Signale überlappen mit dem Wassersignal. |
| **55** | 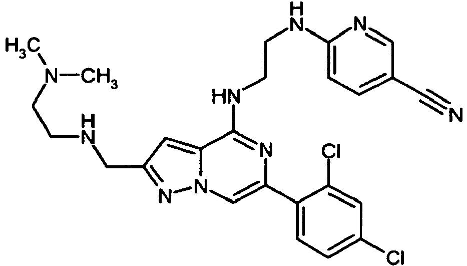 | LCMS (Methode 9): Rₜ = 1.50 min. (m/z = 524 (M+H)⁺). |
| | | ¹H-NMR (400MHz, DMSO-d₆): δ = 10.74 (br, 1H), 9.83 (br, 1H), 8.42 (d, 1H), 8.29 (m, 1H), 8.23 (s, 1H), 7.94 (br, 1H), 7.75 (d, 1H), 7.69 (d, 1H), 7.54 (dd, 1H), 7.20 (s, 1H), 6.56 (m, 1H), 4.45 (m, 2H), 3.66 (m, 4H), 3.47 (m, 4H), 2.85 (d, 3H). |
| **56** | 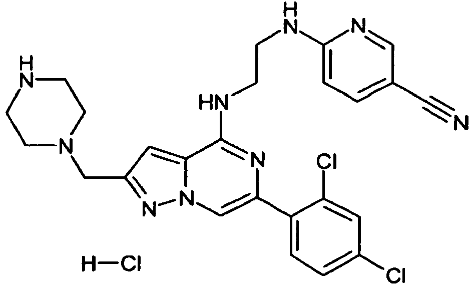 | LCMS (Methode 9): Rₜ = 1.71 min. (m/z = 522 (M+H)⁺). |
| | | ¹H-NMR (400MHz, DMSO-d₆): δ = 11.75 (br, 1H), 9.25 (br, 2H), 8.40 (d, 1H), 8.23 (s, 2H), 7.84 (br, 1H), 7.74 (d, 1H), 7.66 (d, 1H), 7.60 (d, 1H), 7.53 (dd, 1H), 7.21 (s, 1H), 6.57 (m, 1H), 4.50 (br s, 2H). Weitere Signale überlappen mit dem Wassersignal. |
| **57** | 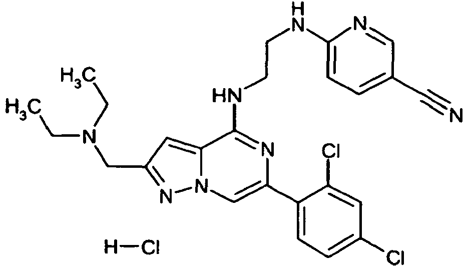 | LCMS (Methode 9): R, = 1.83 min. (m/z = 509 (M+H)⁺). |
| | | ¹H-NMR (400MHz, DMSO-d₆): δ = 10.25 (br, 1H), 8.39 (d, 1H), 8.24 (s, 1H), 8.22 (m, 1H), 7.87 (br, 1H), 7.74 (d, 1H), 7.68 (d, 1H), 7.61 (br d, 1H), 7.53 (dd, 1H), 7.24 (s, 1H), 6.56 (m, 1H), 4.54 (m, 2H), 3.65 (m, 4H), 3.13 (m, 4H), 1.31 (t, 6H). |
| **58** | 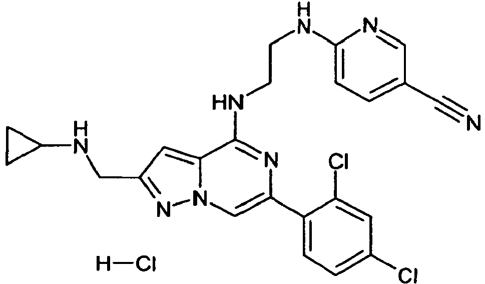 | LCMS (Methode 9): Rₜ = 1.79 min. (m/z = 493 (M+H)⁺). |
| | | ¹H-NMR (400MHz, DMSO-d₆): δ = 8.37 (d, 1H), 8.13 (s, 1H), 7.85 (m, 1H), 7.76 (br, 1H), 7.72 (d, 1H), 7.68 (d, 1H), 7.59 (d, 1H), 7.51 (dd, 1H), 6.90 (s, 1H), 6.54 (m, 1H), 3.89 (s, 2H), 3.64 (m, 4H), 2.15 (m, 1H), 0.37 (m, 2H), 0.28 (m, 2H). |
| **59** | 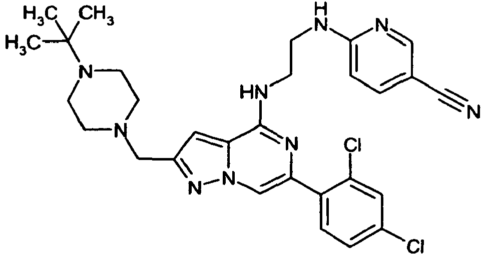 | LCMS (Methode 9): Rₜ = 1.94 min. (m/z = 578 (M+H)⁺). |
| | | ¹H-NMR (400MHz, DMSO-d₆): δ = 11.25 (br, 1H), 8.39 (d, 1H), 8.27 (m, 1H), 8.23 (s, 1H), 7.89 (br, 1H), 7.75 (d, 1H), 7.68 (d, 1H), 7.62 (d, 1H), 7.54 (dd, 1H), 7.24 (s, 1H), 6.57 (m, 1H), 4.58 (m, 2H), 3.68 (m, 8H), 3.45 (m, 4H), 1.36 (s, 9H). |
| **60** | 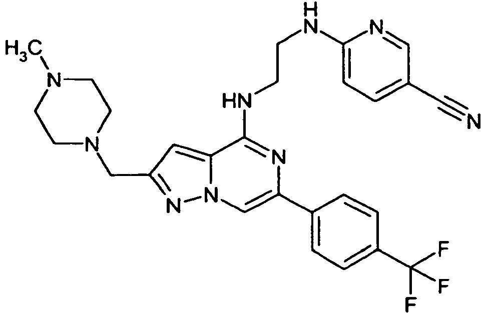 | LCMS (Methode 8): Rₜ = 1.04 min. (m/z = 536 (M+H)⁺). |
| | | ¹H-NMR (400MHz, DMSO-d₆): δ = 8.73 (s, 1H), 8.45 (s, 1H), 8.25 (d, 2H), 8.16 (br, 1H), 7.91 (br, 1H), 7.77 (d, 2H), 7.64 (m, 1H), 7.14 (m, 1H), 6.56 (m, 1H), 2.81 (s, 3H). |
| | | Weitere Signale überlappen mit dem Wassersignal. |
| **61** | 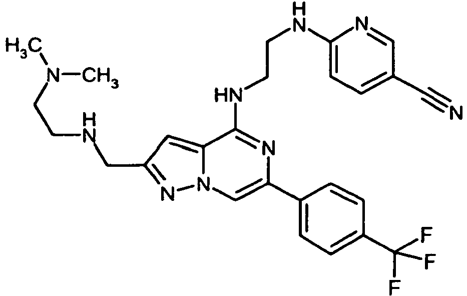 | LCMS (Methode 8): Rₜ = 0.95 min. (m/z = 524 (M+H)⁺). |
| | | ¹H-NMR (400MHz, DMSO-d₆): δ = 10.61 (br, 1H), 9.75 (br, 2H), 8.70 (s, 1H), 8.45 (m, 1H), 8.26 (d, 2H), 8.22 (m, 1H), 7.90 7.79 (d, 2H), 7.64 (d, 1H), 7.17 (s, 1H), 6.54 (m, 1H), 4.47 (m, 2H), 3.79 (m, 2H), 2.86 (d, 6H). |
| | | Weitere Signale überlappen mit dem Wassersignal. |
| **62** | 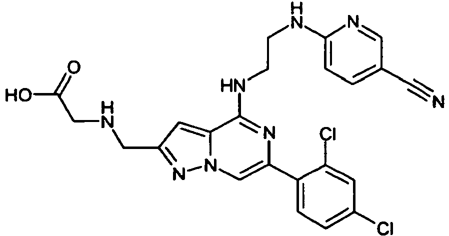 | LCMS (Methode 9): Rₜ = 1.84 min. (m/z = 511 (M+H)⁺). |
| | | ¹H-NMR (400MHz, DMSO-d₆): δ = 9.67 (br, 2H), 8.409 (d, 1H), 8.25 (m, 1H), 8.23 (s, 1H), 7.92 (br, 1H), 7.74 (d, 1H), 7.68 (d, 1H), 7.63 (d, 1H), 7.54 (dd, 2H), 7.16 (s, 1H), 6.57 (m, 1H), 4.41 (m, 2H), 3.96 (m, 2H), 3.64 (m, 4H). |

### Beispiel 63

### 4-({2-[(4-Amino-5-cyano-1,3-thiazol-2-yl)amino]ethyl}amino)-6-(2,4-dichlorphenyl)pyrazolo[1,5-a]pyrazin-2-carbonitril

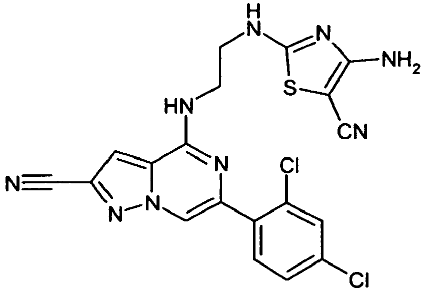

Analog der für Beispiel 3 beschriebenen Vorschrift wurden aus 60 mg (0.19 mmol) 4-Chlor-6-(2,4-dichlorphenyl)pyrazolo[1,5-a]pyrazin-2-carbonitril (Beispiel 57A) durch Umsetzung mit 61.1 mg (0.28 mmol) 4-Amino-2-[(2-aminoethyl)amino]-1,3-thiazol-5-carbonitril Dihydrochlorid (Beispiel 8A) und Aufreinigung durch präparative HPLC 24 mg (28% d. Th.) des Produktes als Feststoff erhalten.
LCMS (Methode 3): Rₜ = 2.63 min. (m/z = 470 (M+H)⁺).
¹H-NMR (400MHz, DMSO-d₆): δ = 8.50 (t, 1H), 8.37 (s, 1H), 8.35 (t, 1H), 7.76 (d, 1H), 7.66 (m, 2H), 7.54 (dd, 1H), 6.69 (br, 2H), 3.78 (m, 2H), 3.53 (m, 2H).

### Beispiel 64

### 4-({2-[(5-Cyanopyridin-2-yl)amino]ethyl}amino)-6-(2,4-dichlorphenyl)pyrazolo[1,5-a]pyrazin-2-carbonitril

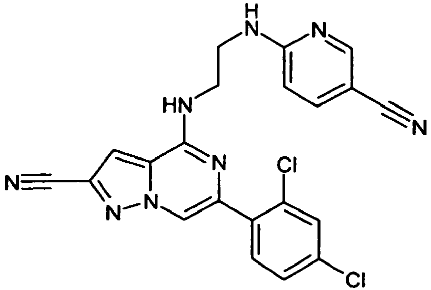

Analog der für Beispiel 3 beschriebenen Vorschrift wurden aus 60 mg (0.19 mmol) 4-Chlor-6-(2,4-dichlorphenyl)pyrazolo[1,5-a]pyrazin-2-carbonitril (Beispiel 57A) durch Umsetzung mit 61.1 mg (0.28 mmol) 6-[(2-Aminoethyl)amino]pyridin-3-carbonitril Dihydrochlorid (Beispiel 2A) und Aufreinigung durch präparative HPLC 42 mg (50% d. Th.) des Produktes als Feststoff erhalten.
LCMS (Methode 3): Rₜ = 2.82 min. (m/z = 449 (M+H)⁺).
¹H-NMR (400MHz, DMSO-d₆): δ = 8.34 (m, 3H), 7.76 (m, 2H), 7.67 (m, 2H), 7.60 (dd, 1H), 7.54 (dd, 1H), 6.51 (m, 1H), 3.69-3.57 (m, 4H).

### B) Bewertung der physiologischen Wirksamkeit

Die Eignung der erfindungsgemäßen Verbindungen zur Behandlung von hämatologischen Erkrankungen kann in folgenden Assaysystemen gezeigt werden:

### In vitro Assay

Die inhibitorische Aktivität von Wirksubstanzen wird in einem biochemischen Assay bestimmt. Die dazu benötigten Bestandteile werden in einer schwarzen 384-Loch-Mikrotitterplatte mit transparentem Boden (Firma Greiner, Katalognummer 781092) gemischt. Benötigt werden dabei pro Loch der 384-Loch-Mikrotitterplatte 5 nM GSK3ß (Firma Upstate, Katalognummer xy), 40 µM GSK3ß-Substrat GSM (Sequenz H-RRRPASVPPSPSLSRHS-(pS)-HQRR, Firma Upstate, Katalognummer 2-533), 30 µM Nicotinsäureamid-Adenin-Dinucleotid NADH (Roche Diagnostics, Katalognummer 10107735), 50 µM Adenosin-triphoshat ATP (Firma Sigma, Katalognummer A7966), und 2 mM Phosphoenolpyruvat (Firma Roche, Katalognummer 128112). Der benötigte Reaktionspuffer, in dem die biochemische Reaktion abläuft, besteht aus 50 mM Trizma Hydrochlorid Tris- HCl pH: 7,5 (Firma Sigma, Katalognummer T3253), 5 mM Magnesiumchlorid MgCl₂ (Firma Sigma, Katalognummer M8266), 0,2mM DL-Dithiothreitol DTT (Firma Sigma, Katalognummer D9779), 2 mM Ethylendiaminethertetrasäure EDTA (Firma Sigma, Katalognummer E6758), 0.01% Triton X-100 (Firma Sigma, Katalognummer T8787) und 0.05% Bovines Serumalbumin BSA (Firma Sigma, Katalognummer B4287).

Wirksubstanzen werden in Dimethylsulfoxid DMSO (Firma Sigma, Katalognummer D8418) in einer Konzentration von 10 mM gelöst. Wirksubstanzen werden in Konzentrationsreihen von 10 µM, 1 µM, 0.1 µM, 0.01 µM, 0.001 µM, 0.0001 µM, 0.00001 µM, 0.000001 µM zu den Ansätzen der biochemischen Reaktion zugegeben. Als Kontrolle wird statt Substanz Dimethylsulfoxid in einer Endkonzentration von 0.1 % zugesetzt.

Die Reaktion wird für 2 Stunden bei 30°C inkubiert und anschließend die entstandene Fluoreszenz in einem Tecan Safire-XFLUOR4-Gerät, Version V4.50 (Serienummer 12901300283) unter den Spezifikationen: Messmodus - Fluoreszenz, von unten gemessen, Extinktionswellenlänge 340 nm, Emissionswellenlänge 465 nm, Spaltbreite Extinktion 5 nm, Spaltbreite Emission 5 nm, Verstärkermodus 120, Verzögerung 0 µs, Anzahl Lichtblitze pro Messung 3, und einer Integrationszeit von 40 µs gemessen.

Die Aktivität der GSK3ß wird in Fluoreszenz-Einheiten ermittelt, wobei die Werte von nichtinhibierter Kinase gleich 100% und vollständig inhibierter Kinase gleich 0% gesetzt werden. Die Aktivität der Wirksubstanzen wird auf diese 0% und 100% verrechnet.

Repräsentative in-vitro-Wirkdaten für die erfindungsgemäßen Verbindungen sind in Tabelle A wiedergegeben:

**Tabelle A**

| **Beispiel-Nr.** | **IC₅₀ [nM]** |
|---|---|
| **2** | 16 |
| **4** | 21 |
| **5** | 30 |
| **9** | 310 |
| **17** | 8 |
| **19** | 3 |
| **24** | 5 |
| **58** | 80 |

### CD34+-Proliefrationsassays zur Testung von GSK3ß-Inhibitoren

Adulte hämatopoetische Stammzellen sind durch die spezifische Ausprägung von membranständigen Proteinen gekennzeichnet. Entsprechend ihrem Molekulargewicht sind diese Oberflächenmarker mit einer entsprechenden Nummer versehen. Zu dieser Klasse gehört auch das als CD34 bezeichnete Molekül, welches zur Identifizierung, Charakterisierung und Isolierung von adulten hämtopoetischen Stammzellen dient. Diese Stammzellen können dabei aus dem Knochenmark, dem peripheren Blut oder aus Nabelschnurblut isoliert werden. In in vitro-Kulturen sind diese Zellen begrenzt lebensfähig, können aber durch unterschiedlichste Zusätze zum Kulutrmedium zu Proliferation und Differenzierung angeregt werden. CD34-positive Zellen werden hier verwendet, um den Einfluss von Substanzen auf die Aktivität der Glykogen Synthase Kinase 3 zu testen. Zu diesem Zweck werden in einem ersten Schritt über differentielle Zentrifugationsschritte mononukleare Zellen aus Nabelschnurblut isoliert.

Dazu wird Nabelschnurblut 1:4 mit Phosphat-gepufferter Salzlösung verdünnt. 50 Milliliter Zentrifugationgefäße werden mit 17 Milliliter Ficoll (Dichte 1.077, Ficoll Paque Plus; Pharmacia, Katalognummer 17-1440-02) beschickt. Darauf werden 30 Milliliter des 1:4 verdünnten Nabelschnurblutes aufgeschichtet und anschließend für 30 Minuten bei 400 x g bei Raumtemperatur zentrifuigert. Die Bremsen der Zentrifuge sind dabei ausgeschaltet. Die mononukleären Zellen sammeln sich durch die Zentrifugation in der Interphase. Diese wird mit Hilfe einer 30 Milliliter-Pipette abgenommen und in ein neues 50 Milliliter Zentrifugationsgefäß überführt und das Volumen anschließend mit der Phosphat-gepufferten Salzlösung auf 30 ml aufgefüllt. Diese Zellen werden für 10 Minuten bei Raumtemperatur mit eingeschalteter Bremse bei 300 x g zentrifuigert. Der Überstand wird verworfen und das entstandene Zellpellet in 30 Milliliter Phosphat-gepufferter Salzlösung resuspendiert. Diese Zellen werden erneut für 15 Minuten bei 20°C bei 200 x g und eingeschalteter Bremse zentrifugiert.

Zur Isolierung der CD34-positiven Zellen aus die angereicherten mononukleären Zellen in einer Konzentration von 1 X 10⁸ Zellen pro 300 Mikroliter MACS-Puffer (0.5% Endotoxin-freies bovines Serumalbumin in Phosphat-gepufferter Salzlösung) resuspendiert. Es erfolgt die Zugabe von 100 Mikrolitern FCR Blocking Reagenz (Miltenyi Biotec, Katalognummer 130-046-702) sowie 100 Mikrolitern an CD34 Micro Beads (Miltenyi Biotec, Katalognummer 130-046-702). Diese Suspension wird für 30 Minuten bei 4°C inkubiert. Anschließend werden die Zellen mit dem 20-fachem Volumen MACS Puffer verdünnen und 10 Minuten bei 300 x g zentrifugiert. Der Überstand wird verworfen und die Zellen in 500 Mikrolitern MACS Puffer resuspendiert. Die so behandelten Zellen werden auf einer LS-Säule (Miltenyi Biotec, katalognummer 130-042-401) aufgetragen und unter Verwendung eines Midi MACS Magneten (Miltenyi Biotec, Katalognummer 130-042-303) aufgereinigt.

Die Anzahl an CD34-positiven Zellen wird über das auszählen der Zellen unter Verwendung einer Neubauer-Kammer durchgeführt. Die Bestimmung der Reinheit der Zellen erfolgt nach Standardprotokollen unter Verwendung der Fluorescent Activated Cell Sorting -Methode (Becton Dickinson, BD FACS™ Sample Prep Assistant SPAII Upgrade Kit, Katalognummer 337642).

Zur Bestimmung des Einflusses einer Modulation der GSK3-Aktivität werden CD34-positive Zellen über 7 Tage in einer 96-Loch-Mikrotitterplatte bei 37°C und 5% Kohlendioxid inkubiert und anschließend die Proliferationsraten anhand der Zellzahlen bestimmt.

Zu diesem Zweck werden 5000 CD34-positive Zellen pro Loch einer 96-U-Boden-Loch-Mikrotitterplatte (Greiner Bio-One, Katalognummer 650 180) in 100 Mikroliter IMDM-Medium (Life Technology, Katalognummer 12440-046), 10% fetales Kälberserum (Life Technology, Katalognummer 10082-139) und 20 Nanogramm pro Milliliter Stem Cell Factor (R&D, Katalognummer 255-SC-010) aufgenommen. Zusätzlich werden die Zellen noch unterschiedlichen Konzentrationen an mit Dimethylsulfoxid (Sigma Aldrich, Katalognummer D5879-1L) gelösten Substanzen versetzt. Dabei werden jeweils 4 Löcher mit der angegebenen Zellzahl von 5000 CD34-positiven Zellen pro Loch mit 10 Mikromol, 4 Löcher mit 5 Mikromol, 4 Löcher mit 2.5 Mikromol, 4 Löcher mit 1.25 Mikromol, 4 Löcher mit 0.625 Mikromol, 4 Löcher mit 0.3125 Mikromol, 4 Löcher mit 0.156 Mikromol, 4 Löcher mit 0.078 Mikromol und als Kontrolle 4 Löcher mit 0.1% Dimethylsulfoxid als Endkonzentration versehen.

Diese so behandelten Zellen werden für 7 Tage in einem Zellkultur-Brutschrank bei 37°C und 5% Kohlendioxid inkubiert. Durch erneutes zählen der Zellen unter Verwendung einer Neubauer-Zählkammer wird die Proliferationsrate bestimmt, wobei die nur mit dem Stem Cell Factor versehenen Zellen als 100%-Wert gesetzt und alle anderen Werte auf diesen Wert bezogen sind.

### In-vivo Assay

Die Untersuchungen der in vivo-Wirkung der erfindungsmäßigen Verbindungen erfolgt unter Verwendung von 6 Wochen alten, 18 - 22 g schweren, männlichen C57BL/6-Mäusen (Charles River, Sulzfeld, Deutschland). Diese Tiere werden artgerecht mit 12-stündigen Licht- und Dunkelzyklen unter konstanten klimatischen Bedingungen gehalten und mit Wasser und Mausfutter *ad libitum* ernährt. Die Konzentrationen an verwendeten Chemotherapeutika werden den Tieren gemäß den Angaben der Hersteller mittels intra-peritonealen (i.p.) Injektionen im kaudalen Drittel des Bauches verabreicht. Gleichermaßen wird mit den erfindungsrelevanten Substanzen verfahren. Blutabnahmen erfolgen mit Hilfe von Pasteur-Pipetten aus dem retrobulbären Venenplexus. Die Bestimmung der Anzahl neutrophiler Granulozyten erfolgt vollautomatisch unter Verwendung von Durchflußzytometriesystemen.

### CYP-Inhibitionstest

Die Fähigkeit von Substanzen, CYP1A2, CYP 2C8, CYP2C9, CYP2D6 und CYP3A4 im Menschen inhibieren zu können, wird untersucht mit gepoolten Human-Lebermikrosomen als Enzymquelle in Gegenwart von Standardsubstraten (s.u.), die CYP-Isoform-spezifische Metaboliten bilden. Die Inhibitionseffekte werden bei sechs verschiedenen Konzentrationen der Testverbindungen untersucht (1.5, 3.1, 6.3, 12.5, 25 sowie 50 µM), mit dem Ausmaß der CYP-Isoform-spezifischen Metabolitenbildung der Standardsubstrate in Abwesenheit der Testverbindungen verglichen und die entsprechenden IC₅₀-Werte berechnet. Ein Standard-Inhibitor, der eine einzelne CYP-Isoform spezifisch inhibiert, dient als Kontrolle der erhaltenen Ergebnisse.

### Durchführung:

Die Inkubation von Phenacetin, Amodiaquin, Diclofenac, Dextromethorphan oder Midazolam mit Human-Lebermikrosomen in Gegenwart von jeweils sechs verschiedenen Konzentrationen einer Testverbindung (als potentiellem Inhibitor) wird auf einer Workstation durchgeführt (Tecan, Genesis, Crailsheim, Deutschland). Standard-Inkubationsgemische enthalten 1.3 mM NADP, 3.3 mM MgCl₂ x 6 H₂O, 3.3 mM Glukose-6-phosphat, Glukose-6-phosphat-Dehydrogenase (0.4 U/ml) und 100 mM Phosphat-Puffer (pH 7.4) in einem Gesamtvolumen von 200 µl. Testverbindungen werden bevorzugt in Acetonitril gelöst. 96-Lochplatten werden eine definierte Zeit bei 37°C mit gepoolten Human-Lebermikrosomen inkubiert. Die Reaktionen werden durch Zugabe von 100 µl Acetonitril, worin sich ein geeigneter interner Standard befindet, abgestoppt. Gefällte Proteine werden durch Zentrifugation abgetrennt, die Überstände werden vereinigt und mittels LC-MS/MS analysiert.

### Bestimmung der Löslichkeit

### Benötigte Reagenzien:

● PBS-Puffer pH 6.5: 61.86 g Natriumchlorid p.a. (z.B. Fa. Merck, Art.-Nr. 1.06404.1000), 39.54 g Natriumdihydrogenphosphat p.a. (z.B. Fa. Merck, Art.-Nr. 1.06346.1000) und 83.35 g 1 N Natriumhydroxid-Lösung (z.B. Fa. Bernd Kraft GmbH, Art.-Nr. 01030.4000) in einen 1 Liter-Messkolben einwiegen, mit Wasser auffüllen und ca. 1 Stunde rühren. Von dieser Lösung 500 ml in einen 5 Liter-Messkolben geben und mit Wasser auffüllen. Mit 1 N Natriumhydroxid-Lösung auf pH 6.5 einstellen.
● Dimethylsulfoxid (z.B. Fa. Baker, Art.-Nr. 7157.2500)
● destilliertes Wasser
● Acetonitril Chromasolv (z.B. Riedel-de Haen Art. Nr. 34851)
● 50%ige Ameisensäure p.a. (z.B. Fluka Art. Nr. 09676)

### Herstellung der Ausgangslösung:

Mindestens 1.5 mg der Testsubstanz werden in ein Wide Mouth 10mm Screw V-Vial (Fa. Glastechnik Gräfenroda GmbH, Art.-Nr. 8004-WM-H/V15µ) mit passender Schraubkappe und Septum genau eingewogen, mit Dimethylsulfoxid zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) (z.B. HJ-Bioanalytik GmbH Art.-Nr. 850289) mittels eines Liquid-Handling-Roboters. Als Lösemittel wird ein Gemisch aus Acetonitril Chromasolv / destilliertem Wasser 8:2 verwendet.

*Herstellung der Ausgangslösung für Kalibrierlösungen (Stammlösung):* 10 µl der Ausgangslösung werden mit 833 µl des Lösemittelgemisch versetzt (Konzentration = 600 µg/ml) und homogenisiert. Es werden von jeder Testsubstanz zwei 1:100 Verdünnungen in separaten DWP's hergestellt und wiederum homogenisiert. Eine der 1:100 Verdünnungen wird für die Herstellung der Kalibrierlösungen verwendet, die zweite Verdünnung wird für die Optimierung der MS/MS-Parameter verwendet.

*Kalibrierlösung 5 (600 ng*/*ml):* 30 µl der Stammlösung werden mit 270 µl Lösemittelgemisch versetzt und homogenisiert.

*Kalibrierlösung 4 (60 nglml):* 30 µl der Kalibrierlösung 5 werden mit 270 µl Lösemittelgemisch versetzt und homogenisiert.

*Kalibrierlösung 3 (12 ng*/*ml):* 100 µl der Kalibrierlösung 4 werden mit 400 µl Lösemittelgemisch versetzt und homogenisiert.

*Kalibrierlösung 2 (1.2 ng*/*ml):* 30 µl der Kalibrierlösung 3 werden mit 270 µl Lösemittelgemisch versetzt und homogenisiert.

*Kalibrierlösung 1 (0.6 ng*/*ml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP (z.B. HJ-Bioanalytik GmbH Art.-Nr. 850289) mittels eines Liquid-Handling-Roboters.

10.1 µl der Stammlösung werden mit 1000 µl PBS-Puffer pH 6.5 versetzt.

### Durchführung:

Die notwendigen Pipettierschritte erfolgen in 1.2 ml 96er DWP (z.B. HJ-Bioanalytik GmbH Art.-Nr. 850289) mittels eines Liquid-Handling-Roboters.

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und 1:10 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 7) Probenlösung 1:10.

### HPLC/MS-MS Methode

HPLC: Agilent 1100 , quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/l; Eluent B: Acetonitril + 0.5 ml Ameisensäure/1; Flussrate: 2.5 ml/min; Stoptime 1.5 min; Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS:WATERS Quattro Micro Tandem MS/MS; Z-Spray API-Interface; HPLC-MS-Eingangssplitter 1:20; Messung im ESI-Mode

Die Geräteparameter werden für jede Testsubstanz durch Injektion der weiter oben beschriebenen Stammlösung (zweite 1:100 Verdünnung) mittels der MassLynx/QuanOptimize-Software automatisch optimiert.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 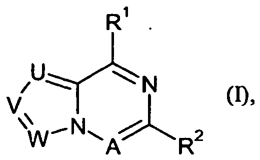 in welcher
entweder
U für N steht,
V für CR¹² steht,
W für CH steht,
A für CR¹⁵ steht,
oder
U für CH steht,
V für CR¹² steht,
W für N steht,
A für CR¹⁵ steht,
oder
U für CR¹⁶ steht,
V für N steht,
W für CR¹⁷ steht,
A für N steht,
wobei
R¹² für Wasserstoff, Hydroxy, Amino, Hydroxycarbonyl, Aminocarbonyl, Trifluormethyl, Trifluormethoxy, Cyano, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonylamino, 5- oder 6-gliedriges Heterocyclylcarbonyl, -CH₂R¹³ oder -CH₂CH₂R¹⁴ steht,
wobei Heterocyclylcarbonyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkoxy, Alkylamino, Alkylcarbonyl, Alkoxycarbonyl, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, 5- oder 6-gliedriges Heterocyclyl und Phenyl,
worin Phenyl substituiert sein kann mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei
R¹³ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, C₃-C₆-Cycloalkylamino oder 5- oder 6-gliedriges Heterocyclyl steht,
worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 3 Substituenten, wobei die Substituenten unabhängig voneinander ausgewählt werden aus der Gruppe bestehend aus Halogen, Oxö, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei
R¹⁴ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino oder 5- oder 6-gliedriges Heterocyclyl steht,
worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
R¹⁵ für Wasserstoff, Halogen, Cyano, Trifluormethyl, C₁-C₃-Alkyl, Methoxy, Methylthio oder Cyclopropyl steht,
R¹⁶ für Wasserstoff oder Methyl steht,
R¹⁷ für Wasserstoff oder Methyl steht,
R¹ für eine Gruppe der Formel 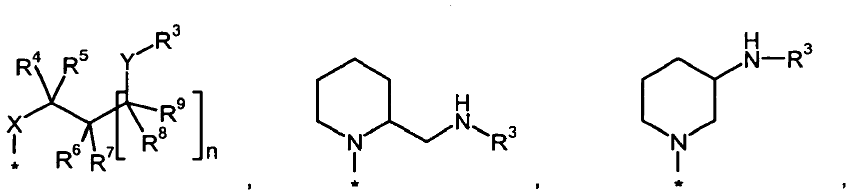 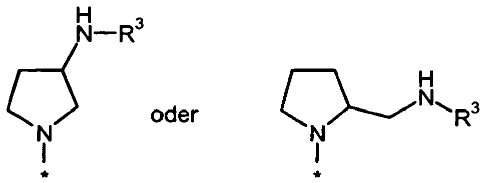 steht,
wobei
* die Anknüpfstelle an den Heterocyclus bedeutet,
n für die Zahl 0 oder 1 steht,
X für NR¹⁰, S oder O steht,
worin
R¹⁰ für Wasserstoff, C₁-C₃-Alkyl oder Cyclopropyl steht,
Y für Nur¹¹ oder S steht,
worin
R¹¹ für Wasserstoff, C₁-C₃-Alkyl oder Cyclopropyl steht,
R³ für 2-Pyridyl, Pyrimid-2-yl, 2-Aminopyrimid-4-yl, 2-(Mono-C₁-C₄-Alkylamino)pyrimid-4-yl, 2-(Mono-C₃-C₄-Cycloalkylamino)pyrimid-4-yl, Pyridazin-3(2H)-on-6-yl, 1,3-Oxazol-2-yl, 1,3-Oxazol-4-yl, 1,2,4-Oxadiazol-3-yl, 1,2,3-Oxadiazol-4-yl, 1,3-Thiazol-2-yl, 1,3-Thiazol-4-yl, 1H-1,2,4-Triazol-5-yl, 2,4-Dihydro-3H-1,2,4- triazol-3-on-5-yl oder 1,2-Pyrazol-5-yl steht,
wobei 2-Pyridyl, Pyrimid-2-yl, 1,3-Oxazol-2-yl, 1,3-Oxazol-4-yl, 1,3-Thiazol-2-yl und 1,3-Thiazol-4-yl substituiert sind mit 1 oder 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₃-C₄-Cycloalkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
und
wobei 2-Aminopyrimid-4-yl, 2-(Mono-C₁-C₄-Alkylamino)pyrimid-4-yl, 2-(Mono-C₃-C₄-Cycloalkylamino)pyrimid-4-yl, Pyridazin-3(2H)-on-6-yl, 1,2,4-Oxadiazol-3-yl, 1,2,3-Oxadiazol-4-yl, 1H-1,2,4-Triazol-5-yl, 2,4-Dihydro-3H-1,2,4-triazol-3-on-5-yl und 1,2-Pyrazol-5-yl substituiert sein können mit einem Substituenten, wobei der Substituent ausgewählt wird aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₃-C₄-Cycloalkylamino, C₁-C₄-Alkoxycarbonyl, C₁-C₄-Alkylaminocarbonyl und C₃-C₆-Cycloalkylcarbonyl,
R⁴ für Wasserstoff, C₁-C₃-Alkyl oder Cyclopropyl steht,
R⁵ für Wasserstoff oder C₁-C₃-Alkyl steht,
R⁶ für Wasserstoff, C₁-C₃-Alkyl oder Cyclopropyl steht,
R⁷ für Wasserstoff oder C₁-C₃-Alkyl steht,
R⁸ für Wasserstoff, C₁-C₃-Alkyl oder Cyclopropyl steht,
R⁹ für Wasserstoff oder C₁-C₃-Alkyl steht,
R² für C₆-C₁₀-Aryl oder 5- bis 10-gliedriges Heteroaryl steht,
wobei Aryl und Heteroaryl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5-oder 6-gliedrigem Heterocyclylcarbonyl, 5- oder 6-gliedrigem Heterocyclylmethyl und 5- oder 6-gliedrigem Heteroaryl,
worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino,
oder
zwei der Substituenten am Aryl zusammen mit den Kohlenstoffatomen, an die sie gebunden sind, ein 1,3-Dioxolan oder 1,4-Dioxan bilden,
oder eines ihrer Salze, ihrer Solvate oder der Solvate ihrer Salze.

2. Verbindung nach Anspruch 1, **dadurch gekennzeichnet, dass**
entweder
U für N steht,
V für CR¹² steht,
W für CH steht,
A für CR¹⁵ steht,
oder
U für CH steht,
V für CR¹² steht,
W für N steht,
A für CR¹⁵ steht,
oder
U für CR¹⁶ steht,
V für N steht,
W für CR¹⁷ steht,
A für N steht, wobei
R¹² für Wasserstoff, Hydroxycarbonyl, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl, C₁-C₄-Alkylcarbonylamino, 5- oder 6-gliedriges Heterocyclylcarbonyl, -CH₂R¹³ oder -CH₂CH₂R¹⁴ steht,
wobei Heterocyclylcarbonyl substituiert sein kann mit 1 bis 3 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkoxy, Alkylamino, Alkylcarbonyl, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
worin Heterocyclyl substituiert sein kann mit 1 bis 2 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei
R¹³ für Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, C₃-C₆-Cycloalkylamino oder 5- oder 6-gliedriges Heterocyclyl steht,
worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 2 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei
R¹⁴ für Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino oder 5- oder 6-gliedriges Heterocyclyl steht,
worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino,
und
worin Heterocyclyl substituiert sein kann mit 1 bis 2 Substituenten, wobei die Substituenten unabhängig voneinander ausgewählt werden aus der Gruppe bestehend aus Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
R¹⁵ für Wasserstoff, Halogen, Cyano oder Trifluormethyl steht,
R¹⁶ für Wasserstoff oder Methyl steht,
R¹⁷ für Wasserstoff oder Methyl steht,
R¹ für eine Gruppe der Formel 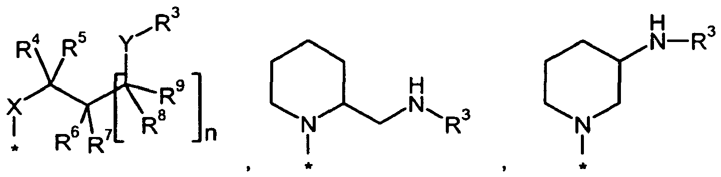 oder 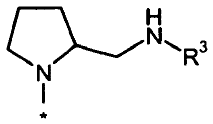 steht,
wobei
* die Anknüpfstelle an den Heterocyclus bedeutet,
n für die Zahl 0 oder 1 steht,
X für NR¹⁰, S oder O steht,
worin
R¹⁰ für Wasserstoff oder Methyl steht,
Y für NR¹¹ oder S steht,
worin
R¹¹ für Wasserstoff oder Methyl steht,
R³ für 2-Pyridyl, Pyrimid-2-yl, 2-Aminopyrimid-4-yl, 1,3-Oxazol-2-yl, 1,3-Oxazol-4-yl, 1,2,4-Oxadiazol-3-yl, 1,2,3-Oxadiazol-4-yl, 1,3-Thiazol-2-yl oder 1,3-Thiazol-4-yl steht,
wobei 2-Pyridyl, Pyrimid-2-yl, 1,3-Oxazol-2-yl, 1,3-Oxazol-4-yl, 1,3-Thiazol-2-yl und 1,3-Thiazol-4-yl substituiert sind mit 1 oder 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl Methyl, Ethyl, Methoxy, Ethoxy, C₁-C₄-Alkylamino, Methylcarbonyl, Ethylcarbonyl, Cyclopropylcarbonyl, Methoxycarbonyl und Ethoxycarbonyl,
und
wobei 2-Aminopyrimid-4-yl, 1,2,4-Oxadiazol-3-yl und 1,2,3-Oxadiazol-4-yl substituiert sein können mit einem Substituenten, wobei der Substituent ausgewählt wird aus der Gruppe bestehend aus Halogen, Cyano, Nitro, Amino, Trifluormethyl, Trifluormethoxy, Aminocarbonyl, Trifluormethylcarbonyl, Methyl, Ethyl, Methoxy, Ethoxy, C₁-C₄-Alkylamino, Methylcarbonyl, Ethylcarbonyl, Cyclopropylcarbonyl, Methoxycarbonyl und Ethoxycarbonyl,
R⁴ für Wasserstoff oder Methyl steht,
R⁵ für Wasserstoff oder Methyl steht,
R⁶ für Wasserstoff oder Methyl steht,
R⁷ für Wasserstoff oder Methyl steht,
R⁸ für Wasserstoff oder Methyl steht,
R⁹ für Wasserstoff oder Methyl steht,
R² für C₆-C₁₀-Aryl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Chinolinyl, Benzfuranyl oder Benzoxazolyl steht,
wobei Aryl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Chinolinyl, Benzfuranyl und Benzoxazolyl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonytamino, C₁-C₄-Alkylaminosulfonyl, Phenyl, Benzyloxy, 5- oder 6-gliedrigem Heterocyclyl, 5-oder 6-gliedrigem Heterocyclylcarbonyl, 5- oder 6-gliedrigem Heterocyclylmethyl und 5- oder 6-gliedrigem Heteroaryl,
worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Allcoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino,
oder eines ihrer Salze, ihrer Solvate oder der Solvate ihrer Salze.

3. Verbindung nach einem der Ansprüche 1 oder 2, **dadurch gekennzeichnet, dass** entweder
U für N steht,
V für CR¹² steht,
W für CH steht,
A für CR¹⁵ steht,
oder
U für CH steht,
V für CR¹² steht,
W für N steht,
A für CR¹⁵ steht,
oder
U für CR¹⁶ steht,
V für N steht,
W für CR¹⁷ steht,
A für N steht,
wobei
R¹² für Wasserstoff, Hydroxycarbonyl, Aminocarbonyl, Methyl, Ethyl, C₁-C₄-Alkylcarbonyl, C₁-C₄-Alkoxycarbonyl, C₁-C₄-Alkylaminocarbonyl, C₁-C₄-Alkylcarbonylamino, Pyrrolidinylcarbonyl, Piperidinylcarbonyl, Piperazinylcarbonyl, Mopholinylcarbonyl oder -CH₂R¹³ steht,
wobei Pyrrolidinylcarbonyl, Piperidinylcarbonyl, Piperazinylcarbonyl und Mopholinylcarbonyl substituiert sein können mit 1 bis 2 Substituenten, wobei die Substituenten unabhängig voneinander ausgewählt werden aus der Gruppe bestehend aus Oxo, Methyl und Ethyl,
und
wobei Alkylcarbonyl, C₂-C₄-Alkoxycarbonyl und C₂-C₄-Alkylaminocarbonyl substituiert sein können mit einem Substituenten, wobei der Substituent ausgewählt wird aus der Gruppe bestehend aus Hydroxy, Amino, C₁-C₄-Alkylamino, Pyrrolidinyl, Piperidinyl, Piperazinyl und Morphlinyl,
worin Pyrrolidinyl, Piperidinyl, Piperazinyl und Morphlinyl substituiert sein können mit 1 bis 2 Substituenten, wobei die Substituenten unabhängig voneinander ausgewählt werden aus der Gruppe bestehend aus Oxo, Methyl und Ethyl,
und
wobei
R¹³ für Hydroxy, Amino, Hydroxycarbonyl, Aminocarbonyl, C₁-C₄-Alkoxy, C₁-C₄-Alkylamino, Pyrrolidinyl, Piperidinyl, Piperazinyl oder Morphlinyl steht,
worin Pyrrolidinyl, Piperidinyl, Piperazinyl und Morphlinyl substituiert sein können mit 1 bis 2 Substituenten, wobei die Substituenten unabhängig voneinander ausgewählt werden aus der Gruppe bestehend aus Oxo, Methyl und Ethyl,
R¹⁵ für Wasserstoff steht,
R¹⁶ für Wasserstoff oder Methyl steht,
R¹⁷ für Wasserstoff oder Methyl steht,
R¹ für eine Gruppe der Formel 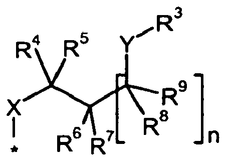 steht,
wobei
* die Anknüpfstelle an den Heterocyclus bedeutet,
n für die Zahl 0 steht,
X für NR¹⁰ steht,
worin
R¹⁰ für Wasserstoff steht,
Y für NR¹¹ steht,
worin
R¹¹ für Wasserstoff oder Methyl steht,
R³ für 2-Pyridyl, Pyrimid-2-yl, 2-Aminopyrimid-4-yl, 1,3-Thiazol-2-yl oder 1,3-Thiazol-4-yl steht,
wobei 2-Pyridyl, Pyrimid-2-yl, 1,3-Thiazol-2-yl und 1,3-Thiazol-4-yl substituiert sind mit 1 oder 2 Substituenten, wobei die Substituenten unabhängig voneinander ausgewählt werden aus der Gruppe bestehend aus Fluor, Chlor, Cyano, Nitro, Amino und Trifluormethyl,
und
wobei 2-Aminopyrimid-4-yl substituiert sein kann mit einem Substituenten, wobei der Substituent ausgewählt wird aus der Gruppe bestehend aus Fluor, Chlor, Cyano, Nitro, Amino und Trifluormethyl,
R⁴ für Wasserstoff steht,
R⁵ für Wasserstoff oder Methyl steht,
R⁶ für Wasserstoff steht,
R⁷ für Wasserstoff oder Methyl steht,
R8 für Wasserstoff steht,
R⁹ für Wasserstoff oder Methyl steht,
R² für Phenyl, Thienyl, Pyrazolyl oder Pyridyl steht,
wobei Phenyl, Thienyl, Pyrazolyl und Pyridyl substituiert sein können mit 1 bis 2 Substituenten, wobei die Substituenten unabhängig voneinander ausgewählt werden aus der Gruppe bestehend aus Halogen, Trifluormethyl, Trifluormethoxy, Aminocarbonyl, C₁-C₄-Alkyl, C₁-C₄-Alkoxy, C₁-C₄-Alkoxycarbonyl, C₁-C₄-Alkylaminocarbonyl, Pyrrolidinyl, Piperidinyl, Morpholinyl und Morpholinylcarbonyl,
oder eines ihrer Salze, ihrer Solvate oder der Solvate ihrer Salze.

4. Verbindung nach einem der Ansprüche 1, 2 oder 3, **dadurch gekennzeichnet, dass** entweder
U für N steht,
V für CR¹² steht,
W für CH steht,
A für CR¹⁵ steht,
oder
U für CH steht,
V für CR¹² steht,
W für N steht,
A für CR¹⁵ steht,
oder
U für CR¹⁶ steht,
V für N steht,
W für CR¹⁷ steht,
A für N steht,
wobei
R¹² für Wasserstoff, Hydroxycarbonyl, Methyl, Ethyl, Methoxycarbonyl, Ethoxycarbonyl, C₁-C₄-Alkylaminocarbonyl, Piperidinylcarbonyl oder Mopholinylcarbonyl steht,
wobei Piperidinylcarbonyl und Mopholinylcarbonyl substituiert sein können mit einem Substituenten, wobei der Substituent ausgewählt wird aus der Gruppe bestehend aus Methyl und Ethyl,
und
wobei C₂-C₄-Alkylaminocarbonyl substituiert sein kann mit einem Substituenten, wobei der Substituent ausgewählt wird aus der Gruppe bestehend aus C₁-C₄-Alkylamino, Piperazinyl und Morphlinyl,
worin Piperazinyl und Morphlinyl substituiert sein können mit einem Substituenten, wobei der Substituent ausgewählt wird aus der Gruppe bestehend aus Methyl und Ethyl,
R¹⁵ für Wasserstoff steht,
R¹⁶ für Methyl steht,
R¹⁷ für Methyl steht,
R¹ für eine Gruppe der Formel 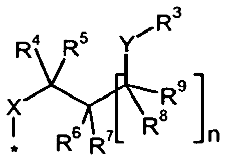 steht,
wobei
* die Anknüpfstelle an den Heterocyclus bedeutet,
n für die Zahl 0 steht,
X für NR¹⁰ steht,
worin
R¹⁰ für Wasserstoff steht,
Y für NR¹¹ steht,
worin
R¹¹ für Wasserstoff oder Methyl steht,
R³ für eine Gruppe der Formel 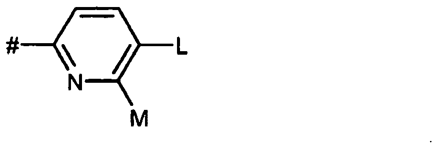 steht,
wobei
# die Anknüpfstelle an Y bedeutet,
L für Cyano, Nitro oder Trifluormethyl steht,
M für Wasserstoff oder Amino steht,
R⁴ für Wasserstoff steht,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R² für Phenyl steht,
wobei Phenyl substituiert sein kann mit 1 bis 2 Substituenten, wobei die Substituenten unabhängig voneinander ausgewählt werden aus der Gruppe bestehend aus Fluor, Chlor, Trifluormethyl, Trifluormethoxy, C₁-C₃-Alkyl, Methoxy, Methoxycarbonyl und Ethoxycarbonyl,
oder eines ihrer Salze, ihrer Solvate oder der Solvate ihrer Salze.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass**
[A] eine Verbindung der Formel 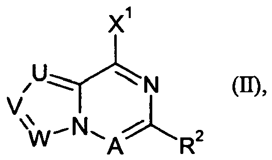 in welcher
A, U, V, W und R² die in Anspruch 1 angegebene Bedeutung haben, und
X¹ für Halogen, bevorzugt Chlor oder Fluor, steht,
mit einer Verbindung der Formel
R¹-H (III),
in welcher
R¹ die in Anspruch 1 angegebene Bedeutung hat,
umgesetzt wird,
oder
[B] eine Verbindung der Formel 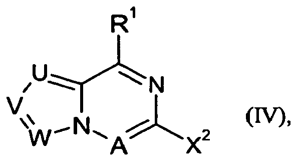 in welcher
R¹ die in Anspruch 1 angegebene Bedeutung hat,
und
A für CR¹⁵ steht,
wobei R¹⁵ die in Anspruch 1 angegebene Bedeutung hat,
U für N steht,
V für CR¹² steht,
wobei R¹² die in Anspruch 1 angegebene Bedeutung hat,
W für CH steht,
X² für Iod, Brom, Chlor oder Trifluormethansulfonyl, bevorzugt Iod oder Brom, steht,
mit einer Verbindung der Formel
Q-R² (V),
in welcher
R² die in Anspruch 1 angegebene Bedeutung hat, und
Q für -B(OH)₂, einen Boronsäure-Ester, bevorzugt Boronsäurepinakolester, oder -BF₃⁻K⁺ steht,
unter Suzuki-Kupplungsbedingungen zu einer Verbindung der Formel 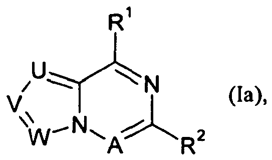 in welcher
R¹ und R² die in Anspruch 1 angegebene Bedeutung haben, und
A für CR¹⁵ steht,
wobei R¹⁵ die in Anspruch 1 angegebene Bedeutung hat,
U für N steht,
V für CR¹² steht,
wobei R¹² die in Anspruch 1 angegebene Bedeutung hat,
W für CH steht,
umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung nach Anspruch 7, wobei es sich bei den Krankeiten um hämatologische Erkrankungen handelt.

9. Arzneimittel enthaltend eine Verbindung nach einem der Ansprüche 1 bis 4 in Kombination mit einem inerten, nichttoxischen, pharmazeutisch geeigneten Hilfsstoff.

10. Arzneimittel nach Anspruch 9 zur Behandlung und/oder Prophylaxe von hämatologischen Erkrankungen.

11. Verwendung einer Verbindung nach einem der Ansprüche 1 bis 4, zur effizienten *ex vivo* Vermehrung von adulten hämatopoetischen Stammzellen aus dem Knochenmark, aus peripherem Blut oder Nabelschnurblut.

12. Verfahren zur *ex vivo* Vermehrung von adulten hämatopoetischen Stammzellen aus dem Knochenmark, aus peripherem Blut oder Nabelschnurblut, **dadurch gekennzeichnet, dass** eine wirksame Menge einer Verbindung nach einem der Ansprüche 1 bis 4 zugegeben wird.

## Claims

1. Compound of the formula 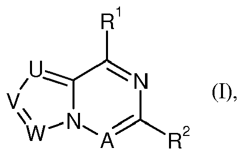 in which
either
U represents N,
V represents CR¹²,
W represents CH,
A represents CR¹⁵,
or
U represents CH,
V represents CR¹²,
W represents N,
A represents CR¹⁵,
or
U represents CR¹⁶,
V represents N,
W represents CR¹⁷,
A represents N,
where
R¹² represents hydrogen, hydroxyl, amino, hydroxycarbonyl, aminocarbonyl, trifluoromethyl, trifluoromethoxy, cyano, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl, C₁-C₄-alkylcarbonylamino, C₁-C₄-alkylsulfonylamino, 5- or 6-membered heterocyclylcarbonyl, -CH₂R¹³ or -CH₂CH₂R¹⁴,
where heterocyclylcarbonyl may be substituted by 1 to 3 substituents, where the substituents independently of one another are selected from the group consisting of halogen, oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where alkoxy, alkylamino, alkylcarbonyl, alkoxycarbonyl, alkylaminocarbonyl, alkylcarbonylamino and alkylsulfonylamino may be substituted by a substituent, where the substituent is selected from the group consisting of hydroxyl, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino, 5- or 6-membered heterocyclyl and phenyl,
where phenyl may be substituted by 1 to 3 substituents, where the substituents independently of one another are selected from the group consisting of halogen, cyano, trifluoromethyl, trifluoromethoxy, aminocarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₁-C₄-alkylcarbonylamino,
and
where heterocyclyl may be substituted by 1 to 3 substituents, where the substituents independently of one another are selected from the group consisting of halogen, oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where
R¹³ represents hydroxyl, amino, cyano, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino, C₃-C₆-cycloalkylamino or 5- or 6-membered heterocyclyl,
where alkoxy, alkylamino, alkoxycarbonyl, alkylaminocarbonyl and alkylcarbonylamino may be substituted by a substituent, where the substituent is selected from the group consisting of hydroxyl, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino,
and
where heterocyclyl may be substituted by 1 to 3 substituents, where the substituents independently of one another are selected from the group consisting of halogen, oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where
R¹⁴ represents hydroxyl, amino, cyano, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino or 5- or 6-membered heterocyclyl,
where alkoxy, alkylamino, alkoxycarbonyl, alkylaminocarbonyl and alkylcarbonylamino may be substituted by a substituent, where the substituent is selected from the group consisting of hydroxyl, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino,
and
where heterocyclyl may be substituted by 1 to 3 substituents, where the substituents independently of one another are selected from the group consisting of halogen, oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
R¹⁵ represents hydrogen, halogen, cyano, trifluoromethyl, C₁-C₃-alkyl, methoxy, methylthio or cyclopropyl,
R¹⁶ represents hydrogen or methyl,
R¹⁷ represents hydrogen or methyl,
R¹ represents a group of the formula 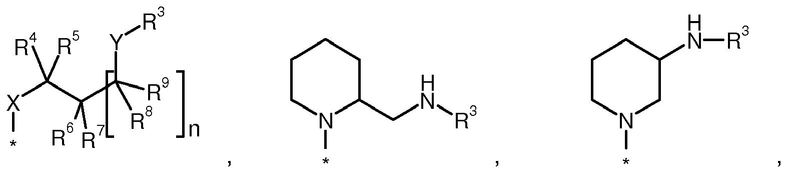 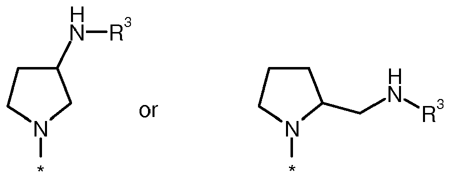 where
* is the point of attachment to the heterocycle,
n represents the number 0 or 1,
X represents NR¹⁰, S or O, where
R¹⁰ represents hydrogen, C₁-C₃-alkyl or cyclopropyl,
Y represents NR¹¹ or S,
where
R¹¹ represents hydrogen, C₁-C₃-alkyl or cyclopropyl,
R³ represents 2-pyridyl, pyrimid-2-yl, 2-aminopyrimid-4-yl, 2-(mono-C₁-C₄-alkylamino)pyrimid-4-yl, 2-(mono-C₃-C₄-cycloalkylamino)pyrimid-4-yl, pyridazin-3(2H)-on-6-yl, 1,3-oxazol-2-yl, 1,3-oxazol-4-yl, 1,2,4-oxadiazol-3-yl, 1,2,3-oxadiazol-4-yl, 1,3-thiazol-2-yl, 1,3-thiazol-4-yl, 1H-1,2,4-triazol-5-yl, 2,4-dihydro-3H-1,2,4-triazol-3-on-5-yl or 1,2-pyrazol-5-yl, where 2-pyridyl, pyrimid-2-yl, 1,3-oxazol-2-yl, 1,3-oxazol-4-yl, 1,3-thiazol-2-yl and 1,3-thiazol-4-yl are substituted by 1 or 2 substituents, where the substituents independently of one another are selected from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₃-C₄-cycloalkylamino, C₁-C₄-alkylcarbonyl, C₁-C₄-alkoxycarbonyl, C₁-C₄-alkylaminocarbonyl and C₃-C₆-cycloalkylcarbonyl,
where alkyl, alkoxy, alkylamino, alkylcarbonyl, alkoxycarbonyl, alkylaminocarbonyl and cycloalkylcarbonyl may be substituted by a substituent, where the substituent is selected from the group consisting of halogen, cyano, hydroxyl, amino, trifluoromethyl and C₃-C₆-cycloalkyl,
and
where 2-aminopyrimid-4-yl, 2-(mono-C₁-C₄-alkylamino)pyrimid-4-yl, 2-(mono-C₃-C₄-cycloalkylamino)pyrimid-4-yl, pyridazin-3(2H)-on-6-yl, 1,2,4-oxadiazol-3-yl, 1,2,3-oxadiazol-4-yl, 1H-1,2,4-triazol-5-yl, 2,4-dihydro-3H-1,2,4-triazol-3-on-5-yl and 1,2-pyrazol-5-yl may be substituted by a substituent, where the substituent is selected from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₃-C₄-cycloalkylamino, C₁-C₄-alkoxycarbonyl, C₁-C₄-alkylaminocarbonyl and C₃-C₆-cycloalkylcarbonyl,
R⁴ represents hydrogen, C₁-C₃-alkyl or cyclopropyl,
R⁵ represents hydrogen or C₁-C₃-alkyl,
R⁶ represents hydrogen, C₁-C₃-alkyl or cyclopropyl,
R⁷ represents hydrogen or C₁-C₃-alkyl,
R⁸ represents hydrogen, C₁-C₃-alkyl or cyclopropyl,
R⁹ represents hydrogen or C₁-C₃-alkyl,
R² represents C₆-C₁₀-aryl or 5- to 10-membered heteroaryl,
where aryl and heteroaryl may be substituted by 1 to 3 substituents, where the substituents independently of one another are selected from the group consisting of hydroxyl, hydroxymethyl, amino, halogen, cyano, trifluoromethyl, trifluoromethoxy, aminocarbonyl, C₁-C₄-alkyl, C₁-C₄-alkoxy, C₁-C₄-alkoxymethyl, C₁-C₄-alkylamino, C₁-C₄-alkylaminomethyl, C₁-C₄-alkylcarbonyl, C₁-C₄-alkoxycarbonyl, C₁-C₄-alkylaminocarbonyl, C₁-C₄-alkylcarbonylamino, C₁-C₄-alkylsulfonyl, C₁-C₄-alkylsulfonylamino, C₁-C₄-alkylaminosulfonyl, phenyl, benzyloxy, 5- or 6-membered heterocyclyl, 5- or 6-membered heterocyclylcarbonyl, 5- or 6-membered heterocyclylmethyl and 5- or 6-membered heteroaryl,
where phenyl, benzyloxy, heterocyclyl, heterocyclylcarbonyl, heterocyclylmethyl and heteroaryl may be substituted by 1 to 3 substituents, where the substituents independently of one another are selected from the group consisting of halogen, cyano, trifluoromethyl, trifluoromethoxy, aminocarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₁-C₄-alkylcarbonylamino,
or
two of the substituents on aryl together with the carbon atoms to which they are attached form a 1,3-dioxolane or 1,4-dioxane,
or one of its salts, its solvates or the solvates of its salts.

2. Compound according to Claim 1, **characterized in that**
either
U represents N,
V represents CR¹²,
W represents CH,
A represents CR¹⁵,
or
U represents CH,
V represents CR¹²,
W represents N,
A represents CR¹⁵,
or
U represents CR¹⁶,
V represents N,
W represents CR¹⁷,
A represents N,
where
R¹² represents hydrogen, hydroxycarbonyl, aminocarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl, C₁-C₄-alkylcarbonylamino, 5- or 6-membered heterocyclylcarbonyl, -CH₂R¹³ or -CH₂CH₂R¹⁴,
where heterocyclylcarbonyl may be substituted by 1 to 3 substituents, where the substituents independently of one another are selected from the group consisting of oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where alkoxy, alkylamino, alkylcarbonyl, alkoxycarbonyl, alkylaminocarbonyl and alkylcarbonylamino may be substituted by a substituent, where the substituent is selected from the group consisting of hydroxyl, amino, hydroxycarbonyl, aminocarbonyl, C₁-C₄-alkoxy, C₁-C₄-alkylamino and 5- or 6-membered heterocyclyl,
where heterocyclyl may be substituted by 1 to 2 substituents, where the substituents independently of one another are selected from the group consisting of oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where
R¹³ represents hydroxyl, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino, C₃-C₆-cycloalkylamino or 5- or 6-membered heterocyclyl,
where alkoxy, alkylamino, alkoxycarbonyl, alkylaminocarbonyl and alkylcarbonylamino may be substituted by a substituent, where the substituent is selected from the group consisting of hydroxyl, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino,
and
where heterocyclyl may be substituted by 1 to 2 substituents, where the substituents independently of one another are selected from the group consisting of oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where
R¹⁴ represents hydroxyl, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino or 5- or 6-membered heterocyclyl,
where alkoxy, alkylamino, alkoxycarbonyl, alkylaminocarbonyl and alkylcarbonylamino may be substituted by a substituent, where the substituent is selected from the group consisting of hydroxyl, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino,
and
where heterocyclyl may be substituted by 1 to 2 substituents, where the substituents independently of one another are selected from the group consisting of oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
R¹⁵ represents hydrogen, halogen, cyano or trifluoromethyl,
R¹⁶ represents hydrogen or methyl,
R¹⁷ represents hydrogen or methyl,
R¹ represents a group of the formula 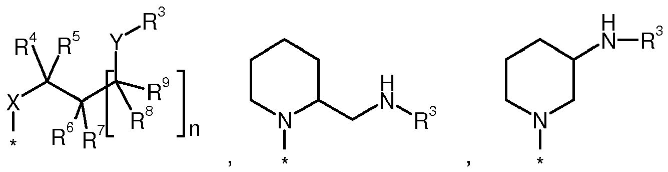 or 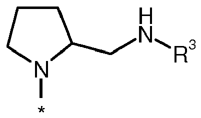 where
* is the point of attachment to the heterocycle,
n represents the number 0 or 1,
X represents NR¹⁰, S or O,
where
R¹⁰ represents hydrogen or methyl,
Y represents NR¹¹ or S,
where
R¹¹ represents hydrogen or methyl,
R³ represents 2-pyridyl, pyrimid-2-yl, 2-aminopyrimid-4-yl, 1,3-oxazol-2-yl, 1,3-oxazol-4-yl, 1,2,4-oxadiazol-3-yl, 1,2,3-oxadiazol-4-yl, 1,3-thiazol-2-yl or 1,3-thiazol-4-yl,
where 2-pyridyl, pyrimid-2-yl, 1,3-oxazol-2-yl, 1,3-oxazol-4-yl, 1,3-thiazol-2-yl and 1,3-thiazol-4-yl are substituted by 1 or 2 substituents, where the substituents independently of one another are selected from the group consisting of halogen, cyano, nitro, amino, trifluoromethyl, trifluoromethoxy, aminocarbonyl, trifluoromethylcarbonyl methyl, ethyl, methoxy, ethoxy, C₁-C₄-alkylamino, methylcarbonyl, ethylcarbonyl, cyclopropylcarbonyl, methoxycarbonyl and ethoxycarbonyl,
and
where 2-aminopyrimid-4-yl, 1,2,4-oxadiazol-3-yl and 1,2,3-oxadiazol-4-yl may be substituted by a substituent, where the substituent is selected from the group consisting of halogen, cyano, nitro, amino, trifluoromethyl, trifluoromethoxy, aminocarbonyl, trifluoromethylcarbonyl, methyl, ethyl, methoxy, ethoxy, C₁-C₄-alkylamino, methylcarbonyl, ethylcarbonyl, cyclopropylcarbonyl, methoxycarbonyl and ethoxycarbonyl,
R⁴ represents hydrogen or methyl,
R⁵ represents hydrogen or methyl,
R⁶ represents hydrogen or methyl,
R⁷ represents hydrogen or methyl,
R⁸ represents hydrogen or methyl,
R⁹ represents hydrogen or methyl,
R² represents C₆-C₁₀-aryl, thienyl, furyl, pyrrolyl, thiazolyl, oxazolyl, oxadiazolyl, pyrazolyl, imidazolyl, pyridyl, pyrimidyl, pyridazinyl, pyrazinyl, indolyl, indazolyl, quinolinyl, benzofuranyl or benzoxazolyl,
where aryl, thienyl, furyl, pyrrolyl, thiazolyl, oxazolyl, oxadiazolyl, pyrazolyl, imidazolyl, pyridyl, pyrimidyl, pyridazinyl, pyrazinyl, indolyl, indazolyl, quinolinyl, benzofuranyl and benzoxazolyl may be substituted by 1 to 3 substituents, where the substituents independently of one another are selected from the group consisting of hydroxyl, hydroxymethyl, amino, halogen, cyano, trifluoromethyl, trifluoromethoxy, aminocarbonyl, C₁-C₄-alkyl, C₁-C₄-alkoxy, C₁-C₄-alkoxymethyl, C₁-C₄-alkylamino, C₁-C₄-alkylaminomethyl, C₁-C₄-alkylcarbonyl, C₁-C₄-alkoxycarbonyl, C₁-C₄-alkylaminocarbonyl, C₁-C₄-alkylcarbonylamino, C₁-C₄-alkylsulfonyl, C₁-C₄-alkylsulfonylamino, C₁-C₄-alkylaminosulfonyl, phenyl, benzyloxy, 5- or 6-membered heterocyclyl, 5- or 6-membered heterocyclylcarbonyl, 5- or 6-membered heterocyclylmethyl and 5- or 6-membered heteroaryl,
where phenyl, benzyloxy, heterocyclyl, heterocyclylcarbonyl, heterocyclylmethyl and heteroaryl may be substituted by 1 to 3 substituents, where the substituents independently of one another are selected from the group consisting of halogen, cyano, trifluoromethyl, trifluoromethoxy, aminocarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₁-C₄-alkylcarbonylamino,
or one of its salts, its solvates or the solvates of its salts.

3. Compound according to Claim 1 or 2, **characterized in that**
either
U represents N,
V represents CR¹²,
W represents CH,
A represents CR¹⁵,
or
U represents CH,
V represents CR¹²,
W represents N,
A represents CR¹⁵,
or
U represents CR¹⁶,
V represents N,
W represents CR¹⁷,
A represents N,
where
R¹² represents hydrogen, hydroxycarbonyl, aminocarbonyl, methyl, ethyl, C₁-C₄-alkylcarbonyl, C₁-C₄-alkoxycarbonyl, C₁-C₄-alkylaminocarbonyl, C₁-C₄-alkylcarbonylamino, pyrrolidinylcarbonyl, piperidinylcarbonyl, piperazinylcarbonyl, morpholinylcarbonyl or -CH₂R¹³,
where pyrrolidinylcarbonyl, piperidinylcarbonyl, piperazinylcarbonyl and morpholinylcarbonyl may be substituted by 1 to 2 substituents, where the substituents independently of one another are selected from the group consisting of oxo, methyl and ethyl,
and
where alkylcarbonyl, C₂-C₄-alkoxycarbonyl and C₂-C₄-alkylaminocarbonyl may be substituted by a substituent, where the substituent is selected from the group consisting of hydroxyl, amino, C₁-C₄-alkylamino, pyrrolidinyl, piperidinyl, piperazinyl and morpholinyl,
where pyrrolidinyl, piperidinyl, piperazinyl and morpholinyl may be substituted by 1 to 2 substituents, where the substituents independently of one another are selected from the group consisting of oxo, methyl and ethyl,
and
where
R¹³ represents hydroxyl, amino, hydroxycarbonyl, aminocarbonyl, C₁-C₄-alkoxy, C₁-C₄-alkylamino, pyrrolidinyl, piperidinyl, piperazinyl or morpholinyl,
where pyrrolidinyl, piperidinyl, piperazinyl and morpholinyl may be substituted by 1 to 2 substituents, where the substituents independently of one another are selected from the group consisting of oxo, methyl and ethyl,
R¹⁵ represents hydrogen,
R¹⁶ represents hydrogen or methyl,
R¹⁷ represents hydrogen or methyl,
R¹ represents a group of the formula 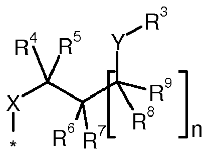 where
* is the point of attachment to the heterocycle,
n represents the number 0,
X represents NR¹⁰,
where
R¹⁰ represents hydrogen,
Y represents NR¹¹, where
R¹¹ represents hydrogen or methyl,
R³ represents 2-pyridyl, pyrimid-2-yl, 2-aminopyrimid-4-yl, 1,3-thiazol-2-yl or 1,3-thiazol-4-yl,
where 2-pyridyl, pyrimid-2-yl, 1,3-thiazol-2-yl and 1,3-thiazol-4-yl are substituted by 1 or 2 substituents, where the substituents independently of one another are selected from the group consisting of fluorine, chlorine, cyano, nitro, amino and trifluoromethyl,
and
where 2-aminopyrimid-4-yl may be substituted by a substituent, where the substituent is selected from the group consisting of fluorine, chlorine, cyano, nitro, amino and trifluoromethyl,
R⁴ represents hydrogen,
R⁵ represents hydrogen or methyl,
R⁶ represents hydrogen,
R⁷ represents hydrogen or methyl,
R⁸ represents hydrogen,
R⁹ represents hydrogen or methyl,
R² represents phenyl, thienyl, pyrazolyl or pyridyl,
where phenyl, thienyl, pyrazolyl and pyridyl may be substituted by 1 to 2 substituents, where the substituents independently of one another are selected from the group consisting of halogen, trifluoromethyl, trifluoromethoxy, aminocarbonyl, C₁-C₄-alkyl, C₁-C₄-alkoxy, C₁-C₄-alkoxycarbonyl, C₁-C₄-alkylaminocarbonyl, pyrrolidinyl, piperidinyl, morpholinyl and morpholinylcarbonyl,
or one of its salts, its solvates or the solvates of its salts.

4. Compound according to any of Claims 1, 2 and 3, **characterized in that**
either
U represents N,
V represents CR¹²,
W represents CH,
A represents CR¹⁵,
or
U represents CH,
V represents CR¹²,
W represents N,
A represents CR¹⁵,
or
U represents CR¹⁶,
V represents N,
W represents CR¹⁷,
A represents N,
where
R¹² represents hydrogen, hydroxycarbonyl, methyl, ethyl, methoxycarbonyl, ethoxycarbonyl, C₁-C₄-alkylaminocarbonyl, piperidinylcarbonyl or morpholinylcarbonyl,
where piperidinylcarbonyl and morpholinylcarbonyl may be substituted by a substituent, where the substituent is selected from the group consisting of methyl and ethyl,
and
where C₂-C₄-alkylaminocarbonyl may be substituted by a substituent, where the substituent is selected from the group consisting of C₁-C₄-alkylamino, piperazinyl and morpholinyl,
where piperazinyl and morpholinyl may be substituted by a substituent, where the substituent is selected from the group consisting of methyl and ethyl,
R¹⁵ represents hydrogen,
R¹⁶ represents methyl,
R¹⁷ represents methyl,
R¹ represents a group of the formula 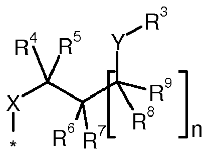 where
* is the point of attachment to the heterocycle,
n represents the number 0,
X represents NR¹⁰,
where
R¹⁰ represents hydrogen,
Y represents NR¹¹,
where
R¹¹ represents hydrogen or methyl,
R³ represents a group of the formula 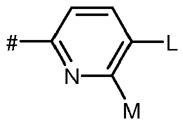 where
# is the point of attachment to Y,
L represents cyano, nitro or trifluoromethyl,
M represents hydrogen or amino,
R⁴ represents hydrogen,
R⁵ represents hydrogen or methyl,
R⁶ represents hydrogen,
R⁷ represents hydrogen or methyl,
R⁸ represents hydrogen,
R⁹ represents hydrogen,
R² represents phenyl,
where phenyl may be substituted by 1 to 2 substituents, where the substituents independently of one another are selected from the group consisting of fluorine, chlorine, trifluoromethyl, trifluoromethoxy, C₁-C₃-alkyl, methoxy, methoxycarbonyl and ethoxycarbonyl,
or one of its salts, its solvates or the solvates of its salts.

5. Process for preparing a compound of the formula (I) or one of its salts, its solvates or the solvates of its salts according to Claim 1, **characterized in that**
[A] a compound of the formula 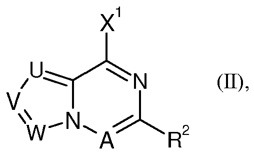 in which
A, U, V, W and R² have the meaning given in Claim 1,
and
X¹ represents halogen, preferably chlorine or fluorine,
is reacted with a compound of the formula
R¹-H (III),
in which
R¹ has the meaning given in Claim 1,
or
[B] a compound of the formula 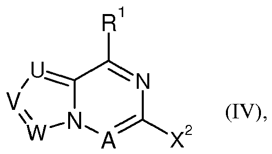 in which
R¹ has the meaning given in Claim 1,
and
A represents CR¹⁵,
where R¹⁵ has the meaning given in Claim 1,
U represents N,
V represents CR¹²,
where R¹² has the meaning given in Claim 1,
W represents CH,
X² represents iodine, bromine, chlorine or trifluoromethanesulfonyl, preferably iodine or bromine,
is reacted under Suzuki coupling conditions with a compound of the formula
Q-R² (V),
in which
R² has the meaning given in Claim 1, and
Q represents -B(OH)₂, a boronic acid ester, preferably boronic acid pinacolate, or -BF₃⁻K⁺,
to give a compound of the formula 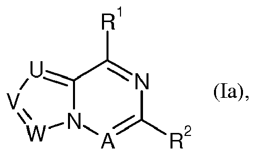 in which
R¹ and R² have the meaning given in Claim 1, and
A represents CR¹⁵,
where R¹⁵ has the meaning given in Claim 1,
U represents N,
V represents CR¹²,
where R¹² has the meaning given in Claim 1,
W represents CH,

6. Compound according to any of Claims 1 to 4 for the treatment and/or prophylaxis of diseases.

7. Use of a compound according to any of Claims 1 to 4 for preparing a medicament for the treatment and/or prophylaxis of diseases.

8. Use according Claim 7, the diseases being hematologic disorders.

9. Medicament comprising a compound according to any of Claims 1 to 4 in combination with an inert non-toxic pharmaceutically acceptable auxiliary.

10. Medicament according to Claim 9 for the treatment and/or prophylaxis of hematological disorders.

11. Use of a compound according to any of Claims 1 to 4 for the efficient *ex vivo* expansion of adult hematopoietic stem cells from bone marrow, from peripheral blood or umbilical cord blood.

12. Method for the *ex vivo* expansion of adult hematopoietic stem cells from bone marrow, from peripheral blood or umbilical cord blood, **characterized in that** an effective amount of a compound according to any of Claims 1 to 4 is added.

## Revendications

1. Composé de formule 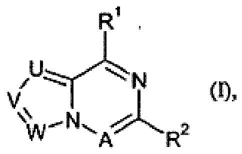 dans laquelle
soit
U représente N,
V représente CR¹²,
W représente CH,
A représente CR¹⁵,
soit
U représente CH,
V représente CR¹²,
W représente N,
A représente CR¹⁵,
ou
U représente CR¹⁶,
V représente N,
W représente CR¹⁷_{,}
A représente N,
R¹² représentant un atome d'hydrogène, un groupe hydroxy, amino, hydroxycarbonyle, aminocarbonyle, trifluorométhyle, trifluorométhoxy, cyano, alkyle en C₁-C₄, alcoxy en C₁-C₄, alkyl(C₁-C₄)amino, alkyl(C₁-C₄)-carbonyle, alcoxy(C₁-C₄)carbonyle, alkyl(C₁-C₄)aminocarbonyle, alkyl(C₁-C₄)-carbonylamino, alkyl(C₁-C₄)sulfonylamino, hétérocyclyl(à 5 ou 6 chaînons)carbonyle, -CH₂R¹³ ou -CH₂CH₂R¹⁴,
le groupe hétérocyclylcarbonyle pouvant être substitué par 1 à 3 substituants, les substituants étant choisis chacun indépendamment dans l'ensemble constitué par des atomes d'halogène, des groupes oxo, alkyle en C₁-C₄, alcoxy en C₁-C₄, alkyl(C₁-C₄)-amino, alkyl(C₁-C₄)carbonyle, alcoxy(C₁-C₄)-carbonyle et alkyl(C₁-C₄)aminocarbonyle,
et
les groupes alcoxy, alkylamino, alkylcarbonyle, alcoxycarbonyle, alkylaminocarbonyle, alkylcarbonylamino et alkylsulfonylamino pouvant être substitués par un substituant, le substituant étant choisi dans l'ensemble constitué par des groupes hydroxy, amino, hydroxycarbonyle, aminocarbonyle, alcoxy en C₁-C₄, alkyl(C₁-C₄)amino, alcoxy(C₁-C₄)carbonyle, alkyl(C₁-C₄)aminocarbonyle, alkyl(C₁-C₄)carbonylamino, hétérocyclyle à 5 ou 6 chaînons et phényle,
où le groupe phényle peut être substitué par 1 à 3 substituants, les substituants étant choisis chacun indépendamment dans l'ensemble constitué par des atomes d'halogène, des groupes cyano, trifluorométhyle, trifluorométhoxy, aminocarbonyle, alkyle en C₁-C₄, alcoxy en C₁-C₄, alkyl(C₁-C₄)amino, alkyl(C₁-C₄)-carbonyle, alcoxy(C₁-C₄)carbonyle, alkyl(C₁-C₄)aminocarbonyle et alkyl(C₁-C₄)carbonylamino,
et
où le groupe hétérocyclyle peut être substitué par 1 à 3 substituants, les substituants étant choisis chacun indépendamment dans l'ensemble constitué par des atomes d'halogène, des groupes oxo, alkyle en C₁-C₄, alcoxy en C₁-C₄, alkyl(C₁-C₄)amino, alkyl(C₁-C₄)carbonyle, alcoxy(C₁-C₄)carbonyle et alkyl(C₁-C₄)-aminocarbonyle,
et
R¹³ représentant un groupe hydroxy, amino, cyano, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle, alkyl(C₁-C₄)-carbonylamino, cycloalkyl(C₃-C₆)amino ou hétérocyclyle à 5 ou 6 chaînons,
où les groupes alcoxy, alkylamino, alcoxycarbonyle, alkylaminocarbonyle et alkylcarbonylamino peuvent être substitués par un substituant, le substituant étant choisi dans l'ensemble constitué par des groupes hydroxy, amino, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle et alkyl(C₁-C₄)carbonyl-amino,
et
où le groupe hétérocyclyle peut être substitué par 1 à 3 substituants, les substituants étant choisis chacun indépendamment dans l'ensemble constitué par des atomes d'halogène, des groupes oxo, alkyle en C₁-C₄, alcoxy en C₁-C₄, alkyl(C₁-C₄)amino, alkyl(C₁-C₄)carbonyle, alcoxy(C₁-C₄)carbonyle et alkyl(C₁-C₄)-aminocarbonyle,
et
R¹⁴ représentant un groupe hydroxy, amino, cyano, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle, alkyl(C₁-C₄)carbonylamino ou hétérocyclyle à 5 ou 6 chaînons,
où les groupes alcoxy, alkylamino, alcoxycarbonyle, alkylaminocarbonyle et alkylcarbonylamino peuvent être substitués par un substituant, le substituant étant choisi dans l'ensemble constitué par des groupes hydroxy, amino, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle et alkyl(C₁-C₄)carbonyl-amino,
et
où le groupe hétérocyclyle peut être substitué par 1 à 3 substituants, les substituants étant choisis chacun indépendamment dans l'ensemble constitué par des atomes d'halogène, des groupes oxo, alkyle en C₁-C₄, alcoxy en C₁-C₄, alkyl(C₁-C₄)amino, alkyl(C₁-C₄)carbonyle, alcoxy(C₁-C₄)carbonyle et alkyl(C₁-C₄)-aminocarbonyle,
R¹⁵ représente un atome d'hydrogène ou d'halogène, un groupe cyano, trifluorométhyle, alkyle en C₁-C₃, méthoxy, méthylthio ou cyclopropyle,
R¹⁶ représente un atome d'hydrogène ou le groupe méthyle,
R¹⁷ représente un atome d'hydrogène ou le groupe méthyle,
R¹ représente un groupe de formule 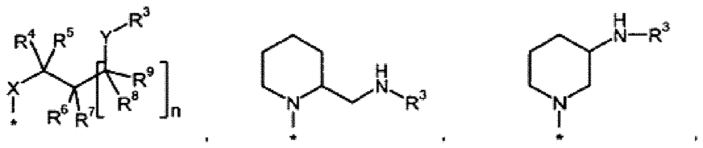 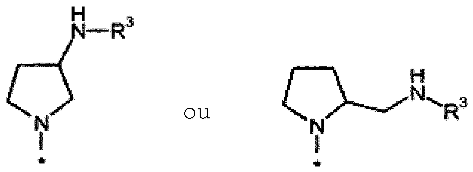 formules dans lesquelles
* représente la position de liaison à l'hétérocycle,
n représente le nombre 0 ou 1,
X représente NR¹⁰, S ou 0,
où
R¹⁰ représente un atome d'hydrogène, un groupe alkyle en C₁-C₃ ou cyclopropyle,
Y représente NR¹¹ ou S,
où
R¹¹ représente un atome d'hydrogène, un groupe alkyle en C₁-C₃ ou cyclopropyle,
R³ représente le groupe 2-pyridyle, pyrimid-2-yle, 2-aminopyrimid-4-yle, 2-[mono-alkyl(C₁-C₄)amino]pyrimid-4-yle, 2-[mono-cycloalkyl(C₃-C₄)amino]pyrimid-4-yle, pyridazin-3(2H)-on-6-yle, 1,3-oxazol-2-yle, 1,3-oxazol-4-yle, 1,2,4-oxadiazol-3-yle, 1,2,3-oxadiazol-4-yle, 1,3-thiazol-2-yle, 1,3-thiazol-4-yle, 1H-1,2,4-triazol-5-yle, 2,4-dihydro-3H-1,2,4-triazol-3-on-5-yle ou 1,2-pyrazol-5-yle, les groupes 2-pyridyle, pyrimid-2-yle, 1,3-oxazol-2-yle, 1,3-oxazol-4-yle, 1,3-thiazol-2-yle et 1,3-thiazol-4-yle étant substitués par 1 ou 2 substituants, les substituants étant choisis indépendamment l'un de l'autre dans l'ensemble constitué par des atomes d'halogène, des groupes cyano, nitro, amino, trifluorométhyle, trifluorométhoxy, aminocarbonyle, trifluorométhylcarbonyle, alkyle en C₁-C₄, alcoxy en C₁-C₄, alkyl(C₁-C₄)amino, cycloalkyl(C₃-C₄)amino, alkyl(C₁-C₄)carbonyle, alcoxy(C₁-C₄)carbonyle, alkyl(C₁-C₄)aminocarbonyle et cycloalkyl(C₃-C₆)carbonyle,
ou les groupes alkyle, alcoxy, alkylamino, alkylcarbonyle, alcoxycarbonyle, alkylaminocarbonyle et cycloalkylcarbonyle pouvant être substitués par un substituant, le substituant étant choisi dans l'ensemble constitué par des atomes d'halogène, des groupes cyano, hydroxy, amino, trifluorométhyle et cycloalkyle en C₃-C₆,
et
les groupes 2-aminopyrimid-4-yle, 2-[mono-alkyl(C₁-C₄)amino]pyrimid-4-yle, 2-[mono-cycloalkyl(C₃-C₄)amino]pyrimid-4-yle, pyridazin-3(2H)-on-6-yle, 1,2,4-oxadiazol-3-yle, 1,2,3-oxadiazol-4-yle, 1H-1,2,4-triazol-5-yle, 2,4-dihydro-3H-1,2,4-triazol-3-on-5-yle et 1,2-pyrazol-5-yle peuvent être substitués par un substituant, le substituant étant choisi dans l'ensemble constitué par des atomes d'halogène, des groupes cyano, nitro, amino, trifluorométhyle, trifluorométhoxy, aminocarbonyle, trifluorométhylcarbonyle, alkyle en C₁-C₄, alcoxy en C₁-C₄, alkyl(C₁-C₄)amino, cycloalkyl(C₃-C₄)amino, alcoxy(C₁-C₄)carbonyle, alkyl(C₁-C₄)aminocarbonyle et cycloalkyl(C₃-C₆)carbonyle,
R⁴ représente un atome d'hydrogène, un groupe alkyle en C₁-C₃ ou cyclopropyle,
R⁵ représente un atome d'hydrogène ou un groupe alkyle en C₁-C₃,
R⁶ représente un atome d'hydrogène, un groupe alkyle en C₁-C₃ ou cyclopropyle,
R⁷ représente un atome d'hydrogène ou un groupe alkyle en C₁-C₃,
R⁸ représente un atome d'hydrogène, un groupe alkyle en C₁-C₃ ou cyclopropyle,
R⁹ représente un atome d'hydrogène ou un groupe alkyle en C₁-C₃,
R² représente un groupe aryle en C₆-C₁₀ ou hétéroaryle à 5 à 10 chaînons,
les groupes aryle et hétéroaryle pouvant être substitués par 1 à 3 substituants, les substituants étant choisis chacun indépendamment dans l'ensemble constitué par des atomes d'halogène, des groupes hydroxy, hydroxyméthyle, amino, cyano, trifluorométhyle, trifluorométhoxy, aminocarbonyle, alkyle en C₁-C₄, alcoxy en C₁-C₄, alcoxy(C₁-C₄)méthyle, alkyl(C₁-C₄)amino, alkyl(C₁-C₄)aminométhyle, alkyl(C₁-C₄)carbonyle, alcoxy(C₁-C₄)carbonyle, alkyl(C₁-C₄)aminocarbonyle, alkyl(C₁-C₄)carbonylamino, alkyl(C₁-C₄)sulfonyle, alkyl(C₁-C₄)sulfonylamino, alkyl(C₁-C₄)amino-sulfonyle, phényle, benzyloxy, hétérocyclyle à 5 ou 6 chaînons, hétérocyclyl(à 5 ou 6 chaînons)-carbonyle, hétérocyclyl(à 5 ou 6 chaînons)-méthyle et hétéroaryle à 5 ou 6 chaînons,
où les groupes phényle, benzyloxy, hétérocyclyle, hétérocyclylcarbonyle, hétérocyclylméthyle et hétéroaryle peuvent être substitués par 1 à 3 substituants, les substituants étant choisis chacun indépendamment dans l'ensemble constitué par des atomes d'halogène, des groupes cyano, trifluorométhyle, trifluorométhoxy, aminocarbonyle, alkyle en C₁-C₄, alcoxy en C₁-C₄, alkyl(C₁-C₄)amino, alkyl(C₁-C₄)carbonyle, alcoxy(C₁-C₄)carbonyle, alkyl(C₁-C₄)aminocarbonyle et alkyl(C₁-C₄)carbonylamino,
ou
deux des substituants sur le groupe aryle forment conjointement avec les atomes de carbone, auxquels ils sont liés, un groupe 1,3-dioxolane ou 1,4-dioxane
ou un de ses sels, de ses produits de solvatation ou des produits de solvatation de ses sels.

2. Composé selon la revendication 1, **caractérisé en ce que** soit
U représente N,
V représente CR¹²,
W représente CH,
A représente CR¹⁵,
soit
U représente CH,
V représente CR¹²,
W représente N,
A représente CR¹⁵,
ou
U représente CR¹⁶,
V représente N,
W représente CR¹⁷,
A représente N,
R¹² représentant un atome d'hydrogène, un groupe hydroxycarbonyle, aminocarbonyle, alkyle en C₁-C₄, alcoxy en C₁-C₄, alkyl(C₁-C₄)amino, alkyl(C₁-C₄)carbonyle, alcoxy(C₁-C₄)carbonyle, alkyl(C₁-C₄)aminocarbonyle, alkyl(C₁-C₄)-carbonylamino, hétérocyclyl(à 5 ou 6 chaînons)carbonyle, -CH₂R¹³ ou -CH₂CH₂R¹⁴,
le groupe hétérocyclylcarbonyle pouvant être substitué par 1 à 3 substituants, les substituants étant choisis chacun indépendamment dans l'ensemble constitué par des groupes oxo, alkyle en C₁-C₄, alcoxy en C₁-C₄, alkyl(C₁-C₄)amino, alkyl(C₁-C₄)-carbonyle, alcoxy(C₁-C₄)carbonyle et alkyl(C₁-C₄)aminocarbonyle,
et
les groupes alcoxy, alkylamino, alkylcarbonyle, alcoxycarbonyle, alkylaminocarbonyle et alkylcarbonylamino pouvant être substitués par un substituant, le substituant étant choisi dans l'ensemble constitué par des groupes hydroxy, amino, hydroxycarbonyle, aminocarbonyle, alcoxy en C₁-C₄, alkyl(C₁-C₄)amino et hétérocyclyle à 5 ou 6 chaînons,
où le groupe hétérocyclyle peut être substitué par 1 ou 2 substituants, les substituants étant choisis indépendamment l'un de l'autre dans l'ensemble constitué par des groupes oxo, alkyle en C₁-C₄, alcoxy en C₁-C₄, alkyl(C₁-C₄)amino, alkyl(C₁-C₄)carbonyle, alcoxy(C₁-C₄)carbonyle et alkyl(C₁-C₄)-aminocarbonyle,
et
R¹³ représentant un groupe hydroxy, amino, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle, alkyl(C₁-C₄)-carbonylamino, cycloalkyl(C₃-C₆)amino ou hétérocyclyle à 5 ou 6 chaînons,
où les groupes alcoxy, alkylamino, alcoxycarbonyle, alkylaminocarbonyle et alkylcarbonylamino peuvent être substitués par un substituant, le substituant étant choisi dans l'ensemble constitué par des groupes hydroxy, amino, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle et alkyl(C₁-C₄)carbonyl-amino,
et
où le groupe hétérocyclyle peut être substitué par 1 ou 2 substituants, les substituants étant choisis indépendamment l'un de l'autre dans l'ensemble constitué par des groupes oxo, alkyle en C₁-C₄, alcoxy en C₁-C₄, alkyl(C₁-C₄)amino, alkyl(C₁-C₄)carbonyle, alcoxy(C₁-C₄)carbonyle et alkyl(C₁-C₄)aminocarbonyle,
et
R¹⁴ représentant un groupe hydroxy, amino, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle, alkyl(C₁-C₄)carbonylamino ou hétérocyclyle à 5 ou 6 chaînons, où les groupes alcoxy, alkylamino, alcoxycarbonyle, alkylaminocarbonyle et alkylcarbonylamino peuvent être substitués par un substituant, le substituant étant choisi dans l'ensemble constitué par des groupes hydroxy, amino, hydroxycarbonyle, aminocarbonyle, alcoxy en C₁-C₄, alkyl(C₁-C₄)amino, alcoxy(C₁-C₄)carbonyle, alkyl(C₁-C₄)-aminocarbonyle et alkyl(C₁-C₄)carbonyl-amino,
et où le groupe hétérocyclyle peut être substitué par 1 ou 2 substituants, les substituants étant choisis indépendamment l'un de l'autre dans l'ensemble constitué par des groupes oxo, alkyle en C₁-C₄, alcoxy en C₁-C₄, alkyl(C₁-C₄)amino, alkyl(C₁-C₄)carbonyle, alcoxy(C₁-C₄)carbonyle et alkyl(C₁-C₄)-aminocarbonyle,
R¹⁵ représente un atome d'hydrogène ou d'halogène, le groupe cyano ou trifluorométhyle,
R¹⁶ représente un atome d'hydrogène ou le groupe méthyle,
R¹⁷ représente un atome d'hydrogène ou le groupe méthyle,
R¹ représente un groupe de formule 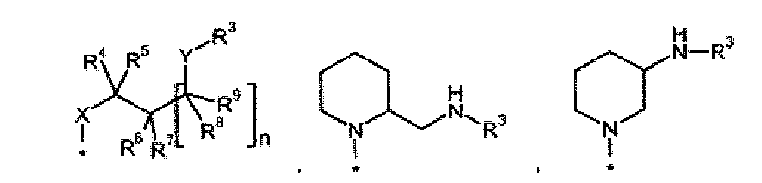 ou 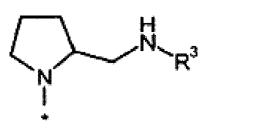 formules dans lesquelles
* représente la position de liaison à l'hétérocycle,
n représente le nombre 0 ou 1,
X représente NR¹⁰, S ou 0, où
R¹⁰ représente un atome d'hydrogène ou le groupe méthyle,
Y représente NR¹¹ ou S,
où
R¹¹ représente un atome d'hydrogène ou le groupe méthyle,
R³ représente le groupe 2-pyridyle, pyrimid-2-yle, 2-aminopyrimid-4-yle, 1,3-oxazol-2-yle, 1,3-oxazol-4-yle, 1,2,4-oxadiazol-3-yle, 1,2,3-oxadiazol-4-yle, 1,3-thiazol-2-yle ou 1,3-thiazol-4-yle,
les groupes 2-pyridyle, pyrimid-2-yle, 1,3-oxazol-2-yle, 1,3-oxazol-4-yle, 1,3-thiazol-2-yle et 1,3-thiazol-4-yle étant substitués par 1 ou 2 substituants, les substituants étant choisis indépendamment l'un de l'autre dans l'ensemble constitué par des atomes d'halogène, des groupes cyano, nitro, amino, trifluorométhyle, trifluorométhoxy, aminocarbonyle, trifluorométhylcarbonyle, méthyle, éthyle, méthoxy, éthoxy, alkyl(C₁-C₄)amino, méthylcarbonyle, éthylcarbonyle, cyclopropyl-carbonyle, méthoxycarbonyle et éthoxycarbonyle,
et
les groupes 2-aminopyrimid-4-yle, 1,2,4-oxadiazol-3-yle et 1,2,3-oxadiazol-4-yle pouvant être substitués par un substituant, le substituant étant choisi dans l'ensemble constitué par des atomes d'halogène, des groupes cyano, nitro, amino, trifluorométhyle, trifluorométhoxy, aminocarbonyle, trifluorométhylcarbonyle, méthyle, éthyle, méthoxy, éthoxy, alkyl(C₁-C₄)amino, méthylcarbonyle, éthylcarbonyle, cyclopropyl-carbonyle, méthoxycarbonyle et éthoxycarbonyle,
R⁴ représente un atome d'hydrogène ou le groupe méthyle,
R⁵ représente un atome d'hydrogène ou le groupe méthyle,
R⁶ représente un atome d'hydrogène ou le groupe méthyle,
R⁷ représente un atome d'hydrogène ou le groupe méthyle,
R⁸ représente un atome d'hydrogène ou le groupe méthyle,
R⁹ représente un atome d'hydrogène ou le groupe méthyle,
R² représente un groupe aryle en C₆-C₁₀, thiényle, furyle, pyrrolyle, thiazolyle, oxazolyle, oxadiazolyle, pyrazolyle, imidazolyle, pyridyle, pyrimidyle, pyridazinyle, pyrazinyle, indolyle, indazolyle, quinolinyle, benzofuranyle ou benzoxazolyle,
les groupes aryle, thiényle, furyle, pyrrolyle, thiazolyle, oxazolyle, oxadiazolyle, pyrazolyle, imidazolyle, pyridyle, pyrimidyle, pyridazinyle, pyrazinyle, indolyle, indazolyle, quinolinyle, benzofuranyle et benzoxazolyle pouvant être substitués par 1 à 3 substituants, les substituants étant choisis chacun indépendamment dans l'ensemble constitué par des atomes d'halogène, des groupes hydroxy, hydroxyméthyle, amino, cyano, trifluorométhyle, trifluorométhoxy, aminocarbonyle, alkyle en C₁-C₄, alcoxy en C₁-C₄, alcoxy(C₁-C₄)méthyle, alkyl(C₁-C₄)amino, alkyl(C₁-C₄)aminométhyle, alkyl(C₁-C₄)carbonyle, alcoxy(C₁-C₄)carbonyle, alkyl(C₁-C₄)aminocarbonyle, alkyl(C₁-C₄)carbonylamino, alkyl(C₁-C₄)sulfonyle, alkyl(C₁-C₄)sulfonylamino, alkyl(C₁-C₄)amino-sulfonyle, phényle, benzyloxy, hétérocyclyle à 5 ou 6 chaînons, hétérocyclyl(à 5 ou 6 chaînons)-carbonyle, hétérocyclyl(à 5 ou 6 chaînons)-méthyle et hétéroaryle à 5 ou 6 chaînons,
où les groupes phényle, benzyloxy, hétérocyclyle, hétérocyclylcarbonyle, hétérocyclylméthyle et hétéroaryle peuvent être substitués par 1 à 3 substituants, les substituants étant choisis chacun indépendamment dans l'ensemble constitué par des atomes d'halogène, des groupes cyano, trifluorométhyle, trifluorométhoxy, aminocarbonyle, alkyle en C₁-C₄, alcoxy en C₁-C₄, alkyl(C₁-C₄)amino, alkyl(C₁-C₄)carbonyle, alcoxy(C₁-C₄)carbonyle, alkyl(C₁-C₄)aminocarbonyle et alkyl(C₁-C₄)carbonylamino,
ou un de ses sels, de ses produits de solvatation ou des produits de solvatation de ses sels.

3. Composé selon la revendication 1 ou 2, **caractérisé en ce que**
soit
U représente N,
V représente CR¹²,
W représente CH,
A représente CR¹⁵,
soit
U représente CH,
V représente CR¹²,
W représente N,
A représente CR¹⁵,
ou
U représente CR¹⁶,
V représente N,
W représente CR¹⁷,
A représente N,
R¹² représentant un atome d'hydrogène, un groupe hydroxycarbonyle, aminocarbonyle, méthyle, éthyle, alkyl(C₁-C₄)carbonyle, alcoxy(C₁-C₄)-carbonyle, alkyl(C₁-C₄)aminocarbonyle, alkyl(C₁-C₄)carbonylamino, pyrrolidinylcarbonyle, pipéridinylcarbonyle, pipérazinylcarbonyle, morpholinylcarbonyle ou -CH₂R¹³,
les groupes pyrrolidinylcarbonyle, pipéridinylcarbonyle, pipérazinylcarbonyle et morpholinylcarbonyle pouvant être substitués par 1 ou 2 substituants, les substituants étant choisis indépendamment l'un de l'autre dans l'ensemble constitué par les groupes oxo, méthyle et éthyle,
et
les groupes alkylcarbonyle, alcoxycarbonyle en C₂-C₄ et alkylaminocarbonyle en C₂-C₄ pouvant être substitués par un substituant, le substituant étant choisi dans l'ensemble constitué par des groupes hydroxy, amino, alkyl(C₁-C₄)amino, pyrrolidinyle, pipéridinyle, pipérazinyle et morpholinyle,
où les groupes pyrrolidinyle, pipéridinyle, pipérazinyle et morpholinyle peuvent être substitués par 1 ou 2 substituants, les substituants étant choisis indépendamment l'un de l'autre dans l'ensemble constitué par les groupes oxo, méthyle et éthyle, et
R¹³ représentant un groupe hydroxy, amino, hydroxycarbonyle, aminocarbonyle, alcoxy en C₁-C₄, alkyl(C₁-C₄)amino, pyrrolidinyle, pipéridinyle, pipérazinyle ou morpholinyle,
R¹⁵ où les groupes pyrrolidinyle, pipéridinyle, pipérazinyle et morpholinyle peuvent être substitués par 1 ou 2 substituants, les substituants étant choisis indépendamment l'un de l'autre dans l'ensemble constitué par les groupes oxo, méthyle et éthyle, représente un atome d'hydrogène,
R¹⁶ représente un atome d'hydrogène ou le groupe méthyle,
R¹⁷ représente un atome d'hydrogène ou le groupe méthyle,
R¹ représente un groupe de formule 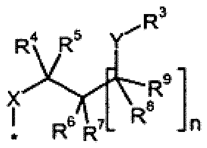 dans laquelle
* représente la position de liaison à l'hétérocycle,
n représente le nombre 0,
X représente NR¹⁰,
où
R¹⁰ représente un atome d'hydrogène,
Y représente NR¹¹,
où
R¹¹ représente un atome d'hydrogène ou le groupe méthyle,
R³ représente le groupe 2-pyridyle, pyrimid-2-yle, 2-aminopyrimid-4-yle, 1,3-thiazol-2-yle ou 1,3-thiazol-4-yle, les groupes 2-pyridyle, pyrimid-2-yle, 1,3-thiazol-2-yle et 1,3-thiazol-4-yle étant substitués par 1 ou 2 substituants, les substituants étant choisis indépendamment l'un de l'autre dans l'ensemble constitué par les atomes de fluor, chlore, les groupes cyano, nitro, amino et trifluorométhyle,
et
le groupe 2-aminopyrimid-4-yle pouvant être substitué par un substituant, le substituant étant choisi dans l'ensemble constitué par les atomes de fluor, chlore, les groupes cyano, nitro, amino et trifluorométhyle,
R⁴ représente un atome d'hydrogène,
R⁵ représente un atome d'hydrogène ou le groupe méthyle,
R⁶ représente un atome d'hydrogène,
R⁷ représente un atome d'hydrogène ou le groupe méthyle,
R⁸ représente un atome d'hydrogène,
R⁹ représente un atome d'hydrogène ou le groupe méthyle,
R² représente le groupe phényle, thiényle, pyrazolyle ou pyridyle,
les groupes phényle, thiényle, pyrazolyle et pyridyle pouvant être substitués par 1 ou 2 substituants, les substituants étant choisis indépendamment l'un de l'autre dans l'ensemble constitué par des atomes d'halogène, des groupes trifluorométhyle, trifluorométhoxy, aminocarbonyle, alkyle en C₁-C₄, alcoxy en C₁-C₄, alcoxy(C₁-C₄)carbonyle, alkyl(C₁-C₄)aminocarbonyle, pyrrolidinyle, pipéridinyle, morpholinyle et morpholinylcarbonyle,
ou un de ses sels, de ses produits de solvatation ou des produits de solvatation de ses sels.

4. Composé selon l'une quelconque des revendications 1, 2 ou 3, **caractérisé en ce que** soit
U représente N,
V représente CR¹²,
W représente CH,
A représente CR¹⁵,
soit
U représente CH,
V représente CR¹²,
W représente N,
A représente CR¹⁵,
ou
U représente CR¹⁶,
V représente N,
W représente CR¹⁷,
A représente N,
R¹² représentant un atome d'hydrogène, un groupe hydroxycarbonyle, méthyle, éthyle, méthoxycarbonyle, éthoxycarbonyle, alkyl(C₁-C₄)aminocarbonyle, pipéridinylcarbonyle ou morpholinylcarbonyle,
les groupes pipéridinylcarbonyle et morpholinylcarbonyle pouvant être substitués par un substituant, le substituant étant choisi dans l'ensemble constitué par les groupes méthyle et éthyle,
et
le groupe alkylaminocarbonyle en C₂-C₄ pouvant être substitué par un substituant, le substituant étant choisi dans l'ensemble constitué par des groupes alkyl(C₁-C₄)amino, pipérazinyle et morpholinyle,
où les groupes pipérazinyle et morpholinyle peuvent être substitués par un substituant, le substituant étant choisi dans l'ensemble constitué par les groupes méthyle et éthyle,
R¹⁵ représente un atome d'hydrogène,
R¹⁶ représente le groupe méthyle,
R¹⁷ représente le groupe méthyle,
R¹ représente un groupe de formule 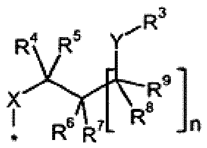 dans laquelle
* représente la position de liaison à l'hétérocycle,
n représente le nombre 0,
X représente NR¹⁰,
où
R¹⁰ représente un atome d'hydrogène,
Y représente NR¹¹,
où
R¹¹ représente un atome d'hydrogène ou le groupe méthyle,
R³ représente un groupe de formule 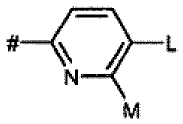 dans laquelle
# représente la position de liaison à Y,
L représente le groupe cyano, nitro ou trifluorométhyle,
M représente un atome d'hydrogène ou le groupe amino,
R⁴ représente un atome d'hydrogène,
R⁵ représente un atome d'hydrogène ou le groupe méthyle,
R⁶ représente un atome d'hydrogène,
R⁷ représente un atome d'hydrogène ou le groupe méthyle,
R⁸ représente un atome d'hydrogène,
R⁹ représente un atome d'hydrogène,
R² représente le groupe phényle,
le groupe phényle pouvant être substitué par 1 ou 2 substituants, les substituants étant choisis indépendamment l'un de l'autre dans l'ensemble constitué par les atomes de fluor, chlore, des groupes trifluorométhyle, trifluorométhoxy, alkyle en C₁-C₃, méthoxy, méthoxycarbonyle et éthoxycarbonyle,
ou un de ses sels, de ses produits de solvatation ou des produits de solvatation de ses sels.

5. Procédé pour la préparation d'un composé de formule (I) ou d'un de ses sels, de ses produits de solvatation ou des produits de solvatation de ses sels selon la revendication 1, **caractérisé en ce que**
[A] on fait réagir un composé de formule 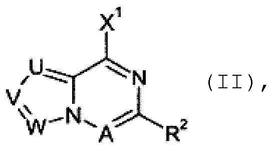 dans laquelle
A, U, V, W et R² ont la signification indiquée dans la revendication 1,
et
X¹ représente un atome d'halogène, de préférence de chlore ou de fluor,
avec un composé de formule
R¹-H (III),
dans laquelle
R¹ a la signification indiquée dans la revendication 1,
ou
[B] on fait réagir un composé de formule 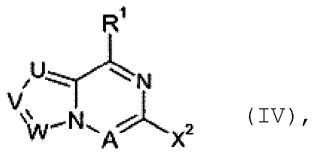 dans laquelle
R¹ a la signification indiquée dans la revendication 1,
et
A représente CR¹⁵,
R¹⁵ ayant la signification indiquée dans la revendication 1,
U représente N,
V représente CR¹²,
R¹² ayant la signification indiquée dans la revendication 1,
W représente CH,
X² représente un atome d'iode, de brome ou de chlore ou le groupe trifluorométhanesulfonyle, de préférence un atome d'iode ou de brome,
avec un composé de formule
Q-R² (V),
dans laquelle
R² a la signification indiquée dans la revendication 1, et
Q représente -B(OH)₂, un ester d'acide boronique, de préférence le boronate de pinacol, ou -BF₃⁻K⁺,
dans les conditions d'un couplage de Suzuki, pour obtenir un composé de formule 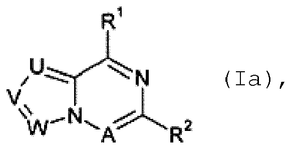 dans laquelle
R¹ et R² ont la signification indiquée dans la revendication 1,
et
A représente CR¹⁵,
R¹⁵ ayant la signification indiquée dans la revendication 1,
U représente N,
V représente CR¹²,
R¹² ayant la signification indiquée dans la revendication 1,
W représente CH.

6. Composé selon l'une quelconque des revendications 1 à 4, destiné au traitement et/ou à la prophylaxie de maladies.

7. Utilisation d'un composé selon l'une quelconque des revendications 1 à 4, pour la fabrication d'un médicament destiné au traitement et/ou à la prophylaxie de maladies.

8. Utilisation selon la revendication 7, dans laquelle les maladies consistent en des maladies hématologiques.

9. Médicament contenant un composé selon l'une quelconque des revendications 1 à 4, en association avec un adjuvant inerte, non toxique, pharmaceutiquement convenable.

10. Médicament selon la revendication 9, destiné au traitement et/ou à la prophylaxie de maladies hématologiques.

11. Utilisation d'un composé selon l'une quelconque des revendications 1 à 4, pour la multiplication efficace ex vivo de cellules souches hématopoïétiques adultes provenant de la moelle osseuse, de sang périphérique ou de sang de cordon ombilical.

12. Procédé pour la multiplication ex vivo de cellules souches hématopoïétiques adultes provenant de la moelle osseuse, de sang périphérique ou de sang de cordon ombilical, **caractérisé en ce qu'**on ajoute une quantité efficace d'un composé selon l'une quelconque des revendications 1 à 4.
